# FASCICULE DE BREVET EUROPEEN

(11) **EP 1 312 606 B1**
(45) Date de publication et mention de la délivrance du brevet: **21.09.2011**
(21) Numéro de dépôt: 02292801.4
(22) Date de dépôt: 08.11.2002
(51) Int. Cl.: C07D 213/74, C07D 401/04, A61Q 5/10

(54) **Nouvelles bases d'oxydation 2,5-diaminopyridine utiles pour la teinture des fibres kératiniques**
2,5-Diaminopyridin Oxidationsbasen, deren Verwendung zur Färbung von keratinischen Fasern
2,5-diaminopyridine oxidation bases and their use for dyeing of keratin fibres

(30) Priorité: 14.11.2001 FR 0114717
(43) Date de publication de la demande: 21.05.2003
(73) Titulaire: L'Oréal, 75008 Paris (FR)
(72) Inventeur: Vidal, Laurent, 75013 Paris (FR); Fadli, Aziz, 77500 Chelles (FR)
(74) Mandataire: Fevrier, Murielle Françoise E.

(56) Documents cités:
- EP-A- 0 303 878
- FR-A- 1 546 317
- US-A- 3 359 168

## Description

L'invention a pour objet une composition tinctoriale utile pour la teinture des fibres kératiniques contenant une base d'oxydation du type 2,5-diaminopyridine, l'utilisation de cette composition pour la teinture des fibres kératiniques ainsi que le procédé de teinture mettant en oeuvre cette composition. L'invention a aussi pour objet de nouveaux composés 2,5-diaminopyridines utiles comme base d'oxydation.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs,

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est déjà connu d'utiliser des 2,5-diaminopyridines à titre de base d'oxydation dans des compositions tinctoriales capillaires. Par exemple, le brevet FR1546317 décrit un procédé de teinture du cheveu à l'aide de composition contenant des dérivés de pyridines substituées en position 2 par un groupement amino éventuellement substitué ou un groupement alcoxy, les autres positions du noyau pyridinique pouvant être substituées par un ou deux radicaux hydroxy, amino, alkyle éventuellement substitué par un hydroxy, un amino ou un methoxy et plus particulièrement la 2,3,6-triaminopyridine. Ces dérivés de pyridines sont mis en oeuvre selon un procédé d'oxydation assuré par de l'eau oxygénée en présence d'une solution aqueuse alcaline. Selon ce document, la composition contenant ces dérivés de pyridine réagit pendant dix heures avant d'être appliquée sur cheveux humains. Il s'agit donc de l'utilisation des colorants résultant de la réaction d'oxydation qui se forment durant ces dix heures. Le procédé présente donc clairement l'inconvénient d'être peu pratique compte tenu du temps nécessaire à sa réalisation. De plus, les ténacités des colorations de cheveux obtenus à l'aide de ces diaminopyridines sont bien inférieures à celle obtenues classiquement par la coloration dite d'oxydation et les nuances sont de plus très peu variées. Le brevet US 3 359 168 décrit l'utilisation de la 2,3,6-triaminopyridine comme base d'oxydation pour la teinture d'oxydation des cheveux. Ces compositions donnent des colorations vertes intenses mais instables à la lumière. On connaît également dans les brevets GB 1 026 978 et GB 1 153 196 des compositions tinctoriales contenant à titre de base(s) d'oxydation des 2,5 diaminopyridines non substitutées sur le noyau pyridinique.

Le but de la présente invention est de fournir de nouvelles compositions tinctoriales pour la teinture de fibres kératiniques ne présentant pas les inconvénients de celles de la technique antérieure. En particulier, le but de la présente invention est de fournir des compositions qui présentent des teintures puissantes, peu sélectives et particulièrement résistantes, tout en étant capables d'engendrer des colorations intenses dans des nuances variées, en particulier dans les nuances fondamentales.

Ce but est atteint avec la présente invention qui a pour objet une composition tinctoriale comprenant, dans un milieu de teinture approprié, à titre de base d'oxydation au moins un dérivé 2,5-diaminopyridine de formule (I) ou les sels d'addition correspondants : 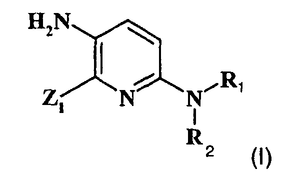 dans laquelle
Z₁ représente un radical OR₃ ou NR₄R₅,
R₁, R₂, R₄ et R₅ représentent, indépendamment l'un de l'autre, un atome d'hydrogène, un alkyle en C₁-C₆, un radical (poly)aminoalkyle en C₂-C₆, un radical (poly)alkylaminoalkyle en C₂-C₆, un radical amino-hydroxyalkyle en C₂-C₆, un radical acylaminoalkyle en C₂-C₆, un radical alcoxyalkyle en C₂-C₆, un radical hydroxyalcoxyalkyle en C₂-C₆, un radical aminoalcoxyalkyle en C₂-C₆, un radical (poly)hydroxyalkyle en C₂-C₆ un radical carboxyalkyle en C₂-C₆, un radical sulfonylalkyle en C₂-C₆ ;
R₁ et R₂ d'une part, et R₄ et R₅ d'autre part, indépendamment les uns des autres, forment avec l'atome d'azote auquel ils sont rattachés un hétérocycle saturé comportant 5 à 8 chaînons, éventuellement substitué par un ou plusieurs radicaux choisis dans le groupe constitué par les atomes d'halogène, les radicaux amino, (di)alkylamino, hydroxy, carboxy, carboxamido, alkylsulfonylamino, acétamido, alcoxy, les radicaux alkyle en C₁-C₆ éventuellement substitués par un ou plusieurs radicaux hydroxy, amino, (di)-alkylamino, alcoxy, hydroxyalcoxy, amionalcoxy, carboxy, carboxamido, sulfonamido, sulfonyl, l'hétérocycle pouvant contenir un ou plusieurs hétéroatomes additionnels choisis parmi l'oxygène, l'azote éventuellement substitué, le soufre ou un groupement SO₂, l'hétérocycle ne comptant pas de liaison peroxyde, ni de radicaux diazo ou nitroso ;
R₃ représente un radical alkyle en C₁-C₆, linéaire ou ramifié, pouvant être substitué par un ou plusieurs radicaux choisis parmi les radicaux hydroxy, amino, mono- ou di-alkylamino en C₁-C₃ dont la partie alkyle est éventuellement substituée par un ou plusieurs groupements hydroxy, amino, alcoxyalcoxy en C₁-C₂, carboxy; à l'exception de la 2,3,6-triaminopyridine et de ses sels d'addition.

Cette composition permet d'obtenir une coloration plus tenace au shampooing, à la lumière et une coloration avec une meilleure chromaticité.

Un autre objet de l'invention est l'utilisation de la composition de la présente invention pour la teinture des fibres kératiniques, en particulier les fibres kératiniques humaines telles que les cheveux.

L'invention a aussi pour objet un procédé de teinture mettant en oeuvre la composition de l'invention.

Dans le cadre de la présente invention, on entend par alkyle, des radicaux linéaires ou ramifiés par exemple méthyle, éthyle, n-propyle, iso-propyle, butyle, etc. Un radical alcoxy est un radical alk-O, le radical alkyle ayant la définition donnée ci dessus. Halogène désigne de préférence Cl, Br, I.

Les radicaux (poly)aminoalkyle, sont des radicaux alkyles substitués par un ou plusieurs groupes amino, eux mêmes éventuellement substitués par un ou plusieurs alkyles en C₁-C₄ pouvant être substitués par un ou plusieurs radicaux hydroxy, amino, carboxy, alcoxy. Les radicaux (poly)hydroxyalkyles sont des radicaux alkyles substitués par un ou plusieurs substituants hydroxy.

Selon un mode de réalisation particulier de composition de l'invention, la base d'oxydation de formule (I) est telle que R₃ désigne un radical méthyle, éthyle, hydroxyéthyle, carboxyméthyle , carboxyéthyle avec pour préférence le radical méthyle

Selon un mode de réalisation particulier de la composition de l'invention, la base d'oxydation de formule (I) est telle que R₁, R₂, R₄ et R₅ représentent, indépendamment les uns des autres, un radical (di)alkylaminoalkyle en C₂-C₄, un radical carboxyalkyle en C₂-C₄, un radical sulfonylalkyle en C₂-C₄, un radical (poly)-hydroxyalkyle en C₂-C₄, un radical (poly)aminoalkyl en C₂-C₄.

Selon un mode de réalisation différent, R₁ et R₂ d'une part, et R₄ et R₅ d'autre part, indépendamment les uns des autres, forment avec l'atome d'azote auquel ils sont rattachés un hétérocycle de 5 à 8 chaînons choisi parmi les hétérocycles pyrrolidine, pipéridine, homopipéridine, pipérazine, homopipérazine, diazépane, lesdits cycles pouvant être substitués par un ou plusieurs radicaux alkyle en C₁-C₄ éventuellement substitués par un ou plusieurs hydroxy, amino, (di)alkylamino en C₁-C₂, carboxy, carboxamido ou sulfonamido.

Selon ce dernier mode de réalisation, R₁ et R₂ d'une part, et R₄ et R₅ d'autre part indépendamment les uns des autres forment avec l'atome d'azote auquel ils sont rattachés un hétérocycle choisi parmi la 2,5-diméthylpyrrolidine, l'acide pyrrolidine 2-carboxylique, l'acide 3-hydroxypyrrolidine 2-carboxylique, l'acide 4-hydroxypyrrolidine 2-carboxylique, la 2,4dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la -3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 4-amino-3-hydroxy pyrrolidine, la 3-hydroxy-4-(2-hydroxyéthyl)amino- pyrrolidine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, la homopipéridine, la 2-carboxyhomopipéridine, la 2-carboxamidohomopipéridine, le diazépane, le N-méthyl diazépane, le N-(2-hydroxyéthyl)-diazépane.

De préférence, R₁ et R₂ d'une part, et R₄ et R₅ d'autre part, indépendamment les uns des autres, forment ensemble un hétérocycle choisi parmi la pyrrolidine, la 3-hydroxypyrrolidine, la 3-aminopyrrolidine, la 3-acétamidopyrrolidine, la 3-(méthylsulfonylamino)-pyrrolidine, l'acide pyrrolidine-2-carboxylique, l'acide 3-hydroxypyrrolidine 2-carboxylique, la pipéridine, l'hydroxypipéridine, l'homopipéridine, le diazépane, la N-méthyl homopipérazine, la N β-hydroxyéthylhomopipérazine.

Parmi les composés de formule (I) utilisables selon l'invention , on peut citer :
la 2-méthoxy-6-(pyrrolidin-1-yl)-3-aminopyridine
la 1-(5-amino-6-méthoxy-pyridin-2-yl)-pyrrolidin-3-ol
la 3-amino-2-méthoxy-6-(morpholin-4-yl) pyridine,
la 3-amino-2-méthoxy-6-(pipéridin-1-yl)-pyridine,
la 6-N,N-(2-hydroxyéthyl)-méthyl)amino)-3-amino-2-méthoxypyridine
la 5,6-diamino-2-pyrrolidin-1-yl-pyridine
la N-(5,6-diamino-pyndm-2-yl)-3-hydroxy-pyrrolidine
la 2,6-di-(pyrrolidin-1-yl)-5-aminopyridine
la 6-(pyrrolidin-1-yl)-2,3-diaminopyridine
la 6-(3-hydroxypyrrolidin-1-yl)-2,3-diaminopyridine
la 6-(3-amino-pyrrolidin-1-yl)-2,3-diaminopyridine
la 6-(3-acétamido-pyrrolidin-1-yl)-2,3-diaminopyridine
la 6-(3,4-dihydroxy -pyrrolidin-1-yl)-2,3-diaminopyridine
la 6-(2-carboxy-pyrrolidin-1-yl)-2,3-diaminopyridine
la 6-(2-carboxy-3-hydroxy-pyrrolidin-1-yl)-2,3-diaminopyridine
la 6-(2-carboxy-4-hydroxy-pyrrolidin-1-yl)-2,3-diaminopyridine
la 6-(2-hydroxyméthyl-pyrrolidin-1-yl)-2,3-diaminopyridine
la 6-(2-hydroxyméthyl-4-hydroxy-pyrrolidin-1-yl)-2,3-diaminopyridine
la 6-(2-hydroxyméthyl-2-hydroxy-pyrrolidin-1-yl)-2,3-diaminopyridine
la 6-(2-carboxamido-pyrrolidin-1-yl)-2,3-diaminopyridine
la 6-(2-diméthylcarboxamido-pyrrolidin-1-yl)-2,3-diaminopyridine
la 6-(2-carboxamido-3-hydroxy-pyrrolidin-1-yl)-2,3-diaminopyridine
la 6-(2-carboxamido-4-hydroxy-pyrrolidin-1-yl)-2,3-diaminopyridine
la 6-(pyrrolidin-1-yl)-3-amino-2-méthylaminopyridine
la 6-(3-hydroxypyrrolidin-1-yl)-3-amino-2-méthylaminopyridine
la 6-(3-amino-pyrrolidin-1-yl)-3-amino-2-méthylaminopyridine
la 6-(3-acétatamido-pyrrolidin-1-yl)-3-amino-2-méthylaminopyridine
la 6-(3,4-dihydroxy -pyrrolidin-1-yl)-3-amino-2-méthylaminopyridine
la 6-(2-carboxy-pyrrolidin-1-yl)-3-amino-2-méthylaminopyridine
la 6-(2-carboxy-3-hydroxy-pyrrolidin-1-yl)-3-amino-2-méthylaminopyridine
la 6-(2-carboxy-4-hydroxy-pyrrolidin-1-yl)-3-amino-2-méthylaminopyridine
la 6-(2-hydroxyméthyl-pyrrolidin-1-yl)-3-amino-2-méthylaminopyridine
la 6-(2-hydroxyméthyl-4-hydroxy-pyrrolidin-1-yl)-3-amino-2-méthylaminopyridine
la 6-(2-hydroxyméthyl-2-hydroxy-pyrrolidin-1-yl)-3-amino-2-méthylaminopyridine
la 6-(2-carboxamido-pyrrolidin-1-yl)-3-amino-2-méthylaminopyridine
la 6-(2-diméthylcarboxamido-pyrrolidin-1-yl)-3-amino-2-méthylaminopyridine
la 6-(2-carboxamido-3-hydroxy-pyrrolidin-1-yl)-3-amino-2-méthylaminopyridine
la 6-(2-carboxamido-4-hydroxy-pyrrolidin-1-yl)-3-amino-2-méthylaminopyridine
la 6-(pyrrolidin-1-yl)-3-amino-2-éthylaminopyridine
la 6-(3-hydroxypyrrolidin-1-yl)-3-amino-2-éthylaminopyridine
la 6-(3-amino-pyrrolidin-1-yl)-3-amino-2-éthylaminopyridine
la 6-(3-acétamido-pyrrolidin-1-yl)-3-amino-2-éthylaminopyridine
la 6-(3,4-dihydroxy -pyrrolidin-1-yl)-3-amino-2-éthylaminopyridine
la 6-(2-carboxy-pyrrolidin-1-yl)-3-amino-2-éthylaminopyridine
la 6-(2-carboxy-3-hydroxy-pyrrolidin-1-yl)-3-amino-2-éthylaminopyridine
la 6-(2-carboxy-4-hydroxy-pyrrolidin-1-yl)-3-amino-2-éthylaminopyridine
la 6-(2-hydroxyméthyl-pyrrolidin-1-yl)-3-amino-2-éthylaminopyridine
la 6-(2-hydroxyméthyl-4-hydroxy-pyrrolidin-1-yl)-3-amino-2-éthylaminopyridine
la 6-(2-hydroxyméthyl-2-hydroxy-pyrrolidin-1-yl)-3-amino-2-éthylaminopyridine
la 6-(2-carboxamido-pyrrolidin-1-yl)-3-amino-2-éthylaminopyridine
la 6-(2-diméthylcarboxamido-pyrrolidin-1-yl)-3-amino-2-éthylaminopyridine
la 6-(2-carboxamido-3-hydroxy-pyrrolidin-1-yl)-3-amino-2-éthylaminopyridine
la 6-(2-carboxamido-4-hydroxy-pyrrolidin-1-yl)-3-amino-2-éthylaminopyridine
la 6-(pyrrolidin-1-yl)-3-amino-2-(2-hydroxyéthylamino)pyridine
la 6-(3-hydroxypyrrolidin-1-yl)-3-amino-2-(2-hydroxyéthylamino)pyridine
la 6-(3-amino-pyrrolidin-1-yl)-3-amino-2-(2-hydroxyéthylamino)pyridine
la 6-(3-acétamido-pyrrolidin-1-yl)-3-amino-2-(2-hydroxyéthylamino)pyridine
la 6-(3,4-dihydroxy -pyrrolidin-1-yl)-3-amino-2-(2-hydroxyéthylamino)pyridine
la 6-(2-carboxy-pyrrolidin-1-yl)-3-amino-2-(2-hydroxyéthylamino)pyridine
la 6-(2-carboxy-3-hydroxy-pyrrolidin-1-yl)-3-amino-2-(2-hydroxyéthylamino)pyridine
la 6-(2-carboxy-4-hydroxy-pyrrolidin-1-yl)-3-amino-2-(2-hydroxyéthylamino)pyridine
la 6-(2-hydroxyméthyl-pyrrolidin-1-yl)-3-amino-2-(2-hydroxyéthylamino)pyridine
la 6-(2-hydroxyméthyl-4-hydroxy-pyrrolidin-1-yl)-3-amino-2-(2-hydroxyéthylamino)pyridine
la 6-(2-hydroxyméthyl-2-hydroxy-pyrrolidin-1-yl)-3-amino-2-(2-hydroxyéthylamino)pyridine
la 6-(2-carboxamido-pyrrolidin-1-yl)-3-amino-2-(2-hydroxyéthylamino)pyridine
la 6-(2-diméthylcarboxamido-pyrrolidin-1-yl)-3-amino-2-(2-hydroxyéthylamino)pyridine
la 6-(2-carboxamido-3-hydroxy-pyrrolidin-1-yl)-3-amino-2-(2-hydroxyéthylamino)pyridine
la 6-(2-carboxamido-4-hydroxy-pyrrolidin-1-yl)-3-aminopyridine
la 6-(pyrrolidin-1-yl)-3-amino-2-(2-aminoéthylamino)pyridine
la 6-(3-hydroxypyrrolidin-1-yl)-3-amino-2-(2-aminoéthylamino)pyridine
la 6-(3-amino-pyrrolidin-1-yl)-3-amino-2-(2-aminoéthylamino)pyridine
la 6-(3-acétamido-pyrrolidin-1-yl)-3-amino-2-(2-aminoéthylamino)pyridine
la 6-(3,4-dihydroxy -pyrrolidin-1-yl)-3-amino-2-(2-aminoéthylamino)pyridine
la 6-(2-carboxy-pyrrolidin-1-yl)-3-amino-2-(2-aminoéthylamino)pyridine
la 6-(2-carboxy-3-hydroxy-pyrrolidn-1-yl)-3-amino-2-(2-aminoéthylamino)pyridine
la 6-(2-carboxy-4-hydroxy-pyrrolidin-1-yl)-3-amino-2-(2-aminoéthylamino)pyridine
la 6-(2-hydroxyméthyl-pyrrolidin-1-yl)-3-amino-2-(2-aminoéthylamino)pyridine
la 6-(2-hydroxyméthyl-4-hydroxy-pyrrolidin-1-yl)-3-amino-2-(2-aminoéthylamino)pyridine
la 6-(2-hydroxyméthyl-2-hydroxy-pyrrolidin-1-yl)-3-amino-2-(2-aminoéthylamino)pyridine
la 6-(2-carboxamido-pyrrolidin-1-yl)-3-amino-2-(2-aminoéthylamino)pyridine
la 6-(2-diméthylcarboxamido-pyrrolidin-1-yl)-3-amino-2-(2-aminioéthylamino)pyridine
la 6-(2-carboxamido-3-hydroxy-pyrrolidin-1-yl)-3-amino- 2-(2-aminoéthylamino)pyridine
la 6-(2-carboxamido-4-hydroxy-pyrrolidin-1-yl)-3-amino-2-(2-aminoéthylamino)pyridine
la 6-(pyrrolidin-1-yl)-3-amino-2-(2-acétamidoéthylamino)pyridine
la 6-(3-hydroxypyrrolidin-1-yl)-3-amino-2-(2-acétamidoéthylamino)pyridine
la 6-(3-amino-pyrrolidin-1-yl)-3-amino-2-(2-acétamidoéthylamino)pyridine
la 6-(3-acétamido-pyrrolidin-1-yl)-3-amino-2-(2-acétamidoéthylamino)pyridine
la 6-(3,4-dihydroxy -pyrrolidin-1-yl)-3-amino-2-(2-acétamidoéthylamino)pyridine
la 6-(2-carboxy-pyrrolidin-1-yl)-3-amino-2-(2-acétamidoéthylamino)pyridine
la 6-(2-carboxy-3-hydroxy-pyrrolidin-1-yl)-3-amino-2-(2-acétamidoéthylamino)pyridine
la 6-(2-carboxy-4-hydroxy-pyrrolidin-1-yl)-3-amino-2-(2-acétamidoéthylamino)pyridine
la 6-(2-hydroxyméthyl-pyrrolidin-1-yl)-3-amino-2-(2-acétamidoéthylamino)pyridine
la 6-(2-hydroxyméthyl-4-hydroxy-pyrrolidin-1-yl)-3-amino-2-(2-acétamidoéthylamino)pyridine
la 6-(2-hydroxyméthyl-2-hydroxy-pyrrolidin-1-yl)-3-amino-2-(2-acétamidoéthylamino)pyridine
la 6-(2-carboxamido-pyrrolidin-1-yl)-3-amino-2-(2-acétamidoéthylamino)pyridine
la 6-(2-diméthylcarboxamido-pyrrolidin-1-yl)-3-amino-2-(2-acétamidoéthylamino)pyridine
la 6-(2-carboxamido-3-hydroxy-pyrrolidin-1-yl)-3-amino-2-(2-acétamidoéthylamino)pyridine
la 6-(2-carboxamido-4-hydroxy-pyrrolidin-1-yl)-3-amino-2-(2-acétamidoéthylamino)pyridine
la 6-(pyrrolidin-1-yl)-3-amino-2-(pyrrolidin-1-yl)-pyridine
la 6-(3-hydroxypyrrolidin-1-yl)-3-amino-2-(pyrrolidin-1-yl)-pyridine
la 6-(3-amino-pyrrolidin-1-yl)-3-amino-2-(pyrrolidin-1-yl)-pyridine
la 6-(3-acétamido-pyrrolidin-1-yl)-3-amino-2-(pyrrolidin-1-yl)-pyridine
la 6-(3,4-dihydroxy -pyrrolidin-1-yl)-3-amino-2-(pyrrolidin-1-yl)-pyridine
la 6-(2-carboxy-pyrrolidin-1-yl)-3-amino-2-(pyrrolidin-1-yl)-pyridine
la 6-(2-carboxy-3-hydroxy-pyrrolidin-1-yl)-3-amino-2-(pyrrolidin-1-yl)-pyridine
la 6-(2-carboxy-4-hydroxy-pyrrolidin-1-yl)-3-amino-2-(pyrrolidin-1-yl)-pyridine
la 6-(2-hydroxyméthyl-pyrrolidin-1-yl)-3-amino-2-(pyrrolidin-1-yl)-pyridine
la 6-(2-hydroxyméthyl-4-hydroxy-pyrrolidin-1-yl)-3-amino-2-(pyrrolidin-1-yl)-pyridine
la 6-(2-hydroxyméthyl-2-hydroxy-pyrrolidin-1-yl)-3-amino-2-(pyrrolidin-1-yl)-pyridine
la 6-(2-carboxamido-pyrrolidin-1-yl)-3-amino-2-(pyrrolidin-1-yl)-pyridine
la 6-(2-diméthylcarboxamido-pyrrolidin-1-yl)-3-amino-2-(pyrrolidin-1-yl)-pyridine
la 6-(2-carboxamido-3-hydroxy-pyrrolidin-1-yl)-3-amino-2-(pyrrolidin-1-yl)-pyridine
la 6-(2-carboxamido-4-hydroxy-pyrrolidin-1-yl)-3-amino-2-(pyrrolidin-1-yl)-pyridine
la 6-(pyrrolidin-1-yl)-3-amino-2-(3-hydroxy-pyrrolidin-1-yl)-pyridine
la 6-(3-hydroxypyrrolidin-1-yl)-3-amino-2-(3-hydroxy-pyrrolidin-1-yl)-pyridine
la 6-(3-amino-pyrrolidin-1-yl)-3-amino-2-(3-hydroxy-pyrrolidin-1-yl)-pyridine
la 6-(3-acétamido-pyrrolidin-1-yl)-3-amino-2-(3-hydroxy-pyrrolidin-1-yl)-pyridine
la 6-(3,4-dihydroxy -pyrrolidin-1-yl)-3-amino-2-(3-hydroxy-pyrrolidin-1-yl)-pyridine
la 6-(2-carboxy-pyrrolidin-1-yl)-3-amino-2-(3-hydroxy-pyrrolidin-1-yl)-pyridine
la 6-(2-carboxy-3-hydroxy-pyrrolidin-1-yl)-3-amino-2-(3-hydroxy-pyrrolidin-1-yl)-pyridine
la 6-(2-carboxy-4-hydroxy-pyrrolidin-1-yl)-3-amino-2-(3-hydroxy-pyrrolidin-1-yl)-pyridine
la 6-(2-hydroxyméthyl-pyrrolidin-1-yl)-3-amino-2-(3-hydroxy-pyrrolidin-1-yl)-pyridine
la 6-(2-hydroxyméthyl-4-hydroxy-pyrrolidin-1-yl)-3-amino-2-(3-hydroxy-pyrrolidin-1-yl)-pyridine
la 6-(2-hydroxyméthyl-2-hydroxy-pyrrolidin-1-yl)-3-amino-2-(3-hydroxy-pyrrolidin-1-yl)-pyridine
la 6-(2-carboxamido-pyrrolidin-1-yl)-3-amino-2-(3-hydroxy-pyrrolidin-1-yl)-pyridine
la 6-(2-diméthylcarboxamido-pyrrolidin-1-yl)-3-amino-2-(3-hydroxy-pyrrolidin-1-yl)-pyridine
la 6-(2-carboxamido-3-hydroxy-pyrrolidin-1-yl)-3-amino-2-(3-hydroxy-pyrrolidin-1-yl)-pyridine
la 6-(2-carboxamido-4-hydroxy-pyrrolidin-1-yl)-3-amino-2-(3-hydroxy-pyrrolidin-1-yl)-pyridine
la 6-(pyrrolidin-1-yl)-3-amino-2-(2-carboxy-pyrrolidin-1-yl)-pyridine
la 6-(3-hydroxypyrrolidin-1-yl)-3-amino-2-(2-carboxy-pyrrolidin-1-yl)-pyridine
la 6-(3-amino-pyrrolidin-1-yl)-3-amino-2-(2-carboxy-pyrrolidin-1-yl)-pyridine
la 6-(3-acétamido-pyrrolidin-1-yl)-3-amino-2-(2-carboxy-pyrrolidin-1-yl)-pyridine
la 6-(3,4-dihydroxy -pyrrolidin-1-yl)-3-amino-2-(2-carboxy-pyrrolidin-1-yl)-pyridine
la 6-(2-carboxy-pyrrolidin-1-yl)-3-amino-2-(2-carboxy-pyrrolidin-1-yl)-pyridine
la 6-(2-carboxy-3-hydroxy-pyrrolidin-1-yl)-3-amino-2-(2-carboxy-pyrrolidin-1-yl)-pyridine
la 6-(2-carboxy-4-hydroxy-pyrrolidin-1-yl)-3-amino-2-(2-carboxy-pyrrolidin-1-yl)-pyridine
la 6-(2-hydroxyméthyl-pyrrolidin-1-yl)-3-amino-2-(2-carboxy-pyrrolidin-1-yl)-pyridine
la 6-(2-hydroxyméthyl-4-hydroxy-pyrrolidin-1-yl)-3-amino-2-(2-carboxy-pyrrolidin-1-yl)-pyridine
la 6-(2-hydroxyméthyl-2-hydroxy-pyrrolidin-1-yl)-3-amino-2-(2-carboxy-pyrrolidin-1-yl)-pyridine
la 6-(2-carboxamido-pyrrolidin-1-yl)-3-amino-2-(2-carboxy-pyrrolidin-1-yl)-pyridine
la 6-(2-diméthylcarboxamido-pyrrolidin-1-yl)-3-amino-2-(2-carboxy-pyrrolidin-1-yl)-pyridine
la 6-(2-carboxamido-3-hydroxy-pyrrolidin-1-yl)-3-amino-2-(2-carboxy-pyrrolidin-1-yl)-pyridine
la 6-(2-carboxamido-4-hydroxy-pyrrolidin-1-yl)-3-amino-2-(2-carboxy-pyrrolidin-1-yl)-pyridine
la 6-(pyrrolidin-1-yl)-3-amino-2-(2-hydroxyméthyl-pyrrolidin-1-yl)-pyridine
la 6-(3-hydroxypyrrolidin-1-yl)-3-amino-2-(2-hydroxyméthyl-pyrrolidin-1-yl)-pyridine
la 6-(3-amino-pyrrolidin-1-yl)-3-amino-2-(2-hydroxyméthyl-pyrrolidin-1-yl)-pyridine
la 6-(3-acétamido-pyrrolidin-1-yl)-3-amino-2-(2-hydroxyméthyl-pyrrolidin-1-yl)-pyridine
la 6-(3,4-dihydroxy -pyrrolidin-1-yl)-3-amino-2-(2-hydroxyméthyl-pyrrolidin-1-yl)-pyridine
la 6-(2-carboxy-pyrrolidin-1-yl)-3-amino-2-(2-hydroxyméthyl-pyrrolidin-1-yl)-pyridine
la 6-(2-carboxy-3-hydroxy-pyrrolidin-1-yl)-3-amino-2-(2-hydroxyméthyl-pyrrolidin-1-yl)-pyridine
la 6-(2-carboxy-4-hydroxy-pyrrolidin-1-yl)-3-amino-2-(2-hydroxyméthyl-pyrrolidin-1-yl)-pyridine
la 6-(2-hydroxyméthyl-pyrrolidin-1-yl)-3-amino-2-(2-hydroxyméthyl-pyrrolidin-1-yl)-pyridine
la 6-(2-hydroxyméthyl-4-hydroxy-pyrrolidin-1-yl)-3-amino-2-(2-hydroxyméthyl-pyrrolidin-1-yl)-pyridine
la 6-(2-hydroxyméthyl-2-hydroxy-pyrrolidin-1-yl)-3-amino-2-(2-hydroxyméthyl-pyrrolidin-1-yl)-pyridine
la 6-(2-carboxamido-pyrrolidin-1-yl)-3-amino-2-(2-hydroxyméthyl-pyrrolidin-1-yl)-pyridine
la 6-(2-diméthylcarboxamido-pyrrolidin-1-yl)-3-amino-2-(2-hydroxyméthyl-pyrrolidin-1-yl)-pyridine
la 6-(2-carboxamido-3-hydroxy-pyrrolidin-1-yl)-3-amino-2-(2-hydroxyméthyl-pyrrolidin-1-yl)-pyridine
la 6-(2-carboxamido-4-hydroxy-pyrrolidin-1-yl)-3-amino-2-(2-hydroxyméthyl-pyrrolidin-1-yl)-pyridine
la 6-(pyrrolidin-1-yl)-3-amino-2-(2-carboxamido-pyrrolidin-1-yl)-pyridine
la 6-(3-hydroxypyrrolidin-1-yl)-3-amino-2-(2-carboxamido-pyrrolidin-1-yl)-pyridine
la 6-(3-amino-pyrrolidin-1-yl)-3-amino-2-(2-carboxamido-pyrrolidin-1-yl)-pyridine
la 6-(3-acétamido-pyrrolidin-1-yl)-3-amino-2-(2-carboxamido-pyrrolidin-1-yl)-pyridine
la 6-(3,4-dihydroxy -pyrrolidin-1-yl)-3-amino-2-(2-carboxamido-pyrrolidin-1-yl)-pyridine
la 6-(2-carboxy-pyrrolidin-1-yl)-3-amino-2-(2-carboxamido-pyrrolidin-1-yl)-pyridine
la 6-(2-carboxy-3-hydroxy-pyrrolidin-1-yl)-3-amino-2-(2-carboxamido-pyrrolidin-1-yl)-pyridine
la 6-(2-carboxy-4-hydroxy-pyrrolidin-1-yl)-3-amino-2-(2-carboxamido-pyrrolidin-1-yl)-pyridine
la 6-(2-hydroxyméthyl-pyrrolidin-1-yl)-3-amino-2-(2-carboxamido-pyrrolidin-1-yl)-pyridine
la 6-(2-hydroxyméthyl-4-hydroxy-pyrrolidin-1-yl)-3-amino-2-(2-carboxamido-pyrrolidin-1-yl)-pyridine
la 6-(2-hydroxyméthyl-2-hydroxy-pyrrolidin-1-yl)-3-amino-2-(2-carboxamido-pyrrolidin-1-yl)-pyridine
la 6-(2-carboxamido-pyrrolidin-1-yl)-3-amino-2-(2-carboxamido-pyrrolidin-1-yl)-pyridine
la 6-(2-diméthylcarboxamido-pyrrolidin-1-yl)-3-amino-2-(2-carboxamido-pyrrolidin-1-yl)-pyridine
la 6-(2-carboxamido-3-hydroxy-pyrrolidin-1-yl)-3-amino-2-(2-carboxamido-pyrrolidin-1-yl)-pyridine
la 6-(2-carboxamido-4-hydroxy-pyrrolidin-1-yl)-3-amino-2-(2-carboxamido-pyrrolidin-1-yl)-pyridine
la 6-(pyrrolidin-1-yl)-3-amino-2-diméthylaminopyridine
la 6-(3-hydroxypyrrolidin-1-yl)-3-amino-2-diméthylaminopyridine
la 6-(3-amino-pyrrolidin-1-yl)-3-amino-2-diméthylaminopyridine
la 6-(3-acétamido-pyrrolidin-1-yl)-3-amino-2-diméthylaminopyridine
la 6-(3,4-dihydroxy -pyrrolidin-1-yl)-3-amino-2-diméthylaminopyridine
la 6-(2-carboxy-pyrrolidin-1-yl)-3-amino-2-diméthylaminopyridine
la 6-(2-carboxy-3-hydroxy-pyrrolidin-1-yl)-3-amino-2-diméthylaminopyridine
la 6-(2-carboxy-4-hydroxy-pyrrolidin-1-yl)-3-amino-2-diméthylaminopyridine
la 6-(2-hydroxyméthyl-pyrrolidin-1-yl)-3-amino-2-diméthylaminopyridine
la 6-(2-hydroxyméthyl-4-hydroxy-pyrrolidin-1-yl)-3-amino-2-diméthylaminopyridine
la 6-(2-hydroxyméthyl-2-hydroxy-pyrrolidin-1-yl)-3-amino-2-diméthylaminopyridine
la 6-(2-carboxamido-pyrrolidin-1-yl)-3-amino-2-diméthylaminopyridine
la 6-(2-diméthylcarboxamido-pyrrolidin-1-yl)-3-amino-2-diméthylaminopyridine
la 6-(2-carboxamido-3-hydroxy-pyrrolidin-1-yl)-3-amino-2-diméthylaminopyridine
la 6-(2-carboxamido-4-hydroxy-pyrrolidin-1-yl)-3-amino-2-diméthylaminopyridine
la 6-(pyrrolidin-1-yl)-3-amino-2-[méthyl-(2-hydroxyéthyl)amino]pyridine
la 6-(3-hydroxypyrrolidin-1-yl)-3-amino-2-[méthyl-(2-hydroxyéthyl)amino]pyridine
la 6-(3-amino-pyrrolidin-1-yl)-3-ammo-2-[méthyl-(2-hydroxyéthyl)amino]pyridine
la 6-(3-acétamido-pyrrolidin-1-yl)-3-amino-2-[méthyl-(2-hydroxyéthyl)amino]pyridine
la 6-(3,4-dihydroxy -pyrrolidin-1-yl)-3-amino-2-[méthyl-(2-hydroxyéthyl)amino]pyridine
la 6-(2-carboxy-pyrrolidin-1-yl)-3-amino-2-[méthyl-(2-hydroxyéthyl)amino]pyridine
la 6-(2-carboxy-3-hydroxy-pyrrolidin-1-yl)-3-amino-2-[méthyl-(2-hydroxyéthyl)amino]pyridine
la 6-(2-carboxy-4-hydroxy-pyrrolidin-1-yl)-3-amino-2-[méthyl-(2-hydroxyéthyl)amino]pyridine
la 6-(2-hydroxyméthyl-pyrrolidin-1-yl)-3-amino-2-[méthyl-(2-hydroxyéthyl)amino]pyridine
la 6-(2-hydroxyméthyl-4-hydroxy-pyrrolidin-1-yl)-3-amino-2-[méthyl-(2-hydroxyéthyl)amino]pyridine
la 6-(2-hydroxyméthyl-2-hydroxy-pyrrolidin-1-yl)-3-amino-2-[méthyl-(2-hydroxyéthyl)amino]pyridine
la 6-(2-carboxamido-pyrrolidin-1-yl)-3-amino-2-[méthyl-(2-hydroxyéthyl)amino]pyridine la 6-(2-diméthylcarboxamido-pyrrolidin-1-yl)-3-amino-2-[méthyi-(2-hydroxyéthyl)amino]pyridine
la 6-(2-carboxamido-3-hydroxy-pyrrolidin-1-yl)-3-amino-2-[méthyl-(2-hydroxyéthyl)amino]pyridine
la 6-(2-carboxamido-4-hydroxy-pyrrolidin-1-yl)-3-amino-2-[méthyl-(2-hydroxyéthyl)amino]pyridine
la 6-(pyrrolidin-1-yl)-3-amino-2-[méthyl-(2-aminoéthyl)amino]pyridine
la 6-(3-hydroxypyrrolidin-1-yl)-3-amino-2-[méthyl-(2-aminoéthyl)amino]pyridine
la 6-(3-amino-pyrrolidin-1-yl)-3-amino-2-[méthyl-(2-aminoéthyl)amino]pyridine
la 6-(3-acétamido-pyrrolidin-1-yl)-3-amino-2-[méthyl-(2-aminoéthyl)amino]pyridine
la 6-(3,4-dihydroxy -pyrrolidin-1-yl)-3-amino-2-[méthyl-(2-aminoéthyl)amino]pyridine
la 6-(2-carboxy-pyrrolidin-1-yl)-3-amino-2-[méthyl-(2-aminoéthyl)amino]pyridine
la 6-(2-carboxy-3-hydroxy-pyrrolidin-1-yl)-3-amino-2-[méthyl-(2-aminoéthyl)amino]pyridine
la 6-(2-carboxy-4-hydroxy-pyrrolidin-1-yl)-3-amino-2-[méthyl-(2-aminoéthyl)amino]pyridine
la 6-(2-hydroxyméthyl-pyrrolidin-1-yl)-3-amino-2-[méthyl-(2-aminoéthyl)amino]pyridine
la 6-(2-hydroxyméthyl-4-hydroxy-pyrrolidin-1-yl)-3-amino-2-[méthyl-(2-aminoéthyl)amino]pyridine
la 6-(2-hydroxyméthyl-2-hydroxy-pyrrolidin-1-yl)-3-amino-2-[methyl-(2-aminoéthyl)amino]pyridine
la 6-(2-carboxamido-pyrrolidin-1-yl)-3-amino-2-[méthyl-(2-aminoéthyl)amino]pyridine
la 6-(2-diméthylcarboxamido-pyrrolidin-1-yl)-3-amino-2-[méthyl-(2-aminoéthyl)amino]pyridine
la 6-(2-carboxamido-3-hydroxy-pyrrolidin-1-yl)-3-amino-2-[méthyl-(2-aminoéthyl)amino]pyridine
la 6-(2-carboxamido-4-hydroxy-pyrrolidin-1-yl)-3-amino-2-[méthyl-(2-aminoéthyl)amino]pyridine
la 6-(pyrrolidin-1-yl)-3-amino-2-[méthyl-(2-acétamidoéthyl)amino]pyridine
la 6-(3-hydroxypyrrolidin-1-yl)-3-amino-2-[méthyl-(2-acétamidoéthyl)amino]pyridine
la 6-(3-amino-pyrrolidin-1-yl)-3-amino-2-[méthyl-(2-acétamidoéthyl)amino]pyridine
la 6-(3-acétamido-pyrrolidin-1-yl)-3-amino-2-[méthyl-(2-acétamidoéthyl)amino]pyridine
la 6-(3,4-dihydroxy -pyrrolidin-1-yl)-3-amino-2-[méthyl-(2-acétamidoéthyl)amino]pyridine
la 6-(2-carboxy-pyrrolidin-1-yl)-3-amino-2-[méthyl-(2-acétamidoéthyl)amino]pyridine
la 6-(2-carboxy-3-hydroxy-pyrrolidin-1-yl)-3-amino-2-[méthyl-(2-acétamidoéthyl)amino]pyridine
la 6-(2-carboxy-4-hydroxy-pyrrolidin-1-yl)-3-amino-2-[méthyl-(2-acétamidoéthyl)amino]pyridine
la 6-(2-hydroxyméthyl-pyrrolidin-1-yl)-3-amino-2-[méthyl-(2-acétamidoéthyl)amino]pyridine
la 6-(2-hydroxyméthyl-4-hydroxy-pyrrolidin-1-yl)-3-amino-2-[méthyl-(2-acétamidoéthyl)amino]pyridine
la 6-(2-hydroxyméthyl-2-hydroxy-pyrrolidin-1-yl)-3-amino-2-[méthyl-(2-acétamidoéthyl)amino]pyridine
la 6-(2-carboxamido-pyrrolidin-1-yl)-3-amino-2-[méthyl-(2-acétamidoéthyl)amino]pyridine
la 6-(2-diméthylcarboxamido-pyrrolidin-1-yl)-3-amino-2-[méthyl-(2-acétamidoéthyl)amino]pyridine
la 6-(2-carboxamido-3-hydroxy-pyrrolidin-1-yl)-3-amino-2-[méthyl-(2-acétamidoéthyl)amino]pyridine
la 6-(2-carboxamido-4-hydroxy-pyrrolidin-1-yl)-3-amino-2-[méthyl-(2-acétamidoéthyl)amino]pyridine
la 6-(pyrrolidin-1-yl)-3-amino-2-méthoxypyridine
la 6-(3-hydroxypyrrolidin-1-yl)-3-amino-2-méthoxypyridine
la 6-(3-amino-pyrrolidin-1-yl)-3-amino-2-méthoxypyridine
la 6-(3-acétamido-pyrrolidin-1-yl)-3-amino-2-méthoxypyridine
la 6-(3,4-dihydroxy -pyrrolidin-1-yl)-3-amino-2-méthoxypyridine
la 6-(2-carboxy-pyrrolidin-1-yl)-3-amino-2-méthoxypyridine
la 6-(2-carboxy-3-hydroxy-pyrrolidin-1-yl)-3-amino-2-méthoxypyridine
la 6-(2-carboxy-4-hydroxy-pyrrolidin-1-yl)-3-amino-2-méthoxypyridine
la 6-(2-hydroxyméthyl-pyrrolidin-1-yl)-3-amino-2-méthoxypyridine
la 6-(2-hydroxyméthyl-4-hydroxy-pyrrolidin-1-yl)-3-amino-2-méthoxypyridine
la 6-(2-hydroxyméthyl-2-hydroxy-pyrrolidin-1-yl)-3-amino-2-méthoxypyridine
la 6-(2-carboxamido-pyrrolidin-1-yl)-3-amino-2-méthoxypyridine
la 6-(2-diméthylcarboxamido-pyrrolidin-1-yl)-3-amino-2-méthoxypyridine
la 6-(2-carboxamido-3-hydroxy-pyrrolidin-1-yl)-3-amino-2-méthoxypyridine
la 6-(2-carboxamido-4-hydroxy-pyrrolidin-1-yl)-3-amino-2-méthoxypyridine
la 6-(pyrrolidin-1-yl)-3-amino-2-(2-hydroxyéthyloxy)pyridine
la 6-(3-hydroxypyrrolidin-1-yl)-3-amino-2-(2-hydroxyéthyloxy)pyridine
la 6-(3-amino-pyrrolidin-1-yl)-3-amino-2-(2-hydroxyéthyloxy)pyridine
la 6-(3-acétamido-pyrrolidin-1-yl)-3-amino-2-(2-hydroxyéthyloxy)pyridine
la 6-(3,4-dihydroxy -pyrrolidin-1-yl)-3-amino-2-(2-hydroxyéthyloxy)pyridine
la 6-(2-carboxy-pyrrolidin-1-yl)-3-amino-2-(2-hydroxyéthyloxy)pyridine
la 6-(2-carboxy-3-hydroxy-pyrrolidin-1-yl)-3-amino-2-(2-hydroxyéthyloxy)pyridine
la 6-(2-carboxy-4-hydroxy-pyrrolidin-1-yl)-3-amino-2-(2-hydroxyéthyloxy)pyridine
la 6-(2-hydroxyméthyl-pyrrolidin-1-yl)-3-amino-2-(2-hydroxyéthyloxy)pyridine
la 6-(2-hydroxyméthyl-4-hydroxy-pyrrolidin-1-yl)-3-amino-2-(2-hydroxyéthyloxy)pyridine
la 6-(2-hydroxyméthyl-2-hydroxy-pyrrolidin-1-yl)-3-amino-2-(2-hydroxyéthyloxy)pyridine
la 6-(2-carboxamido-pyrrolidin-1-yl)-3-amino-2-(2-hydroxyéthyloxy)pyridine
la 6-(2-diméthylcarboxamido-pyrrolidin-1-yl)-3-amino-2-(2-hydroxyéthyloxy)pyridine
la 6-(2-carboxamido-3-hydroxy-pyrrolidin-1-yl)-3-amino-2-(2-hydroxyéthyloxy)pyridine
la 6-(2-carboxamido-4-hydroxy-pyrrolidin-1-yl)-3-amino-2-(2-hydroxyéthyloxy)pyridine
la 6-(pyrrolidin-1-yl)-3-amino-2-éthoxypyridine
la 6-(3-hydroxypyrrolidin-1-yl)-3-amino-2-éthoxypyridine
la 6-(3-amino-pyrrolidin-1-yl)-3-amino-2-éthoxypyridine
la 6-(3-acétamido-pyrrolidin-1-yl)-3-amino-2-éthoxypyridine
la 6-(3,4-dihydroxy -pyrrolidin-1-yl)-3-amino-2-éthoxypyridine
la 6-(carboxy-pyrrolidin-1-yl)-3-amino-2-éthoxypyridine
la 6-(2-carboxy-3-hydroxy-pyrrolidin-1-yl)-3-amino-2-éthoxypyridine
la 6-(2-carboxy-4-hydroxy-pyrrolidin-1-yl)-3-amino-2-éthoxypyridine
la 6-(2-hydroxyméthyl-pyrrolidin-1-yl)-3-amino-2-éthoxypyridine
la 6-(2-hydroxyméthyl-4-hydroxy-pyrrolidin-1-yl)-3-amino-2-éthoxypyridine
la 6-(2-hydroxyméthyl-2-hydroxy-pyrrolidin-1-yl)-3-amino-2-éthoxypyridine
la 6-(2-carboxamido-pyrrolidin-1-yl)-3-amino-2-éthoxypyridine
la 6-(2-diméthylcarboxamido-pyrrolidin-1-yl)-3-amino-2-éthoxypyridine
la 6-(2-carboxamido-3-hydroxy-pyrrolidin-1-yl)-3-amino-2-éthoxypyridine
la 6-(2-carboxamido-4-hydroxy-pyrrolidin-1-yl)-3-amino-2-éthoxypyridine
ainsi que leurs sels d'addition.

Parmi les composés de formule (I) utilisables selon l'invention , on utilisera plus particulièrement :
la 2-méthoxy-6-(pyrrolidin-1-yl)-3-aminopyridine
la 5,6-diamino-2-pyrrolidin-1-yl-pyridine
la N-(5,6-diamino-pyridin-2-yl)-3-hydroxy-pyrrolidine
la 2,6-di-(pyrrolidin-1-yl)-5-aminopyridine,
ainsi que leurs sels d'addition.

La composition de la présente invention peut en outre comprendre une ou plusieurs bases d'oxydation additionnelles classiquement utilisées en teinture d'oxydation autres que celles décrites précédemment. A titre d'exemple, ces bases d'oxydation additionnelles sont choisies parmi les bases d'oxydation, par exemple les paraphénylènediamines, les bis-phénylalkylènediamines, les para-aminophénols, les ortho-aminophénols, les bases hétérocycliques autres que celles décrites précédemment et leur sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 3-hydroxy-1-(4'-amino-phényl)pyrrolidine, la 2-thiényl paraphénylènediamine, le 2-β hydroxyéthylamino 5-amino toluène et leurs sels d'addition .

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques autres que celles de l'invention, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition.

Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N7, N 7-tetramethyl pyrazolo-[1,5-a]-pyrymidine, la 3-amino-5-méthyl-7-imidazolylpropylamino pyrazolo-[1,5-a]-pyrimidine et leurs sels d'addition et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

La ou les bases d'oxydation de formule (I) ou la ou les bases d'oxydation additionnelles sont en général chacune présentes en quantité comprise entre 0,001 et 10 % en poids environ du poids total de la composition tinctoriale, de préférence entre 0,005 et 6 %.

La composition selon l'invention peut contenir un ou plusieurs coupleurs conventionnellement utilisés pour la teinture de fibres kératiniques. Parmi ces coupleurs, on peut notamment citer les métaphénylènediamines, les métaaminophénols, les métadiphénols, les coupleurs naphtaléniques, les coupleurs hétérocycliques et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Dans la composition de la présente invention, le ou les coupleurs sont généralement présents en quantité comprise entre 0,001 et 10 % en poids environ du poids total de la composition tinctoriale et préférentiellement de 0,005 à 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs cationiques, les colorants directs azoiques, les colorants directs méthiniques.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges. Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 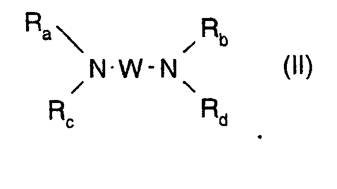 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Selon le procédé de teinture de la présente invention, on applique sur les fibres la composition selon la présente invention, et on révèle la couleur à l'aide d'un agent oxydant. La couleur peut être révélée à pH acide, neutre ou alcalin et l'agent oxydant peut être mélangé à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment pour la composition de l'invention.

Le pH de la composition oxydante renfermant l'agent oxydant est tel qu'après mélange avec la composition tinctoriale, le pH de la composition résultante appliquée sur les fibres kératiniques varie entre 3 et 12 environ, et préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Un autre objet de l'invention est un dispositif à plusieurs compartiments ou "kit" de teinture dans lequel un premier compartiment contient la composition tinctoriale de l'invention définie ci-dessus et un deuxième compartiment contient une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

Enfin l'invention a également pour objet le produit coloré susceptible d'être obtenu par condensation oxydative de la composition tinctoriale de la présente invention, en particulier d'une composition comprenant au moins une base d'oxydation de formule (I) telle que définie ci-dessus et éventuellement en présence d'au moins un coupleur et/ou d'au moins une base d'oxydation additionnelle, en présence d'au moins un agent oxydant.

Ces produits colorés peuvent notamment se présenter sous la forme de pigments et être utilisés à titre de colorant direct pour la teinture directe des cheveux ou bien encore être incorporés dans des produits cosmétiques tels que par exemple dans des produits de maquillage.

Parmi les composés de formule (I) tels que définis précédemment, un certain nombre sont déjà connus en eux mêmes et décrits dans les brevets FR1546317 et US 3,359,168 : la 2,3,6-triaminopyridine, dans la référence Khim. -Farm. Zh. (1982), 16(11), 1329-32 : la 6-(4-morpholinyl)-2,3-diaminopyridine et la 6-(1-piperidinyl)-2,3-diaminopyridine et dans la référence : la 2,6-di-(4-morpholinyl)-3-aminopyridine. Les autres composés de formule (I) sont nouveaux et constituent un autre objet de l'invention ; il s'agit des dérivés de 2,5-diaminopyridine de formule (I'). 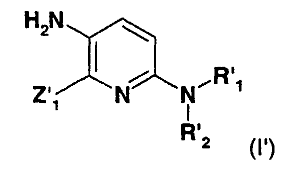 dans laquelle :
Z'₁ représente un radical OR'₃ ou NR'₄R'₅ ;
R'₁, R'₂, R'₄ et R'₅ représentent, indépendamment l'un de l'autre, un atome d'hydrogène, un radical (poly)aminoalkyle en C₂-C₆, un radical (poly)alkylaminoalkyle en C₂-C₆, un radical amino-hydroxyalkyle en C₂-C₆, un radical acylaminoalkyle en C₂-C₆, un radical alcoxyalkyle en C₂-C₆, un radical hydroxyalcoxyalkyle en C₂-C₆, un radical aminoalcoxyalkyle en C₂-C_{6,} un radical (poly)hydroxyalkyle en C₂-C_{6,} un radical carboxyalkyle en C₂-C₆, un radical sulfonylalkyle en C₂-C₆ ;
R'₁ et R'₂ d'une part, et R'₄ et R'₅ d'autre part, indépendamment les uns des autres, forment avec l'atome d'azote auquel ils sont rattachés un hétérocycle saturé comportant 5 à 8 chaînons, éventuellement substitué par un ou plusieurs radicaux choisis dans le groupe constitué par les atomes d'halogène, les radicaux amino, (di)alkylamino, hydroxy, carboxy, carboxamido, alcoxy, les radicaux alkyle en C₁-C₆ éventuellement substitués par un ou plusieurs radicaux hydroxy, amino, (di)-alkylamino, alcoxy, hydroxyalcoxy, aminoalcoxy, carboxy, sulfonyl, l'hétérocycle pouvant contenir un ou plusieurs hétéroatomes additionnels choisis parmi l'oxygène, l'azote éventuellement substitué, le soufre ou un groupement SO₂, l'hétérocycle ne comportant pas de liaison peroxyde, ni de radicaux diazo ou nitroso ;
R'₃ représente un radical alkyle en C₁-C₆, linéaire ou ramifié, pouvant être substitué par un ou plusieurs radicaux choisis parmi les radicaux hydroxy, amino, un radical mono- ou di-alkylamino en C₁-C₃ dont la partie alkyle est éventuellement substituée par un ou plusieurs groupements hydroxy, amino, alcoxyalcoxy en C₁-C₂, carboxy ; à l'exception de la 2,3,6-triaminopyridine, de la 6-(4-morpholinyl)-2,3-diaminopyridine, de la 6-(1-piperidinyl)-2,3-diaminopyridine et de la 2,6-di-(4-morpholinyl)-3-aminopyridine ainsi que de leurs sels d'addition.

On peut citer les composés particulièrement préférés suivants :
la 2-méthoxy-6-(pyrrolidin-1-yl)-3-aminopyridine
la 1-(5-amino-6-méthoxy-pyridin-2-yl)-pyrrolidin-3-ol
la 3-amino-2-méthoxy-6-(morpholin-4-yi) pyridine,
la 3-amino-2-méthoxy-6-(pipéridin-1-yl)-pyridine,
la 6-N,N-(2-hydroxyéthyl)-méthyl)amino)-3-amino-2-méthoxypyridine
la 5,6-diamino-2-pyrrolidin-1-yl-pyridine
la N-(5,6-diamino-pyridin-2-yl)-3-hydroxy-pyrrolidine
la 2,6-di-(pyrrolidin-1 -yl)-5-aminopyridine
la 6-(pyrrolidin-1-yl)-2,3-diaminopyndine
la 6-(3-hydroxypyrrolidin-1-yl)-2,3-diaminopyridine
la 6-(3-amino-pyrrolidin-1-yl)-2,3-diaminopyridine
la 6-(3-acétamido-pyrrolidin-1-yl)-2,3-diaminopyridine
la 6-(3,4-dihydroxy -pyrrolidin-1-yl)-2,3-diaminopyridine
la 6-(2-carboxy-pyrrolidin-1-yl)-2,3-diaminopyridine
la 6-(2-carboxy-3-hydroxy-pyrrolidin-1-yl)-2,3-diaminopyridine
la 6-(2-carboxy-4-hydroxy-pyrrolidin-1-yl)-2,3-diaminopyridine
la 6-(2-hydroxyméthyl-pyrrolidin-1-yl)-2,3-diaminopÿPldirae
la 6-(2-hydroxyméthyl-4-hydroxy-pyrrolidin-1-yl)-2,3-diaminopyridine
la 6-(2-hydroxyméthyl-2-hydroxy-pyrrolidin-1-yl)-2,8-diaminopyridine
la 6-(2-carboxamido-pyrrolidin-1-yl)-2,3-diaminopyridine
la 6-(2-diméthylcarboxamido-pyrrolidin-1-yl)-2,3-diaminopyridine
la 6-(2-carboxamido-3-hydroxy-pyrrolidin-1-yl)-2,3-diaminopyridine
la 6-(2-carboxamido-4-hydroxy-pyrrolidin-1-yl)-2,3-diaminopyridine
la 6-(pyrrolidin-1-yl)-3-amino-2-(2-hydroxyéthylamino)pyridine
la 6-(3-hydroxypyrrolidin-1-yl)-3-amino-2-(2-hydroxyéthylamino)pyridine
la 6-(3-amino-pyrrolidn-1-yl)-3-amino-2-(2-hydroxyéthylamino)pyridine
la 6-(3-acétamido-pyrrolidin-1-yl)-3-amino-2-(2-hydroxyéthylamino)pyridine
la 6-(3,4-dihydroxy -pyrrolidin-1-yl)-3-amino-2-(2-hydroxyéthylamino)pyridine
la 6-(2-carboxy-pyrrolidin-1-yl)-3-amino-2-(2-hydroxyéthylamino)pyridine
la 6-(2-carboxy-3-hydroxy-pyrrolidin-1-yl)-3-amino-2-(2-hydroxyéthylamino)pyridine
la 6-(2-carboxy-4-hydroxy-pyrrolidin-1-yl)-3-amino-2-(2-hydroxyéthylamino)pyridine
la 6-(2-hydroxyméthyl-pyrrolidin-1-yl)-3-amino-2-(2-hydroxyéthylamino)pyridine
la 6-(2-hydroxyméthyl-4-hydroxy-pyrrolidin-1-yl)-3-amino-2-(2-hydroxyéthylamino)pyridine
la 6-(2-hydroxyméthyl-2-hydroxy-pyrrolidin-1-yl)-3-amino-2-(2-hydroxyéthylamino)pyridine
la 6-(2-carboxamido-pyrrolidin-1-yl)-3-amino-2-(2-hydroxyéthylamino)pyridine
la 6-(2-diméthylcarboxamido-pyrrolidin-1-yl)-3-amino-2-(2-hydroxyéthylamino)pyridine
la 6-(2-carboxamido-3-hydroxy-pyrrolidin-1-yl)-3-amino-2-(2-hydroxyéthylamino)pyridine
la 6-(2-carboxamido-4-hydroxy-pyrrolidin-1-yl)-3-aminopyridinela 6-(pyrrolidin-1-yl)-3-amino-2-(2-aminoéthylamino)pyridine
la 6-(3-hydroxypyrrolidin-1-yl)-3-amino-2-(2-aminoéthylamino)pyridine
la 6-(3-amino-pyrrolidin-1-yl)-3-amino-2-(2-aminoéthylamino)pyridine
la 6-(3-acétamido-pyrrolidin-1-yl)-3-amino-2-(2-aminoéthylamino)pyridine
la 6-(3,4-dihydroxy -pyrrolidin-1 -yl)-3-amino-2-(2-aminoéthylamino)pyridine
la 6-(2-carboxy-pyrrolidin-1-yl)-3-amino-2-(2-aminoéthylamino)pyridine
la 6-(2-carboxy-3-hydroxy-pyrrolidin-1-yl)-3-amino-2-(2-aminoéthylamino)pyridine
la 6-(2-carboxy-4-hydroxy-pyrrolidin-1-yl)-3-amino-2-(2-aminoéthylamino)pyridine
la 6-(2-hydroxyméthyl-pyrrolidin-1-yl)-3-amino-2-(2-aminoéthylamino)pyridine
la 6-(2-hydroxyméthyl-4-hydroxy-pyrrolidin-1-yl)-3-amino-2-(2-aminoéthylamino)pyridine
la 6-(2-hydroxyméthyl-2-hydroxy-pyrrolidin-1 -yi)-3-amino-2-(2-aminoéthylamino)pyridine
la 6-(2-carboxamido-pyrrolidin-1-yl)-3-amino-2-(2-aminoéthylamino)pyridine
la 6-(2-diméthylcarboxamido-pyrrolidin-1-yl)-3-amino-2-(2-aminoéthylamino)pyridine
la 6-(2-carboxamido-3-hydroxy-pyrrolidin-1-yl)-3-amino-2-(2-aminoéthylamino)pyridine
la 6-(2-carboxamido-4-hydroxy-pyrrolidin-1-yl)-3-amino-2-(2-aminoéthylamino)pyridine
la 6-(pyrrolidin-1-yl)-3-amino-2-(2-acétamidoéthylamino)pyridine
la 6-(3-hydroxypyrrolidin-1-yl)-3-amino-2-(2-acétamidoéthylamino)pyridine
la 6-(3-amino-pyrrolidin-1-yl)-3-amino-2-(2-acétamidoéthylamino)pyridine
la 6-(3-acétamido-pyrrolidin-1-yl)-3-amino-2-(2-acétamidoéthylamino)pyridine
la 6-(3,4-dihydroxy -pyrrolidin-1-yl)-3-amino-2-(2-acétamidoéthylamino)pyridine
la 6-(2-carboxy-pyrrolidin-1-yl)-3-amino-2-(2-acétamidoéthylamino)pyridine
la 6-(2-carboxy-3-hydroxy-pyrrolidin-1-yl)-3-amino-2-(2-acétamidoéthylamino)pyridine
la 6-(2-carboxy-4-hydroxy-pyrrolidin-1-yl)-3-amino-2-(2-acétamidoéthylamino)pyridine
la 6-(2-hydroxyméthyl-pyrrolidin-1-yl)-3-amino-2-(2-acétamidoéthylamino)pyridine
la 6-(2-hydroxyméthyl-4-hydroxy-pyrrolidin-1-yl)-3-amino-2-(2-acétamidaéthylamino)pyridine
la 6-(2-hydroxyméthyl-2-hydroxy-pyrrolidin-1-yl)-3-amino-2-(2-acétamidoéthylamino)pyridine
la 6-(2-carboxamido-pyrrolidin-1-yl)-3-amino-2-(2-acétamidoéthylamino)pyridine
la 6-(2-diméthylcarboxamido-pyrrolidin-1-yl)-3-amino-2-(2-acétamidoéthyfamino)pyridine
la 6-(2-carboxamido-3-hydroxy-pyrrolidin-1-yl)-3-amino-2-(2-acétamidoéthylamino)pyridine
la 6-(2-carboxamido-4-hydroxy-pyrrolidin-1-yl)-3-amino-2-(2-acétamidoéthylamino)pyridine
la 6-(pyrrolidin-1-yl)-3-amino-2-(pyrrolidin-1-yl)-pyridine
la 6-(3-hydroxypyrrolidin-1-yl)-3-amino-2-(pyrrolidin-1-yl)-pyridine
la 6-(3-amino-pyrrolidin-1-yl)-3-amino-2-(pyrrolidin-1-yl)-pyridine
la 6-(3-acétamido-pyrrolidin-1-yl)-3-amino-2-(pyrrolidin-1-yl)-pyridine
la 6-(3,4-dihydroxy -pyrrolidin-1-yl)-3-amino-2-(pyrrolidin-1-yl)-pyridine
la 6-(2-carboxy-pyrrolidin-1-yl)-3-amino-2-(pyrrolidin-1-yl)-pyridine
la 6-(2-carboxy-3-hydroxy-pyrrolidin-1-yl)-3-amino-2-(pyrrolidin-1-yl)-pyridine
la 6-(2-carboxy-4-hydroxy-pyrrolidin-1-yl)-3-amino-2-(pyrrolidin-1-yl)-pyridine
la 6-(2-hydroxyméthyl-pyrrolidin-1-yl)-3-amino-2-(pyrrolidin-1-yl)-pyridine
la 6-(2-hydroxyméthyl-4-hydroxy-pyrrolidin-1-yl)-3-amino-2-(pyrrolidin-1-1-yl)-pyridine
la 6-(2-hydroxyméthyl-2-hydroxy-pyrrolidin-1-yl)-3-amino-2-(pyrrolidin-1-yl)-pyridine
la 6-(2-carboxamido-pyrrolidin-1-yl)-3-amino-2-(pyrrolidin-1-yl)-pyridine
la 6-(2-diméthylcarboxamido-pyrrolidin-1-yl)-3-amino-2-(pyrrolidin-1-yl)-pyridine
la 6-(2-carboxamido-3-hydroxy-pyrrolidin-1-yl)-3-amino-2-(pyrrolidin-1-yl)-pyridine
la 6-(2-carboxamido-4-hydroxy-pyrrolidin-1-yl)-3-amino-2-(pyrrolidin-1-yl)-pyridine
la 6-(pyrrolidin-1-yl)-3-amino-2-(3-hydroxy-pyrrolidin-1-yl)-pyridine
la 6-(3-hydroxypyrrolidin-1-yl)-3-amino-2-(3-hydroxy-pyrrolidin-1-yl)-pyridine
la 6-(3-amino-pyrrolidin-1-yl)-3-amino-2-(3-hydroxy-pyrrolidin-1-yl)-pyridine
la 6-(3-acétamido-pyrrolidin-1-yl)-3-amino-2-(3-hydroxy-pyrrolidin-1-yl)-pyridine
la 6-(3,4-dihydroxy -pyrrolidin-1-yl)-3-amino-2-(3-hydroxy-pyrrolidin-1-yl)-pyridine
la 6-(2-carboxy-pyrrolidin-1-yl)-3-amino-2-(3-hydroxy-pyrrolidin-1-yl)-pyridine
la 6-(2-carboxy-3-hydroxy-pyrrolidin-1-yl)-3-amino-2-(3-hydroxy-pyrrolidin-1-yl)-pyridine
la 6-(2-carboxy-4-hydroxy-pyrrolidin-1-yl)-3-amino-2-(3-hydroxy-pyrrolidin-1-yl)-pyridine
la 6-(2-hydroxyméthyl-pyrrolidin-1-yl)-3-amino-2-(3-hydroxy-pyrrolidin-1-yl)-pyridine
la 6-(2-hydroxyméthyl-4-hydroxy-pyrrolidin-1-yl)-3-amino-2-(3-hydroxy-pyrrolidin-1-yl)-pyridine
la 6-(2-hydroxyméthyl-2-hydroxy-pyrrolidin-1-yl)-3-amino-2-(3-hydroxy-pyrrolidin-1-yl)-pyridine
la 6-(2-carboxamido-pyrrolidin-1-yl)-3-amino-2-(3-hydroxy-pyrrolidin-1-yl)-pyridine
la 6-(2-diméthylcarboxamido-pyrrolidin-1-yl)-3-amino-2-(3-hydroxy-pyrrolidin-1-yl)-pyridine
la 6-(2-carboxamido-3-hydroxy-pyrrolidin-1-yl)-3-amino-2-(3-hydroxy-pyrrolidin-1-yl)-pyridine
la 6-(2-carboxamido-4-hydroxy-pyrrolidin-1-yl)-3-amino-2-(3-hydroxy-pyrrolidin-1-yl)-pyridine
la 6-(pyrrolidin-1-yl)-3-amino-2-(2-carboxy-pyrrolidin-1-yl)-pyridine
la 6-(3-hydroxypyrrolidin-1-yl)-3-amino-2-(2-carboxy-pyrrolidin-1-yl)-pyridine
la 6-(3-amino-pyrrolidin-1-yl)-3-amino-2-(2-carboxy-pyrrolidin-1-yl)-pyridine
la 6-(3-acétamido-pyrrolidin-1-yl)-3-amino-2-(2-carboxy-pyrrolidin-1-yl)-pyridine
la 6-(3,4-dihydroxy-pyrrolldin-1-yl)-3-amino-2-(2-carboxy-pyrrolidin-1-yl)-pyridine
la 6-(2-carboxy-pyrrolidin-1-yl)-3-amino-2-(2-carboxy-pyrrolidin-1-yl)-pyridine
la 6-(2-carboxy-3-hydroxy-pyrrolidin-1-yl)-3-amino-2-(2-carboxy-pyrrolidin-1-yl)-pyridine
la 6-(2-carboxy-4-hydroxy-pyrrolidin-1-yl)-3-amino-2-(2-carboxy-pyrrolidin-1-yl)-pyridine
la 6-(2-hydroxyméthyl-pyrrolidin-1-yl)-3-amino-2-(2-carboxy-pyrrolidin-1-yl)-pyridine
la 6-(2-hydroxyméthyl-4-hydroxy-pyrrolidin-1-yl)-3-amino-2-(2-carboxy-pyrrolidin-1-yl)-pyridine
la 6-(2-hydroxyméthyl-2-hydroxy-pyrrolidin-1-yl)-3-amino-2-(2-carboxy-pyrrolidin-1-yl)-pyridine
la 6-(2-carboxamido-pyrrolidin-1-yl)-3-amino-2-(2-carboxy-pyrrolidin-1-yl)-pyridine
la 6-(2-diméthylcarboxamido-pyrrolidin-1-yl)-3-amino-2-(2-carboxy-pyrrolidin-1-yl)-pyridine
la 6-(2-carboxamido-3-hydroxy-pyrrolidin-1-yl)-3-amino-2-(2-carboxy-pyrrolidin-1-yl)-pyridine
la 6-(2-carboxamido-4-hydroxy-pyrrolidin-1-yl)-3-amino-2-(2-carboxy-pyrrolidin-1-yl)-pyridine
la 6-(pyrrolidin-1-yl)-3-amino-2-(2-hydroxyméthyl-pyrrolidin-1-yl)-pyridine
la 6-(3-hydroxypyrrolidin-1-yl)-3-amino-2-(2-hydroxyméthyl-pyrrolidin-1-yl)-pyridine
la 6-(3-amino-pyrrolidin-1-yl)-3-amino-2-(2-hydroxyméthyl-pyrrolidin-1-yl)-pyridine
la 6-(3-acétamido-pyrrolidin-1-yl)-3-amino-2-(2-hydroxyméthyl-pyrrolidin-1-yl)-pyridine
la 6-(3,4-dihydroxy -pyrrolidin-1-yl)-3-amino-2-(2-hydroxyméthyl-pyrrolidin-1-yl)-pyridine
la 6-(2-carboxy-pyrrolidin-1-yl)-3-amino-2-(2-hydroxyméthyl-pyrrolidin-1-yl)-pyridine
la 6-(2-carboxy-3-hydroxy-pyrrolidin-1-yl)-3-amino-2-(2-hydroxyméthyl-pyrrolidin-1-yl)-pyridine
la 6-(2-carboxy-4-hydroxy-pyrrolidin-1-yl)-3-amino-2-(2-hydroxyméthyl-pyrrolidin-1-yl)-pyridine
la 6-(2-hydroxyméthyl-pyrrolidin-1-yl)-3-amino-2-(2-hydroxyméthyl-pyrrolidin-1-yl)-pyridine
la 6-(2-hydroxyméthyl-4-hydroxy-pyrrolidin-1-yl)-3-amino-2-(2-hydroxyméthyl-pyrrolidin-1-yl)-pyridine
la 6-(2-hydroxyméthyl-2-hydroxy-pyrrolidin-1-yl)-3-amino-2-(2-hydroxyméthyl-pyrrolidin-1-yl)-pyridine
la 6-(2-carboxamido-pyrrolidin-1-yl)-3-amino-2-(2-hydroxyméthyl-pyrrolidin-1-yl)-pyridine
la 6-(2-diméthylcarboxamido-pyrrolidin-1-yl)-3-amino-2-(2-hydroxyméthyl-pyrrolidin-1-yl)-pyridine
la 6-(2-carboxamido-3-hydroxy-pyrrolidin-1-yl)-3-amino-2-(2 hydroxyméthyl-pyrrolidin-1-yl)-pyridine
la 6-(2-carboxamido-4-hydroxy-pyrrolidin-1-yl)-3-amino-2-(2-hydroxyméthyl-pyrrolidin-1-yl)-pyridine
la 6-(pyrrolidin-1-yl)-3-amino-2-(2-carboxamido-pyrrolidin-1-yl)-pyridine
la 6-(3-hydroxypyrrolidin-1-yl)-3-amino-2-(2-carboxamido-pyrrolidin-1-yl)-pyridine
la 6-(3-amina-pyrrolidin-1-yl)-3-amino-2-(2-carboxamido-pyrrolidin-1-yl)-pyridine
la 6-(3-acétamido-pyrrolidin-1-yl)-3-amino-2-(2-carboxamido-pyrrolidin-1-yl)-pyridine
la 6-(3,4-dihydroxy-pyrrolidin-1-yl)-3-amino-2-(2-carboxamido-pyrrolidin-1 -yl)-pyridine
la 6-(2-carboxy-pyrrolidin-1-yl)-3-amino-2-(2-carboxamido-pyrrolidin-1-yl)-pyridine
la 6-(2-carboxy-3-hydroxy-pyrrolidin-1-yl)-3-amino-2-(2-carboxamido-pyrrolidin-1-yl)-pyridine
la 6-(2-carboxy-4-hydroxy-pyrrolidin-1-yl)-3-amino-2-(2-carboxamido-pyrrolidin-1-yl)-pyridine
la 6-(2-hydroxyméthyl-pyrrolidin-1-yl)-3-amino-2-(2-carboxamido-pyrrolidin-1-yl)-pyridine
la 6-(2-hydroxyméthyl-4-hydroxy-pyrrolidin-1-yl)-3-amino-2-(2-carboxamido-pyrrolidin-1-yl)-pyridine
la 6-(2-hydroxyméthyl-2-hydroxy-pyrrolidin-1-yl)-3-amino-2-(2-carboxamido-pyrrolidin-1-yl)-pyridine
la 6-(2-carboxamido-pyrrolidin-1-yl)-3-amino-2-(2-carboxamido-pyrrolidin-1-yl)-pyridine
la 6-(2-diméthylcarboxamido-pyrrolidin-1-yl)-3-amino-2-(2-carboxamido-pyrrolidin-1-yl)-pyridine
la 6-(2-carboxamido-3-hydroxy-pyrrolidin-1-yl)-3-amino-2-(2-carboxamido-pyrrolidin-1-yl)-pyridine
la 6-(2-carboxamido-4-hydroxy-pyrrolidin-1-yl)-3-amino-2-(2-carboxamido-pyrrolidin-1-yl)-pyridine
la 6-(pyrrolidin-1-yl)-3-amino-2-[méthyl-(2-hydroxyéthyl)amino]pyridine
la 6-(3-hydroxypyrrolidin-1-yl)-3-amino-2-[méthyl-(2-hydroxyéthyl)amino]pyridine
la 6-(3-amino-pyrrolidin-1-yl)-3-amino-2-[méthyl-(2-hydroxyéthyl)amino]pyridine
la 6-(3-acétamido-pyrrolidin-1-yl)-3-amino-2-[méthyl-(2-hydroxyéthyl)amino]pyridine
la 6-(3,4-dihydroxy -pyrrolidin-1-yl)-3-amino-2-[méthyl-(2-hydroxyéthyl)amino]pyridine
la 6-(2-carboxy-pyrrolidin-1-yl)-3-amino-2-[méthyl-(2-hydroxyéthyl)amino]pyridine
la 6-(2-carboxy-3-hydroxy-pyrrolidin-1-yl)-3-amino-2-[méthyl-(2-hydroxyéthyl)amino]pyridine
la 6-(2-carboxy-4-hydroxy-pyrrolidin-1-yl)-3-amino-2-[méthyl-(2-hydroxyéthyl)amino]pyridine
la 6-(2-hydroxyméthyl-pyrrolidin-1-yl)-3-amino-2-[méthyl-(2-hydroxyéthyl)amino]pyridine
la 6-(2-hydroxyméthyl-4-hydroxy-pyrrolidin-1-yl)-3-amino-2-[méthyl-(2-hydroxyéthyl)amino]pyridine
la 6-(2-hydroxyméthyl-2-hydroxy-pyrrolidin-1-yl)-3-amino-2-[méthyl-(2-hydroxyéthyl)amino]pyridine
la 6-(2-carboxamido-pyrrolidin-1-yl)-3-amino-2-[méthyl-(2-hydroxyéthyl)amino]pyridine
la 6-(2-diméthylcarboxamido-pyrrolidin-1-yl)-3-amino-2-[méthyl-(2-hydroxyéthyl)amino]pyridine
la 6-(2-carboxamido-3-hydroxy-pyrrolidin-1-yl)-3-amino-2-[méthyl-(2-hydroxyéthyl)amino]pyridine
la 6-(2-carboxamido-4-hydroxy-pyrrolidin-1-yl)-3-amino-2-[méthyl-(2-hydroxyéthyl)amino]pyridine
la 6-(pyrrolidin-1-yl)-3-amino-2-[méthyl-(2-aminoéthyl)amino]pyridine
la 6-(3-hydroxypyrrolidin-1-yl)-3-amino-2-[méthyl-(2-aminoéthyl)amino]pyridine
la 6-(3-amino-pyrrolidin-1-yl)-3-amino-2-[méthyl-(2-aminoéthyl)amino]pyridine
la 6-(3-acétamido-pyrrolidin-1-yl)-3-amino-2-[méthyl-(2-aminoéthyl)amino]pyridine
la 6-(3,4-dihydroxy -pyrrolidin-1-yl)-3-amino-2-[méthyl-(2-aminoéthyl)amino]pyridine
la 6-(2-carboxy-pyrrolidin-1-yl)-3-amino-2-[méthyl-(2-aminoéthyl)amino]pyridine
la 6-(2-carboxy-3-hydroxy-pyrrolidin-1-yl)-3-amino-2-[méthyl-(2-aminoéthyl)amino]pyridine
la 6-(2-carboxy-4-hydroxy-1-yl)-3-amino-2-[méthyl-(2-aminoéthyl)amino]pyridine
la 6-(2-hydroxyméthyl-pyrrolidin-1-yl)-3-amino-2-[méthyl-(2-aminoéthyl)amino]pyridine
la 6-(2-hydroxyméthyl-4-hydroxy-pyrrolidin-1-yl)-3-amino-2-[méthyl-(2-aminoéthyl)amino]pyridine
la 6-(2-hydroxyméthyl-2-hydroxy-pyrrolidin-1-yl)-3-amino-2-[méthyl-(2-aminoéthyl)amino]pyridine
la 6-(2-carboxamido-pyrrolidin-1-yl)-3-amino-2-[méthyl-(2-aminoéthyl)amino]pyridine
la 6-(2-diméthylcarboxamido-pyrrolidin-1-yl)-3-amino-2-[méthyl-(2-aminoéthyl)amino]pyridine
la 6-(2-carboxamido-3-hydroxy-pyrrolidin-1-yl)-3-amino-2-[méthyl-(2-aminoéthyl)amino]pyridine
la 6-(2-carboxamido-4-hydroxy-pyrrolidin-1-yl)-3-amino-2-[méthyl-(2-aminoéthyl)amino]pyridine
la 6-(pyrrolidin-1-yl)-3-amino-2-[méthyl-(2-acétamidoéthyl)amino]pyridine
la 6-(3-hydroxypyrrolidin-1-yl)-3-amino-2-[méthyl-(2-acétamidoéthyl)amino]pyridine
la 6-(3-amino-pyrrolidin-1-yl)-3-amino-2-[méthyl-(2-acétamidoéthyl)amino]pyridine
la 6-(3-acétamido-pyrrolidin-1-yl)-3-amino-2-[méthyl-(2-acétamidoéthyl)amino]pyridine
la 6-(3,4-dihydroxy-pyrrolidin-1-yl)-3-amino-2-[méthyl-(2-acétamidoéthyl)amino]pyridine
la 6-(2-carboxy-pyrrolidin-1-yl)-3-amino-2-[méthyl-(2-acétamidoéthyl)amino]pyridine
la 6-(2-carboxy-3-hydroxy-pyrrolidin-1-yl)-3-amino-2-[méthyl-(2-acétamidoéthyl)amino]pyridine
la 6-(2-carboxy-4-hydroxy-pyrrolidin-1-yl)-3-amino-2-[méthyl-(2-acétamidoéthyl)amino]pyridine
la 6-(2-hydroxyméthyl-pyrrolidin-1-yl)-3-amino-2-[méthyl-(2-acétamidoéthyl)amino]pyridine
la 6-(2-hydroxyméthyl-4-hydroxy-pyrrolidin-1-yl)-3-amino-2-[méthyl-(2-acétamidoéthyl)amino]pyridine
la 6-(2-hydroxyméthyl-2-hydroxy-pyrrolidin-1-yl)-3-amino-2-[méthyl-(2-acétamidoéthyl)amino]pyridine
la 6-(2-carboxamido-pyrrolidin-1-yl)-3-amino-2-[méthyl-(2-acétamidoéthyl)amino]pyridine
-6-(2-diméthylcarboxamido-pyrrolidin-1-yl)-3-amino-2-[méthyl-(2-acétamidoéthyl)amino]pyridine
la 6-(2-carboxamido-3-hydroxy-pyrrolidin-1-yl)-3-amino-2-[méthyl-(2-acétamidoéthyl)amino]pyridine
la 6-(2-carboxamido-4-hydroxy-pyrrolidin-1-yl)-3-amino-2-[méthyl-(2-acétamidoéthyl)amino]pyridine
la 6-(pyrrolidin-1-yl)-3-amino-2-méthoxypyridine
la 6-(3-hydroxypyrrolidin-1-yl)-3-amino-2-méthoxypyridine
la 6-(3-amino-pyrrolidin-1-yl)-3-amino-2-méthoxypyridine
la 6-(3-acétamido-pyrrolidin-1-yl)-3-amino-2-méthoxypyridine
la 6-(3,4-dihydroxy -pyrrolidin-1-yl)-3-amino-2-méthoxypyridine
la 6-(2-carboxy-pyrrolidin-1-yl)-3-amino-2-méthoxypyridine
la 6-(2-carboxy-3-hydroxy-pyrrolidin-1-yl)-3-amino-2-méthoxypyridine
la 6-(2-carboxy-4-hydroxy-pyrrolidin-1-yl)-3-amino-2-méthoxypyridine
la 6-(2-hydroxyméthyl-pyrrolidin-1-yl)-3-amino-2-méthoxypyridine
la 6-(2-hydroxyméthyl-4-hydroxy-pyrrolidin-1-yl)-3-amino-2-méthoxypyridine
la 6-(2-hydroxyméthyl-2-hydroxy-pyrrolidin-1-yl)-3-amino-2-méthoxypyridine
la 6-(2-carboxamido-pyrrolidin-1-yl)-3-amino-2-méthoxypyridine
la 6-(2-diméthylcarboxamido-pyrrolidin-1-yl)-3-amino-2-méthoxypyridine
la 6-(2-carboxamido-3-hydroxy-pyrrolidin-1-yl)-3-amino-2-méthoxypyridine
la 6-(2-carboxamido-4-hydroxy-pyrrolidin-1-yl)-3-amino-2-méthoxypyridine
la 6-(pyrrolidin-1-yl)-3-amino-2-(2-hydroxyéthyloxy)pyridine
la 6-(3-hydroxypyrrolidin-1-yl)-3-amino-2-(2-hydroxyéthyloxy)pyridine
la 6-(3-amino-pyrrolidin-1-yl)-3-amino-2-(2-hydroxyéthyloxy)pyridine
la 6-(3-acétamido-pyrrolidin-1-yl)-3-amino-2-(2-hydroxyéthyloxy)pyridine
la 6-(3,4-dihydroxy-pyrrolidin-1-yl)-3-amino-2-(2-hydroxyéthyloxy)pyridine
la 6-(2-carboxy-pyrrolidin-1-yl)-3-amino-2-(2-hydroxyéthyloxy)pyridine
la 6-(2-carboxy-3-hydroxy-pyrrolidin-1-yl)-3-amino-2-(2-hydroxyéthyloxy)pyridine
la 6-(2-carboxy-4-hydroxy-pyrrolidin-1-yl)-3-amino-2-(2-hydroxyéthyloxy)pyridine
la 6-(2-hydroxyméthyl-pyrrolidin-1-yl)-3-amino-2-(2-hydroxyéthyloxy)pyridine
la 6-(2-hydroxyméthyl-4-hydroxy-pyrrolidin-1-yl)-3-amino-2-(2-hydroxyéthyloxy)pyridine
la 6-(2-hydroxyméthyl-2-hydroxy-pyrrolidin-1-yl)-3-amino-2-(2-hydroxyéthyloxy)pyridine
la 6-(2-carboxamido-pyrrolidin-1-yl)-3-amino-2-(2-hydroxyéthyloxy)pyridine
la 6-(2-diméthylcarboxamido-pyrrolidin-1-yl)-3-amino-2-(2-hydroxyéthyloxy)pyridine
la 6-(2-carboxamido-3-hydroxy-pyrrolidin-1-yl)-3-amino-2-(2-hydroxyéthyloxy)pyridine
la 6-(2-carboxamido-4-hydroxy-pyrrolidin-1-yl)-3-amino-2-(2-hydroxyéthyloxy)pyridine
la 6-(pyrrolidin-1-yl)-3-amino-2-éthoxypyridine
la 6-(3-hydroxypyrrolidin-1-yl)-3-amino-2-éthoxypyridine
la 6-(3-amino-pyrrolidin-1-yl)-3-amino-2-éthoxypyridine
la 6-(3-acétamido-pyrrolidin-1-yl)-3-amino-2-éthoxypyridine
la 6-(3,4-dihydroxy -pyrrolidin-1-yl)-3-amino-2-éthoxypyridine
la 6-(2-carboxy-pyrrolidin-1-yl)-3-amino-2-éthoxypyridine
la 6-(2-carboxy-3-hydroxy-pyrrolidin-1-yl)-3-amino-2-éthoxypyridine
la 6-(2-carboxy-4-hydroxy-pyrrolidin-1-yl)-3-amino-2-éthoxypyridine
la 6-(2-hydroxyméthyl-pyrrolidin-1-yl)-3-amino-2-éthoxypyridine
la 6-(2-hydroxyméthyl-4-hydroxy-pyrrolidin-1-yl)-3-amino-2-éthoxypyridine
la 6-(2-hydroxyméthyl-2-hydroxy-pyrrolidin-1-yl)-3-amino-2-éthoxypyridine
la 6-(2-carboxamido-pyrrolidin-1-yl)-3-amino-2-éthoxypyridine
la 6-(2-diméthylcarboxamido-pyrrolidin-1-yl)-3-amino-2-éthoxypyridine
la 6-(2-carboxamido-3-hydroxy-pyrrolidin-1-yl)-3-amino-2-éthoxypyridine
la 6-(2-carboxamido-4-hydroxy-pyrrolidin-1-yi)-3-amino-2-éthoxypyridine

Ces composés peuvent être synthétisés selon le schéma de synthèse suivant :

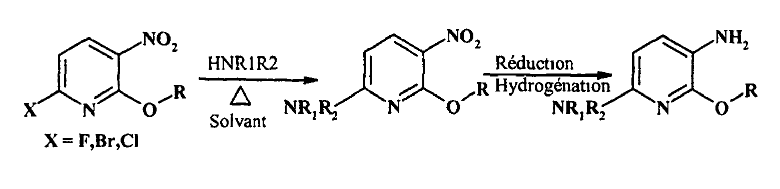

La première étape consiste à faire réagir un dérivé 2-alcoxy-3-nitro-6-halogèno-pyridine avec une amine de type HNR₁R₂ dans un solvant polaire de point d'ébullition compris entre 70°C et 180°C.

La température de réaction varie selon les dérivés de pyridine et l'amine nucléophile, de 75°C à 140°C.

De préférence, on choisira comme solvant, les alcools tels que l'éthanol, isopropanol, le butanol, le pentanol ainsi que l'acide acètique, formique ou le dioxane et la DMF.

La deuxième étape est une réaction de réduction réalisée soit par hydrogénation en catalyse hétérogène, soit par transfert d'hydrogène ou encore par des hydrures métalliques ou encore par le couple acide formique - acide acétique en présence de palladium.

Par exemple, on utilise la méthode largement illustrée dans la littérature d'hydrogénation catalysée par le palladium (0), Pd (II), ou encore par du nickel de Raney ou PtO2.

La réduction par transfert d'hydrogène faisant réagir du cyclohexène en présence de palladium s'avère également très efficace

Les exemples qui suivent servent à illustrer l'invention

### EXEMPLES

### Exemple n°1 : Synthèse du 2-Methoxy-6-(pyrrolidin-1-yl)-3-aminopyridine, trichlorhydrate

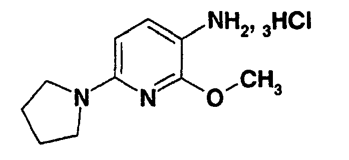

### 1. Synthèse du 2-Methoxy-3-nitro-6-pyrrolidin-1-yl-pyridine:

Dans un ballon tout équipé, on charge 4,79g (0.021 mole)de 6-chloro-3-nitro-2-méthoxypyridine, 40ml d'ethanol et 3,5 ml (0,042mol)de pyrrolidine. Le mélange est porté au reflux pendant 1 heure sous agitation puis le mélange est refroidi à température ambiante. Le produit qui cristallise est essoré puis lavé à l'éther diisopropylique. Après séchage, on obtient 4,6g de poudre jaune, Rdt : 96%.

L'analyse en spectrométrie de masse et en spectroscopie de résonance magnétique est conforme à la structure envisagée.

### 2. Synthèse du 2-Methoxy-6-(pyrrolidin-1-yl)-3-aminopyridine, trichlorhydrate

Dans un autoclave de 300ml, on charge 4g (0,0179 mole) de 2-Methoxy-3-nitro-6-pyrrolidin-1-yl-pyridine, produit synthétisé selon le mode opératoire ci dessus, 200ml d'éthanol et 0,8g de palladium sur charbon. Le mélange est réduit pendant deux heures sous agitation à une pression de 8 bars, le catalyseur est ensuite éliminé par filtration puis le filtrat est acidifié par de l'acide chlorhydrique. Après dilution à l'éther diisopropylique, le précipité formé est essoré et séché sous vide jusqu'à poids constant. On obtient 2,8g de poudre, Rdt = 67.6%.

L'analyse en spectrométrie de masse et en spectroscopie de résonance magnétique est conforme à la structure envisagée.

### Exemple n°2 : 1-(5-Amino-6-methoxy-pyridin-2-yl)-pyrrolidin-3-ol, dichlorhydrate

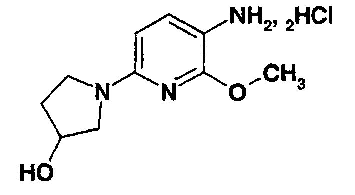

### 1. Synthèse du : 1-(6-Méthoxy-5-nitro-pyridin-2-yl)-pyrrolidin-3-ol

Dans un ballon tout équipé, on charge 2g (0,0106 mole) de 6-chloro-3-nitro-2-méthoxypyridine, 25ml d'éthanol, 1,9 g de 3-pyrrolidinol. Le mélange est porté à 50°C pendant une heure sous agitation puis le mélange est versé sur un mélange de glace/eau sous agitation. Le précipité formé est essoré et séché. On obtient 2g de poudre jaune

L'analyse en spectrométrie de masse et en spectroscopie de résonance magnétique est conforme à la structure envisagée.

### 2. Synthèse du 1-(5-Amino-6-méthoxy-pyridin-2-yl)-pyrrolidin-3-ol dichlorhydrate

Dans un autoclave de 300ml, on charge 2g (8,5 mmoles) de 1-(6-Methoxy-5-nitro-pyridin-2-yl)-pyrrolidin-3-ol, 100ml d'éthanol et 0,5g de palladium sur charbon. On réduit sous 8 bars de pression pendant une heure sous agitation. Le catalyseur est ensuite éliminé par filtration puis le filtrat est acidifié par de l'acide chlorhydrique. Après dilution à l'éther diisopropylique, le précipité formé est essoré et séché sous vide jusqu'à poids constant. On obtient 2,2g de poudre beige claire, Rdt = 88%.
L'analyse en spectrométrie de masse et en spectroscopie de résonance magnétique est conforme à la structure envisagée.

### Exemple n°3 : 3-amino-2-Méthoxy-6(morpholin-4-yl)pyridine, dichlorhydrate

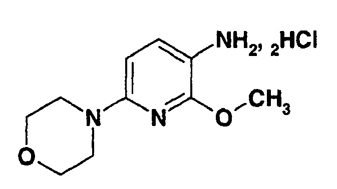

### 1. Synthèse du 4-(6-Methoxy-5-nitro-pyridin-2-yl)-morpholine,:

Dans un ballon tout équipé, on charge 4g (0,02 mole) de 6-Chloro-2-methoxy-3-nitro-pyridine 60ml d'éthanol, 3,69 ml de morpholine. Le mélange est porté au reflux pendant deux heures sous agitation puis le mélange est versé sur un mélange de glace/eau sous agitation. Le précipité formé est essoré et séché. On obtient 2,2g de poudre jaune, Rdt=50%.

L'analyse en spectrométrie de masse et en spectroscopie de résonance magnétique est conforme à la structure envisagée.

### 2. Synthèse du 3-amino-2-Methoxy-6-(morpholin-4-yl)pyridine, dichlorhydrate

Dans un autoclave de 300ml, on charge 2g (0,01 mole)de 4-(6-Methoxy-5-nitro-pyridin-2-yl)-morpholine, 100ml d'éthanol et 0,5g de palladium sur charbon. On réduit sous 8 bars de pression pendant une heure sous agitation, le catalyseur est éliminé par filtration puis le filtrat est acidifié par de l'acide chlorhydrique. Après dilution à l'éther diisopropylique, le précipité formé est essoré et séché. On obtient 2,2g de poudre beige claire, Rdt = 88%.

L'analyse en spectrométrie de masse et en spectroscopie de résonance magnétique est conforme à la structure envisagée.

### Exemple n°4 : 3-amino-2-Méthoxy-6-(pipéridin-1-yl)-pyridine dichlorhydrate

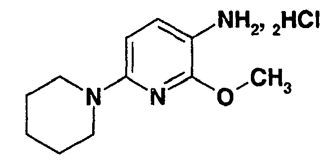

### 1. Synthèse du 2-Méthoxy-5-nitro-6-(pipéridin-1-yl)pyridine

Dans un ballon tout équipé, on charge 4g (0,02 mole)de 6-chloro-3-nitro-2-méthoxypyridine, 60ml d'éthanol, 4,2 ml de pipéridine. Le mélange est porté au reflux pendant deux heures sous agitation puis le mélange est versé sur un mélange de glace/eau sous agitation. Le précipité formé est essoré et séché. On obtient 2g de poudre jaune, Rdt= 42.5%.

L'analyse en spectrométrie de masse et en spectroscopie de résonance magnétique est conforme à la structure envisagée.

### 2. Synthèse du 3-amino-2-Méthoxy-6-(pipéridin-1-yl)-pyridine dichlorhydrate

Dans un autoclave de 300ml, on charge 2g (0,0085 mole) de 6-Methoxy-5-nitro-2-(pipéridin-1-yl)pyridine, 100 ml d'éthanol et 0,5g de palladium sur charbon. On réduit sous 8 bars de pression pendant une heure sous agitation. Le catalyseur est ensuite éliminé par filtration puis le filtrat est acidifié par de l'acide chlorhydrique. Après dilution à l'éther diisopropylique, le précipité formé est essoré et séché sous vide jusqu'à poids constant. On obtient 1,8g de poudre beige claire, Rdt = 75%.

L'analyse en spectrométrie de masse et en spectroscopie de résonance magnétique est conforme à la structure envisagée.

### Exemple n°5: 6-N,N-(2-hydroxyéthyl)-méthyl)amino)-3-amino-2-méthoxypyridine, dichlorhydrate

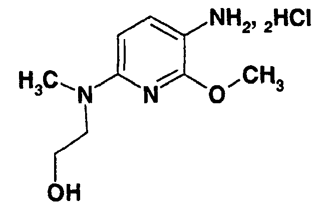

### 1. Synthèse du 6-N,N-(2-hydroxyéthyl)-méthyl)amino)-3-nitro-2-méthoxypyridine:

Dans un ballon tout équipé, on charge 4g (0,02 mole) de 6-chloro-3-nitro-2-méthoxypyridine, 60ml d'éthanol, 3,39 ml de 2-méthylaminoethanol. Le mélange est porté à 50°C pendant une heure sous agitation. Le mélange est versé sur un mélange de glace/eau sous agitation. Le précipité formé est essoré et séché. On obtient 2,5g de poudre jaune, Rdt = 56%.

L'analyse en spectrométrie de masse et en spectroscopie de résonance magnétique est conforme à la structure envisagée.

### 2. Synthèse du 6-N,N-(2-hydroxyéthyl)-méthyl)amino)-3-amino-2-méthoxypyridine:

Dans un autoclave de 300ml, on charge 2g (0,0140 mole)de 6-(N,N-(2-hydroxyéthyl)-méthylamino)-3-nitro-2-méthoxypyridine synthétisé selon le mode opératoire ci dessus, 100ml d'éthanol et 0,5g de palladium sur charbon. Le mélange est réduit pendant une heure à une pression de 8 bars sous agitation, le catalyseur est ensuite éliminé par filtration puis le filtrat est acidifié par de l'acide chlorhydrique. Après dilution à l'éther diisopropylique, le précipité formé est essoré et séché. On obtient 2g de poudre, Rdt = 53%.

L'analyse en spectrométrie de masse et en spectroscopie de résonance magnétique est conforme à la structure envisagée.

### Exemple n°6 : 2-Pyrrolidin-1-yl-5,6-diaminopyridine, dichlorhydrate

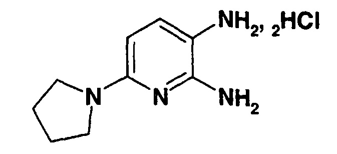

### 1. Synthèse du 6-amino-5-Nitro-2-pyrrolidin-1-pyridine:

Dans un ballon tout équipé, on charge 2g (0,012 mole)de 2-amino-3-nitro-6-chloroxypyridine, 30 ml de dioxane et 20ml d'eau, 1,8g de pyrrolidine. Le mélange est porté à 70°C pendant une heure sous agitation puis le mélange est versé sur un mélange de glace/eau sous agitation. Le précipité formé est essoré et séché. On obtient 2.2g de poudre jaune, Rdt = 88%.

L'analyse en spectrométrie de masse et en spectroscopie de résonance magnétique est conforme à la structure envisagée.

### 2. Synthèse du 5,6-diamino-2-pyrrolidin-1-pyridine :

Dans un autoclave de 300ml, on charge 2g de 2-amino-3-Nitro-6-pyrrolidin-1-yl-pyridine, synthétisé selon le mode opératoire ci dessus, 100ml d'éthanol et 0.5g de palladium sur charbon. Le mélange est réduit pendant une heure à une pression de 8 bars sous agitation. Le catalyseur est ensuite éliminé par filtration puis le filtrat est acidifié par de l'acide chlorhydrique. Après dilution à l'éther diisopropylique, le précipité formé est essoré et séché. On obtient 2,1g de poudre, Rdt = 87.1%

L'analyse en spectrométrie de masse et en spectroscopie de résonance magnétique est conforme à la structure envisagée.

### Exemple n°7 : N-(5,6-Diamino-pyridin-2-yl)-3-hydroxy-pyrrolidine, dichlorhydrate

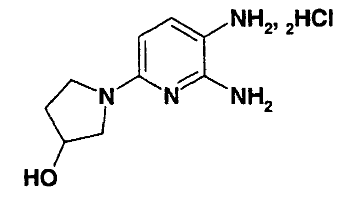

### 1. Synthèse du N-(6-amino-5-nitropyridin-2-yl)-3-hydroxy-pyrrolidine

Dans un ballon tout équipé, on charge 2g (0,012 mole) de 2-amino-3-nitro-6-chloroxypyridine, 30 ml de dioxane et 10 ml d'eau, 1,8g de 3-pyrrolidinol. Le mélange est porté à 70°C pendant une heure sous agitation puis le mélange est versé sur un mélange de glace/eau sous agitation. Le précipité formé est essoré et séché. On obtient 2,36g de poudre jaune, Rdt = 87,4%.

L'analyse en spectrométrie de masse et en spectroscopie de résonance magnétique est conforme à la structure envisagée.

### 2. Synthèse du N-(5,6-Diamino-pyridin-2-yl)-3-hydroxy-pyrrolidine, dichlorhydrate

Dans un autoclave de 300 ml, on charge 2 g de N-(6-amino-5-nitropyridin-2-yl)-3-hydroxy-pyrrolidine synthétisé selon le mode opératoire ci dessus, 100ml d'éthanol et 0,5g de palladium sur charbon. Le mélange est réduit pendant une heure à une pression de 8 bars sous agitation, le catalyseur est ensuite éliminé par filtration puis le filtrat est acidifié par de l'acide chlorhydrique. Après dilution à l'éther diisopropylique, le précipité formé est essoré et séché. On obtient 2,1g de poudre, Rdt = 88%.

L'analyse en spectrométrie de masse et en spectroscopie de résonance magnétique est conforme à la structure envisagée.

### Exemple n°8 : 2,6-Di-(pyrrolidin-1-yl)-3-aminopyridine, dichlorhydrate

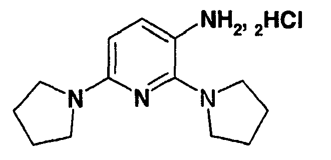

### 1. Synthèse du 2,6-Di-(pyrrolidin-1-yl)-3-nitro-pyridine

Dans un ballon tout équipé, on charge 2g (0,012 mole)de 2,6-dichloro-3-nitropyridine, 30ml de dioxane et 10ml d'eau, 2,9g de pyrrolidine. Le mélange est porté à 70°C pendant une heure sous agitation puis le mélange est versé sur un mélange de glace/eau sous agitation. Le précipité formé est essoré et séché. On obtient 1,9g de poudre jaune, Rdt = 73%.

L'analyse en spectrométrie de masse et en spectroscopie de résonance magnétique est conforme à la structure envisagée.

### 2. Synthèse du 2,6-di-(pyrrolidin-1-yl)-3-aminopyridine, dichlorhydrate

Dans un autoclave de 300ml, on charge 1,5g (0,0056 mole) de 2,6-Di-(pyrrolidin-1-yl)-3-nitro-pyridine synthétisé selon le mode opératoire ci dessus, 100ml d'éthanol et 0,5g de palladium sur charbon. Le mélange est réduit pendant une heure à une pression de 8 bars sous agitation, le catalyseur est ensuite éliminé par filtration puis le filtrat est acidifié par de l'acide chlorhydrique. Après dilution à l'éther diisopropylique, le précipité formé est essoré et séché. On obtient 0,95g de poudre, Rdt = 56%.

L'analyse en spectrométrie de masse et en spectroscopie de résonance magnétique est conforme à la structure envisagée.

### EXEMPLES 1 A 4 DE TEINTURE EN MILIEU ALCALIN

| **Exemples** | **1** | **2** | **3** | **4** |
|---|---|---|---|---|
| 2,6-di-(pyrrolidin-1-yl)-3-aminopyridine, dichlorhydrate (base) | 10⁻³ mole | - | - | - |
| | | | | |
| 5,6-diamino-2-pyrrolidin-1-yl-pyridine, dichlorhydrate (base) | - | 10⁻³ mole | - | - |
| | | | | |
| N-(5,6-diamino-pyridin-2-yl)-3-hydroxy-pyrrolidine, dichlorhydrate (base) | - | - | 10⁻³ mole | - |
| | | | | |
| 2-méthoxy-6-(pyrrolidin-1-yl)-3-aminopyridine, dichlorhydrate (base) | - | - | - | 10⁻³ mole |
| | | | | |
| 2,4-diamino-phénoxyéthanol, dichlorhydrate (coupleur) | - | - | 10⁻³ mole | - |
| | | | | |
| Résorcine (coupleur) | | | - | 10⁻³ mole |
| | | | | |
| 2-méthyl-5-aminophénol (coupleur) | 10⁻³ mole | 10⁻³ mole | - | - |
| | | | | |
| Support de teinture (1) | (*) | (*) | (*) | (*) |
| | | | | |
| Eau déminéralisée q.s.p. | 100 g | 100 g | 100 g | 100 g |

| | | | | |
|---|---|---|---|---|
| (*) Support de teinture (1) pH 9.5 | | | | |

| | |
|---|---|
| Alcool éthylique à 96° | 20 g |
| Métabisulfite de sodium en solution aqueuse à 35% | 0,2275g M.A |
| Sel pentasodique de l'acide diethylene triamino pentacétique | 0,48g M.A |
| Alkyl en C₈-C₁₅polyglucoside vendu en solution à 60% sous la dénomination ORAMIXCG110 par la société SEPPIC | 3,6g M.A |
| Alcool benzylique | 2,0g |
| Polyethylène glycol à 8 moles d'OE | 3,0g |
| NH₄Cl | 4,28g |
| Ammoniaque à 20% de NH3 | 6,8 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 min de pose, les mèches sont rinçées, lavées avec un shampooing standard, rinçées à nouveau puis séchées.

Les résultats de teinture suivants ont été obtenus.

| **Exemples** | **1** | **2** | **3** | **4** |
|---|---|---|---|---|
| **Nuance observée** | Vert turquoise | Gris foncé verdâtre | Vert foncé | Bleu gris |

### EXEMPLES 5 A 7 DE TEINTURE EN MILIEU ACIDE

On a préparé les compositions tinctoriales suivantes :

| **Exemples** | **5** | **6** | **7** |
|---|---|---|---|
| 5,6-diamino-2-pyrrolidin-1-yl-pyridine, dichlorhydrate (base) | 10⁻³ mole | - | - |
| | | | |
| 2-méthoxy-6-(pyrrolidin-1-yl)-3-aminopyridine, dichlorhydrate (base) | - | 10⁻³ mole | - |
| | | | |
| N-(5,6-diamino-pyridin-2-yl)-3-hydroxy-pyrrolidine, dichlorhydrate (base) | - | - | 10⁻³ mole |
| | | | |
| 2,4-diamino-phénoxyéthanol, dichlorhydrate (coupleur) | 10⁻³ mole | 10⁻³ mole | - |
| | | | |
| Résorcine (coupleur) | - | - | 10⁻³ mole |
| | | | |
| Support de teinture (2) | (*) | (*) | (*) |
| | | | |
| Eau déminéralisée q.s.p. | 100 g | 100 g | 100 g |

| | | | |
|---|---|---|---|
| (*) Support de teinture (2) pH 7 | | | |

| | |
|---|---|
| Alcool éthylique à 96° | 20 g |
| Métabisulfite de sodium en solution aqueuse à 35% | 0,2275g M.A |
| Sel pentasodique de l'acide diethylene triamino pentacétique | 0,48g M.A |
| Alkyl en C₈-C₁₅polyglucoside vendu en solution à 60% sous la dénomination ORAMIXCG110 par la société SEPPIC | 3,6g M.A |
| Alcool benzylique | 2,0g |
| Polyethylène glycol à 8 moles d'OE | 3,0 g |
| K₂HPO₄ | 20,9 g |
| KH₂PO₄ | 10,88g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 min de pose, les mèches sont rinçées, lavées avec un shampooing standard, rinçées à nouveau puis séchées.

Les résultats de teinture suivants ont été obtenus.

| **Exemples** | **5** | **6** | **7** |
|---|---|---|---|
| **Nuance observée** | Noir bleu | Bleu foncé | Vert moyen |

## Revendications

1. Composition tinctoriale comprenant, dans un milieu de teinture approprié, à titre de base d'oxydation au moins un dérivé 2,5-diaminopyridine de formule (I) ou les sels d'addition correspondants 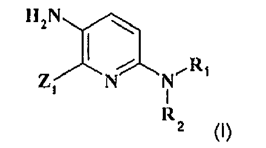 dans laquelle
Z₁ représente un radical OR₃ ou NR₄R₅ ;
R₁ R₂, R₄ et R₅ représentent, indépendamment l'un de l'autre, un atome d'hydrogène, un alkyle en C₁-C₆, un radical (poly)aminoalkyle en C₂-C₆, un radical (poly)alkylaminoalkyle en C₂-C₆, un radical amino-hydroxyalkyle en C₂-C₈, un radical acylaminoalkyle en C₂-C₆, un radical alcoxyalkyle en C₂-C₆, un radical hydroxyalcoxyalkyle en C₂-C₆, un radical aminoalcoxyalkyle en C₂-C₆, un radical (poly)hydroxyalkyle en C₂-C₆, un radical carboxyalkyle en C₂-C₆, un radical sulfonylalkyle en C₂-C₆,
R₁ et R₂ d'une part, et R₄ et R₅ d'autre part, indépendamment les uns des autres, forment avec l'atome d'azote auquel ils sont rattachés un hétérocycle saturé comportant 5 à 8 chaînons, éventuellement substitué par un ou plusieurs radicaux choisis dans le groupe constitué par les atomes d'halogène, les radicaux amino, (di)alkylamino, hydroxy, carboxy, carboxamido, alkylsulfonylamino, acétamido, alcoxy, les radicaux alkyle en C₁-C₆ éventuellement substitués par un ou plusieurs radicaux hydroxy, amino, (di)-alkylamino, alcoxy, hydroxyalcoxy, aminoalcoxy, carboxy, carboxamido, sulfonamido, sulfonyl, l'hétérocycle pouvant contenir un ou plusieurs hétéroatomes additionnels choisis parmi l'oxygene, l'azote éventuellement substitué, le soufre ou un groupement SO₂, l'hétérocycle ne comportant pas de liaison peroxyde, ni de radicaux diazo ou nitroso ;
R₃ représente un radical alkyle en C₁-C₆, linéaire ou ramifié, pouvant être substitué par un ou plusieurs radicaux choisis parmi les radicaux hydroxy, amino, un radical mono- ou di-alkylamino en C₁-C₃ dont la partie alkyle est éventuellement substituée par un ou plusieurs groupements hydroxy, amino, alcoxyalcoxy en C₁-C₂, carboxy ;à l'exception de la 2,3,6-triaminopyridine et ses sels d'addition.

2. Composition selon la revendication 1 dans laquelle la base d'oxydation de formule (I) est telle que R₃ désigne un radical méthyle, éthyle, hydroxyéthyle, carboxyméthyle , carboxyéthyle.

3. Composition selon la revendication 1 ou 2, dans laquelle la base d'oxydation de formule (I) est telle que R₃ désigne le radical méthyle.

4. Composition selon l'une quelconque des revendications 1 à 3, dans laquelle la base d'oxydation de formule (I) est telle que R_{1,} R₂, R₄ et R₅ représentent, indépendamment les uns des autres, un radical (di)alkylaminoalkyle en C₂-C₄, un radical carboxyalkyle en C₂-C₄, un radical sulfonylalkyle en C₂-C₄, un radical (poly)-hydroxyalkyle en C₂-C₄, un radical (poly)aminoalkyl en C₂-C₄.

5. Composition selon l'une quelconque des revendications 1 à 3, dans laquelle la base d'oxydation de formule (1) est telle que R₁ et R₂ d'une part, et R₄ et R₅ d'autre part, indépendamment les uns des autres, forment avec l'atome d'azote auquel ils sont rattachés un hétérocycle de 5 à 8 chaînons choisi parmi les hétérocycles pyrrolidine, pipéridine, homopipéridine, pipérazine, homopipérazine, diazépane, lesdits cycles pouvant être substitués par un ou plusieurs radicaux alkyle en C₁-C₄ éventuellement substitués par un ou plusieurs hydroxy, amino, (di)alkylamino en C₁-C₂, carboxy, carboxamido ou sulfonamido.

6. Composition selon la revendication 5, dans laquelle la base d'oxydation de formule (I) est telle que R₁ et R₂ d'une part, et R₄ et R₅ d'autre part indépendamment les uns des autres forment avec l'atome d'azote auquel ils sont rattachés un hétérocycle choisi parmi la 2,5-diméthylpyrrolidine, l'acide pyrrolidine 2-carboxylique, l'acide 3-hydroxypyrrolidine 2-carboxylique, l'acide 4-hydroxypyrrolidine 2-carboxylique, la 2,4-dicarboxypyrrolidine, la 3-hydroxy-2-hydroxymethylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la -3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 4-amino-3-hydroxy pyrrolidine, la 3-hydroxy-4-(2-hydroxyéthyl)amino- pyrrolidine, la 2,6-diméthylpipéridine, la 2-carboxypipéridine, la 2-carboxamidopipéridine, la 2-hydroxyméthylpipéridine, la 3-hydroxy-2-hydroxyméthylpipéndme, 3-hydroxypipéridine, la 4-hydroxypipéridine, la 3-hydroxyméthylpipéridine, la homopipéridine, la 2-carboxyhomopipéridine, la 2-carboxamidohomopipéridine, le diazépane, le N-méthyl diazépane, le N-(2-hydroxyéthyl)-diazépane.

7. Composition selon la revendication 6, dans laquelle la base d'oxydation de formule (I) est telle que R₁ et R₂ d'une part, et R₄ et R₅ d'autre part, indépendamment les uns des autres, forment ensemble un hétérocycle choisi parmi la pyrrolidine, la 3-hydroxypyrrolidine, la 3-aminopyrrolidine, la 3-acétamidopyrrolidine, la 3-(méthylsulfonylamino)-pyrrolidine, l'acide pyrrolidine 2-carboxylique, l'acide 3-hydroxypyrrolidine 2-carboxylique, la pipéridine, l'hydroxypipéridine, l'homopipéridine, le diazépane, la N-méthyl homopipérazine, la N β-hydroxyéthylhomopipérazine

8. Composition selon l'une quelconque des revendications 1 à 7, dans laquelle la base d'oxydation de formule (I) est choisie parmi :
la 2-méthoxy-6-(pyrrolidin-1-yl)-3-aminopyridine
la 1-(5-amino-6-méthoxy-pyridin-2-yl)-pyrrolidin-3-ol
la 3-ammo-2-méthoxy-6-(morpholin-4-yl) pyridine,
la 3-amino-2-méthoxy-6-(pipéndin-1 -yl)-pyndme,
la 6-N,N-(2-hydroxyéthyl)-méthyl)amino)-3-amino-2-méthoxypyridine
la 5,6-diamino-2-pyrrolidin-1-yl-pyridrne
la N-(5,6-diamino-pyridin-2-yl)-3-hydroxy-pyrrolidine
la 2,6-di-(pyrrolidin-1-yl)-5-aminopyridine
la 6-(pyrrolidin-1-yl)-2,3-diaminopyridine
la 6-(3-hydroxypyrrolidin-1-yl)-2,3-diaminopyridine
la 6-(3-amino-pyrrolidin-1-yl)-2,3-diaminopyridine
la 6-(3-acétamido-pyrrolidin-1-yl)-2,3-diaminopyridine
la 6-(3,4-dihydroxy -pyrrolidin-1-yl)-2,3-diaminopyridine
la 6-(2-carboxy-pyrrolidin-1-yi)-2,3-diaminopyridine
la 6-(2-carboxy-3-hydroxy-pyrrolidin-1-yl)-2,3-diaminopyridine la 6-(2-carboxy-4-hydroxy-pyrrolidin-1-yl)-2,3-diaminopyridine la 6-(2-hydroxyméthyl-pyrrolidin-1-yl)-2,3-diaminopyridine la 6-(2-hydroxymethyl-4-hydroxy-pyrrolidin-1-yl)-2,3-diaminopyridine la 6-(2-hydroxyméthy!-2-hydroxy-pyrrolrdm-1-yl)-2,3-diaminopyridine la 6-(2-carboxamido-pyrrolidin-1-yl)-2,3-diaminopyridine la 6-(2-diméthylcarboxamido-pyrrolidin-1-yl)-2,3-diaminopyridine la 6-(2-carboxamido-3-hydroxy-pyrrolidin-1-yl)-2,3-diaminopyridine la 6-(2-carboxamido-4-hydroxy-pyrrolidin-1-yl)-2,3-diaminopyridine la 6-(pyrrolidin-1-yl)-3-amino-2-methylaminopyridine la 6-(3-hydroxypyrrolidin-1-yl)-3-amino-2-méthylaminopyridine la 6-(3-amino-pyrrolidin-1-yl)-3-amino-2-méthylaminopyridine la 6-(3-acétamido-pyrrolidin-1-yl)-3-amino-2-méthylaminopyridine la 6-(3,4-dihydroxy -pyrrolidin-1-yl)-3-amino-2-méthylaminopyridine la 6-(2-carboxy-pyrrolidin-1-yl)-3-amino-2-méthylaminopyridine la 6-(2-carboxy-3-hydroxy-pyrrolidin-1-yl)-3-amino-2-méthylaminopyridine la 6-(2-carboxy-4-hydroxy-pyrrolidin-1-yl)-3-amino-2-méthylaminopyridine la 6-(2-hydroxyméthyl-pyrrolidin-1-yl)-3-amino-2-méthylaminopyrridine la 6-(2-hydroxyméthyl-4-hydroxy-pyrrolidin-1-yl)-3-amino-2-méthylaminopyridine la 6-(2-hydroxyméthyl-2-hydroxy-pyrrolidin-1-yl)-3-amino-2-méthylaminopyridine la 6-(2-carboxamido-pyrrolidin-1-yl)-3-amino-2-méthylaminopyridine la 6-(2-diméthylcarboxamido-pyrrolidin- i -yl)-3-am ino-2-methylaminopyridine la 6-(2-carboxamido-3-hydroxy-pyrrolidin-1-yl)-3-amino-2-méthylaminopyridine la 6-(2-carboxamido-4-hydroxy-pyrrolidin-1-yl)-3-amino-2-méthylaminopyridine la 6-(pyrrolidn-1-yl)-3-ammo-2-éthylammopyridme la 6-(3-hydroxypyrrolidm-1-yl)-3-amino-2-éthylaminopyridine la 6-(3-amino-pyrrolidin-1-yl)-3-amino-2-éthylaminopyridine la 6-(3-acétamido-pyrrolidin-1-yl)-3-amino-2-éthylaminopyridine la 6-(3,4-dihydroxy -pyrrolidin-1-yl)-3-amino-2-éthylaminopyridine la 6-(2-carboxy-pyrrolidin-1-yi)-3-amino-2-éthylaminopyridine la 6-(2-carboxy-3-hydroxy-pyrrolidin-1-yl)-3-amino-2-éthylaminopyridine
la 6-(2-carboxy-4-hydroxy-pyrrolidin-1-yl)-3-amino-2-éthylaminopyridine la 6-(2-hydroxyméthyl-pyrrolidin-1-yl)-3-amino-2-éthylaminopyridine
la 6-(2-hydroxyméthyl-4-hydroxy-pyrrolidin-1-yl)-3-amino-2-éthylaminopyridine la 6-(2-hydroxyméthyl-2-hydroxy-pyrrolidin-1-yl)-3-amino-2-éthylaminopyridine la 6-(2-carboxamido-pyrrolidin-1-yl)-3-amino-2-éthylaminopyridine la 6-(2-diméthylcarboxamido-pyrrolidin-1-yl)-3-amino-2-éthylaminopyridine la 6-(2-carboxamido-3-hydroxy-pyrrolidin-1-yl)-3-amino-2-éthylaminopyridine la 6-(2-carboxamido-4-hydroxy-pyrrolidin-i-yl)-3-amino-2-éthylaminopyridine la 6-(pyrrolidin-1-yl)-3-amino-2-(2-hydroxyéthylamino)pyridine la 6-(3-hydroxypyrrohdin-1-yl)-3-amino-2-(2-hydroxyethylamino)pyridine la 6-(3-amino-pyrrolidin-1-yl)-3-amino-2-(2-hydroxyéthylamino)pyridine la 6-(3-acétamido-pyrrolidin-1-yl)-3-amino-2-(2-hydroxyéthylamino)pyridine la 6-(3,4-dihydroxy -pyrrohdin-1 -yl)-3-amino-2-(2-hydroxyéthylamino)pyridine la 6-(2-carboxy-pyrrolidin-1-yl)-3-amino-2-(2-hydroxyéthylamino)pyridine la 6-(2-carboxy-3-hydroxy-pyrrolidin-1-yl)-3-amino-2-(2-hydroxyéthylamino)pyridine la 6-(2-carboxy-4-hydroxy-pyrrolidin-1-yl)-3-amino-2-(2-hydroxyéthylamino)pyridine la 6-(2-hydroxyméthyl-pyrrolidin-1-yl)-3-amino-2-(2-hydroxyéthylamino)pyridine la 6-(2-hydroxyméthyl-4-hydroxy-pyrrolidin-1-yl)-3-amino-2-(2-hydroxyéthylamino)pyridine
la 6-(2-hydroxyméthyl-2-hydroxy-pyrrolidin-1-yl)-3-amino-2-(2-hydroxyéthylamino)pyridine
la 6-(2-carboxamido-pyrrolidin-1-yl)-3-amino-2-(2-hydroxyethylamino)pyridine la 6-(2-diméthylcarboxamido-pyrrolidin-1-yl)-3-amino-2-(2-hydroxyéthylamino)pyridine la 6-(2-carboxamido-3-hydroxy-pyrrolidin-1-yl)-3-amino-2-(2-hydroxyéthylamino)pyridine
la 6-(2-carboxamido-4-hydroxy-pyrrolidin-1-yl)-3-aminopyridine la 6-(pyrrolidin-1-yl)-3-amino-2-(2-aminoéthylamino)pyridine la 6-(3-hydroxypyrrolidin-1-yl)-3-amino-2-(2-aminoéthylamino)pyridine la 6-(3-amrno-pyrrolidin-1-yl)-3-amino-2-(2-aminoéthylamino)pyridine la 6-(3-acétamido-pyrrolidin-1-yl)-3-amino-2-(2-aminoéthylamino)pyridine la 6-(3,4-dihydroxy -pyrrolidin-1-yl)-3-amino-2-(2-aminoéthylamino)pyridine la 6-(2-carboxy-pyrrolidin-1-yl)-3-ammo-2-(2-aminoethylamino)pyridine la 6-(2-carboxy-3-hydroxy-pyrrolidin-1-yl)-3-amino-2-(2-aminoéthylamino)pyridine la 6-(2-carboxy-4-hydroxy-pyrrolidin-1-yl)-3-amino-2-(2-aminoéthylamino)pyridine la 6-(2-hydroxyméthyl-pyrrolidin-1-yl)-3-amino-2-(2-aminoéthylamino)pyridine la 6-(2-hydroxymethyl-4-hydroxy-pyrrolidin-1-yl)-3-amino-2-(2-aminoéthylamino)pyridine
a 6-(2-hydroxyméthyl-2-hydroxy-pyrrolidin-1-yl)-3-amino-2-(2-aminoéthylamino)pyridine
la 6-(2-carboxamido-pyrrolidin-1-yl)-3-amino-2-(2-aminoéthylamino)pyridine la 6-(2-dimethylcarboxamido-pyrroridin-1-yl)-3-amino-2-(2-aminoéthylamino)pyridine la 6-(2-carboxamido-3-hydroxy-pyrrolidin-1-yl)-3-amino-2-(2-aminoéthylamino)pyridine la 6-(2-carboxamido-4-hydroxy-pyrrolidin-1-yl)-3-amino-2-(2-aminoéthylamino)pyridine la 6-(pyrrolidin-1-yl)-3-amino-2-(2-acétamidoéthylamino)pyridine la 6-(3-hydroxypyrrolidin-1-yl)-3-amino-2-(2,acétamidoethylamino)pyridine la 6-(3-amino-pyrrolidin-1-yl)-3-amino-2-(2-acétamidoéthylamino)pyridine la 6-(3-acétamido-pyrrolidin-1-yl)-3-amino-2-(2-acétamidoéthylamino)pyridine la 6-(3,4-dihydroxy -pyrrohdin-1-yl)-3-amino-2-(2-acétamidoéthylammo)pyridine la 6-(2-carboxy-pyrrolidin-1-yl)-3-amino-2-(2-acétamidoéthylamino)pyridine la 6-(2-carboxy-3-hydroxy-pyrrolidin-1-yl)-3-amino-2-(2-acétamidoéthylamino)pyridine la 6-(2-carboxy-4-hydroxy-pyrrolidin-1-yl)-3-amino-2-(2-acétamidoéthylamino)pyridine la 6-(2-hydroxyméthyl-pyrrolidin-1-yl)-3-amino-2-(2-acétamidoéthylamino)pyridine la 6-(2-hydroxyméthyl-4-hydroxy-pyrrolidin-1-yl)-3-amino-2-(2-acétamidoéthylamino)pyridine
la 6-(2-hydroxyméthyl-2-hydroxy-pyrrolidin-1-yl)-3-amino-2-(2-acétamidoéthylamino)pyridine
la 6-(2-carboxamido-pyrrolidin-1-yl)-3-amino-2-(2-acétamidoéthylamino)pyridne la 6-(2-diméthylcarboxamido-pyrrolidin-1-yl)-3-amino-2-(2-acétamidoéthylamino)pyridine
la6-(2-carboxamido-3-hydroxy-pyrrolidin-1-yl)-3-amino-2-(2-acétamidoéthylamino)pyridine
la 6-(2-carboxamido-4-hydroxy-pyrrolidin-1-yl)-3-amino-2-(2-acétamidoéthylamino)pyridine
la 6-(pyrrolidin-1-yl)-3-amino-2-(pyrrolidin-1-yl)-pyridine
la 6-(3-hydroxypyrrolidin-1-yl)-3-amino-2-(pyrrolidin-1-yl)-pyridine la 6-(3-amino-pyrrolidin-1-yl)-3-amino-2-(pyrrolidin-1-yl)-pyridine la 6-(3-acétamido-pyrrolidin-1-yl)-3-amino-2-(pyrrolidin-1-yl)-pyridine la 6-(3,4-dihydroxy -pyrrolidin-1-yl)-3-amino-2-(pyrrolidin-1-yl)-pyridine la 6-(2-carboxy-pyrrolidin-1-yl)-3-amino-2-(pyrrolidin-1-yl)-pyridine la 6-(2-carboxy-3-hydroxy-pyrrolidin-1-yl)-3-amino-2-(pyrrolidin-1-yl)-pyridine la 6-(2-carboxy-4-hydroxy-pyrrolidin-1-yl)-3-amino-2-(pyrrolidin-1-yl)-pyridine la 6-(2-hydroxyméthyl-pyrrolidin-1-yl)-3-amino-2-(pyrrolidin-1-yli)-pyridine la 6-(2-hydroxyméthyl-4-hydroxy-pyrrolidin-1-yl)-3-amino-2-(pyrrolidin-1-yl)-pyridine la 6-(2-hydroxyméthyl-2-hydroxy-pyrrolidin-1-yl)-3-amino-2-(pyrrolidin-1-yl)-pyridine la 6-(2-carboxamido-pyrrolidin-1-yl)-3-amino-2-(pyrrolidin-1-yl)-pyridine la 6-(2-diméthylcarboxamido-pyrrolidin-1-yl)-3-amino-2-(pyrrolidin-1-yl)-pyridine la 6-(2-carboxamido-3-hydroxy-pyrrolidin-1-yl)-3-amino-2-(pyrrolidin-1-yl)-pyridine la 6-(2-carboxamido-4-hydroxy-pyrrolidin-1-yl)-3-amino-2-(pyrrolidin-1-yl)-pyridine la 6-(pyrrolidin-1-yl)-3-amino-2-(3-hydroxy-pyrrolidin-1-yl)-pyridine la 6-(3-hydroxypyrrolidin-1-yl)-3-amino-2-(3-hydroxy-pyrrolidin-1-yl)-pyridine la 6-(3-amino-pyrrolidin-1-yl)-3-amino-2-(3-hydroxy-pyrrolidin-1-yl)-pyridine la 6-(3-acétamido-pyrrolidin-1-yl)-3-amino-2-(3-hydroxy-pyrrolidin-1-yl)-pyridine la 6-(3,4-dihydroxy -pyrrolidin-1-yl)-3-amino-2-(3-hydroxy-pyrrolidin-1-yl)-pyridine la 6-(2-carboxy-pyrrolidin-1-yl)-3-amino-2-(3-hydroxy-pyrrolidin-1-yl)-pyridine la 6-(2-carboxy-3-hydroxy-pyrrolidin-1-yl)-3-amino-2-(3-hydroxy-pyrrolidin-1-yl)-pyridine la 6-(2-carboxy-4-hydroxy-pyrrolidin-1-yl)-3-amino-2-(3-hydroxy-pyrrolidin-1-yl)-pyridine la 6-(2-hydroxyméthyl-pyrrolidin-1-yl)-3-amino-2-(3-hydroxy-pyrrolidin-1-yl)-pyridine la 6-(2-hydroxyméthyl-4-hydroxy-pyrrolidin-1-yl)-3-amino-2-(3-hydroxy-pyrrolidin-1-yl)-pyridine
la 6-(2-hydroxyméthyl-2-hydroxy-pyrrolidin-1-yl)-3-amino-2-(3-hydroxy-pyrrolidin-1-yl)-pyridine
la 6-(2-carboxamido-pyrrolidin-1-yl)-3-amino-2-(3-hydroxy-pyrrolidin-1-yl)-pyridine la 6-(2-diméthylcarboxamido-pyrrolidin-1-yl)-3-amino-2-(3-hydroxy-pyrrolidin-1-yl)-pyridine
la 6-(2-carboxamido-3-hydroxy-pyrrolidin-1-yl)-3-amino-2-(3-hydroxy-pyrrolidin-1-yl)-pyridine
la 6-(2-carboxamido-4-hydroxy-pyrrolidin-1-yl)-3-amino-2-(3-hydroxy-pyrrolidin-1-yl)-pyridine
la 6-(pyrrolidin-1-yl)-3-amino-2-(2-carboxy-pyrrolidin-1-yl)-pyridine la 6-(3-hydroxypyrrolidin-1-yl)-3-amino-2-(2-carboxy-pyrrolidin-1-yl)-pyridine la 6-(3-amino-pyrrolidin-1-yl)-3-amino-2-(2-carboxy-pyrrolidin-1-yl)-pyridine la 6-(3-acétamido-pyrrolidin-1-yl)-3-amino-2-(2-carboxy-pyrrolidin-1-yl)-pyridine la 6-(3,4-dihydroxy -pyrrolidin-1-yl)-3-amino-2-(2-carboxy-pyrrolidin-1-yl)-pyridine la 6-(2-carboxy-pyrrolidin-1-yl)-3-amino-2-(2-carboxy-pyrrolidin-1-yl)-pyridine la 6-(2-carboxy-3-hydroxy-pyrrolidin-1-yl)-3-amino-2-(2-carboxy-pyrrolidin-1-yl)-pyridine la 6-(2-carboxy-4-hydroxy-pyrrolidin-1-yl)-3-amino-2-(2-carboxy-pyrrolidin-1-yl)-pyridine la 6-(2-hydroxyméthyl-pyrrolidin-1-yl)-3-amino-2-(2-carboxy-pyrrolidin-1-yl)-pyridine la 6-(2-hydroxyméthyl-4-hydroxy-pyrrolidin-1-yl)-3-amino-2-(2-carboxy-pyrrolidin-1-yl)-pyridine
la 6-(2-hydroxyméthyl-2-hydroxy-pyrrolidin-1-yl)-3-amino-2-(2-carboxy-pyrrolidin-1-yl)-pyridine
la 6-(2-carboxamido-pyrrolidin-1-yl)-3-amino-2-(2-carboxy-pyrrolidin-1-yl)-pyridine la 6-(2-diméthylcarboxamido-pyrrolidm-1-yl)-3-amino-2-(2-carboxy-pyrrolidin-1-yl)-pyridine
la 6-(2-carboxamido-3-hydroxy-pyrrolidin-1-yl)-3-amno-2-(2-carboxy-pyrrolidin-1-yl)-pyridine
la 6-(2-carboxamido-4-hydroxy-pyrrolidin-1-yl)-3-amino-2-(2-carboxy-pyrrolidin-1-yl)-pyridine
la 6-(pyrrolidin-1-yl)-3-amino-2-(2-hydroxyméthyl-pyrrolidin-1-yl)-pyridine la 6-(3-hydroxypyrrolidin-1-yl)-3-amino-2-(2-hydroxyméthyl-pyrrolidin-1-yl)-pyridine la 6-(3-amino-pyrrolidin-1-yl)-3-amino-2-(2-hydroxyméthyl-pyrrolidin-1-yl)-pyridine la 6-(3-acétamido-pyrrolidin-1-yl)-3-amino-2-(2-hydroxyméthyl-pyrrolidin-1-yl)-pyridine la 6-(3,4-dihydroxy-pyrrolidin-1-yl)-3-amino-2-(2-hydroxyméthyl-pyrrolidin-1-yl)-pyridine la 6-(2-carboxy-pyrrolidin-1-yl)-3-amino-2-(2-hydroxyméthyl-pyrrolidin-1-yl)-pyridine la 6-(2-carboxy-3-hydroxy-pyrrolidin-1-yl)-3-amino-2-(2-hydroxyméthyl-pyrrolidin-1-yl)-pyridine
la 6-(2-carboxy-4-hydroxy-pyrrolidin-1-yl)-3-amino-2-(2-hydroxyméthyl-pyrrolidin-1-yl)-pyridine
la 6-(2-hydroxyméthyl-pyrrolidin-1-yl)-3-amino-2-(2-hydroxyméthyl-pyrrolidin-1-yl)-pyridine
la 6-(2-hydroxyméthyl-4-hydroxy-pyrrolidn-1-yl)-3-ammo-2-(2-hydroxyméthyl-pyrrohdm-1-yl)-pyridine
la 6-(2-hydroxyméthyl-2-hydroxy-pyrrolidin-1-yl)-3-amino-2-(2-hydroxyméthyl-pyrrolidin-1-yl)-pyridine
la 6-(2-carboxamido-pyrrolidin-1-yl)-3-amino-2-(2-hydroxyméthyl-pyrrolidin-1-yl)-pyridine
la 6-(2-diméthylcarboxamido-pyrrolidin-1-yl)-3-amino-2-(2-hydroxyméthyl-pyrrolidin-1-yl)-pyridine
la 6-(2-carboxamido-3-hydroxy-pyrrolidin-1-yl)-3-amino-2-(2-hydroxyméthyl-pyrrolidin-1-yl)-pyridine
la 6-(2-carboxamido-4-hydroxy-pyrrolidin-1-yl)-3-amino-2-(2-hydroxyméthyl-pyrrolidin-1-yl)-pyridine
la 6-(pyrrolidin-1-yl)-3-amino-2-(2-carboxamido-pyrrolidin-1-yl)-pyridine la 6-(3-hydroxypyrrolidin-1-yl)-3-amino-2-(2-carboxamido-pyrrolidin-1-yl)-pyridine la 6-(3-amino-pyrrolidin-1-yl)-3-amino-2-(2-carboxamido-pyrrolidin-1-yl)-pyridine la 6-(3-acétamido-pyrrolidin-1-yl)-3-amino-2-(2-carboxamido-pyrrolidin-1-yl)-pyridine la 6-(3,4-dihydroxy -pyrrolidin-1-yl)-3-amino-2-(2-carboxamido-pyrrolidin-1-yl)-pyridine la 6-(2-carboxy-pyrrolidrn-1-yl)-3-ammo-2-(2-carboxamido-pyrrolidm-1-yl)-pyndme la 6-(2-carboxy-3-hydroxy-pyrrolidin-1-yl)-3-amino-2-(2-carboxamido-pyrrolidin-1-yl)-pyridine
la 6-(2-carboxy-4-hydroxy-pyrrolidin-1-yl)-3-amino-2-(2-carboxamido-pyrrolidin-1-yl)-pyridine
la 6-(2-hydroxyméthyl-pyrrolidin-1-yl)-3-amino-2-(2-carboxamido-pyrrolidin-1-yl)-pyridine
la 6-(2-hydroxymethyl-4-hydroxy-pyrrolidin-1-yl)-3-amino-2-(2-carboxamido-pyrrolidin-1-yl)-pyridine
la 6-(2-hydroxyméthyl-2-hydroxy-pyrrolidin-1-yl)-3-amino-2-(2-carboxamido-pyrrolidin-1-yl)-pyridine
la 6-(2-carboxamido-pyrrolidin-1-yl)-3-amino-2-(2-carboxamido-pyrrolidin-1-yl)-pyridine la 6-(2-diméthylcarboxamido-pyrrolidin-1-yl)-3-amino-2-(2-carboxamido-pyrrolidin-1-yl)-pyridine
la 6-(2-carboxamido-3-hydroxy-pyrrolidin-1-yl)-3-amino-2-(2-carboxamido-pyrrohdin-1-yl)-pyridine
la 6-(2-carboxamido-4-hydroxy-pyrrolidin-1-yl)-3-amino-2-(2-carboxamido-pyrrolidin-1-yl)-pyridine
la 6-(pyrrolidin-1-yl)-3-amino-2-diméthylaminopyridine
la 6-(3-hydroxypyrrolidin-1-yl)-3-amino-2-diméthylaminopyridine la 6-(3-ammo-pyrrolidrn-1-yl)-3-ammo-2-drméthylaminopyridine la 6-(3-acétamido-pyrrolidin-1-yl)-3-amino-2-diméthylaminopyridine la 6-(3,4-dihydroxy -pyrrolidin-1-yl)-3-amino-2-diméthylaminopyridine la 6-(2-carboxy-pyrrolidin-1-yl)-3-amino-2-dimèthylaminopyridine la 6-(2-carboxy-3-hydroxy-pyrrolidin-1-yl)-3-amino-2-diméthylaminopyridine la 6-(2-carboxy-4-hydroxy-pyrrolidin-1-yl)-3-amino-2-diméthylaminopyridine la 6-(2-hydroxyméthyl-pyrrolidin-1-yl)-3-amino-2-diméthylaminopyridine la 6-(2-hydroxyméthyl-4-hydroxy-pyrrolidin-1-yl)-3-amino-2-diméthylaminopyridine la 6-(2-hydroxyméthyl-2-hydroxy-pyrrolidin-1-yl)-3-amino-2-diméthylaminopyridine la 6-(2-carboxamido-pyrrolidin-1-yl)-3-amino-2-diméthylaminopyridine la 6-(2-diméthylcarboxamido-pyrrolidin-1-yl)-3-amino-2-diméthylaminopyridine la 6-(2-carboxamido-3-hydroxy-pyrrolidin-1-yl)-3-amino-2-diméthylaminopyridine la 6-(2-carboxamido-4-hydroxy-pyrrolidin-1-yl)-3-amino-2-diméthylaminopyridine la 6-(pyrrolidin-1-yl)-3-amino-2-[méthyl-(2-hydroxyéthyl)amino]pyridine la 6-(3-hydroxypyrrolidin-1-yl)-3-amino-2-[méthyl-(2-hydroxyéthyl)amino]pyridine la 6-(3-amino-pyrrolidin-1-yl)-3-amino-2-[méthyl-(2-hydroxyéthyl)amino]pyridine la 6-(3-acétamido-pyrrolidin-1-yl)-3-amino-2-[méthyl-(2-hydroxyéthyl)amino]pyridine la 6-(3,4-dihydroxy-pyrrolidin-1-yl)-3-amino-2-[méthyl-(2-hydroxyéthyl)amino]pyridine la 6-(2-carboxy-pyrrolidin-1-yl)-3-amino-2-[méthyl-(2-hydroxyéthyl)amino]pyridine la 6-(2-carboxy-3-hydroxy-pyrrolidin-1-yl)-3-amino-2-[méthyl-(2-hydroxyéthyl)amino]pyridine
la 6-(2-carboxy-4-hydroxy-pyrrolidin-1-yl)-3-amino-2-[méthyl-(2-hydroxyéthyl)amino]pyridine
la 6-(2-hydroxyméthyl-pyrrolidin-1-yl)-3-amino-2-[méthyl-(2-hydroxyéthyl)amino]pyridine
la 6-(2-hydroxyméthyl-4-hydroxy-pyrrolidin-1-yl)-3-amino-2-[méthyl-(2-hydroxyéthyl)amino]pyridine
la 6-(2-hydroxyméthyl-2-hydroxy-pyrrolidin-1-yl)-3-amino-2-[méthyl-(2-hydroxyéthyl)amino]pyridine
la 6-(2-carboxamido-pyrrolidin-1-yl)-3-amino-2-[méthyl-(2-hydroxyéthyl)amino]pyridine la 6-(2-diméthylcarboxamido-pyrrolidin-1-yl)-3-amino-2-[méthyl-(2-hydroxyéthyl)amino]pyridine
la 6-(2-carboxamido-3-hydroxy-pyrrolidin-1-yl)-3-amino-2-[méthyl-(2-hydroxyéthyl)amino]pyridine
la 6-(2-carboxamido-4-hydroxy-pyrrolidin-1-yl)-3-amino-2-[méthyl-(2-hydroxyethyl)amino]pyridine
la 6-(pyrrolidin-1-yl)-3-amino-2-[méthyl-(2-ammoéthyl)amino]pyridine la 6-(3-hydroxypyrrolidin-1-yl)-3-amino-2-[méthyl-(2-aminoéthyl)amino]pyridine la 6-(3-amino-pyrrolidin-1-yl)-3-amino-2-[méthyl-(2-aminoéthyl)amino]pyridine la 6-(3-acetamido-pyrrolidin-1-yl)-3-amino-2-[méthyl-(2-aminoéthyl)amino]lpyridine la 6-(3,4-dihydroxy-pyrrolidin-1-yl)-3-amino-2-[méthyl-(2-aminoéthyl)amino]pyridine la 6-(2-carboxy-pyrrolidin-1-yl)-3-amino-2-[méthyl-(2-aminoéthyl)amino]pyridine la 6-(2-carboxy-3-hydroxy-pyrrolidin-1-yl)-3-amino-2-[méthyl-(2-aminoéthyl)amino]pyridine
la 6-(2-carboxy-4-hydroxy-pyrrolidin-1-yl)-3-amino-2-[méthyl-(2-aminoethyl)amino]pyridine
la 6-(2-hydroxyméthyl-pyrrolidin-1-yl)-3-amino-2-[méthyl-(2-aminoéthyl)amino]pyridine la 6-(2-hydroxyméthyl-4-hydroxy-pyrrolidin-1-yl)-3-amino-2-[méthyl-(2-aminoéthyl)amino]pyridine
la 6-(2-hydroxyméthyl-2-hydroxy-pyrrolidin-1-yl)-3-amino-2-[méthyl-(2-aminoéthyl)amino]pyridine
la 6-(2-carboxamido-pyrrolidin-1-yl)-3-amino-2-[méthyl-(2-aminoéthyl)amino]pyridine la 6-(2-diméthylcarboxamido-pyrrolidin-1-yl)-3-amino-2-[methyl-(2-aminoéthyl)amino]pyridine
la 6-(2-carboxamido-3-hydroxy-pyrrolidin-1-yl)-3-amino-2-[méthyl-(2-aminoéthyl)amino]pyridine
la 6-(2-carboxamido-4-hydroxy-pyrrolidin-1-yl)-3-amino-2-[méthyl-(2-aminoéthyl)amino]pyridine
la 6-(pyrrolidin-1-yl)-3-amino-2-[méthyl-(2-acétamidoéthyl)amino]pyridine la 6-(3-hydroxypyrrolidin-1-yl)-3-amino-2-[méthyl-(2-acétamidoéthyl)amino]pyridine la 6-(3-amino-pyrrolidin-1-yl)-3-amino-2-[méthyl-(2-acétamidoéthyl)amino]pyridine la 6-(3-acétamido-pyrrolidin-1-yl)-3-amino-2-[méthyl-(2-acétamidoéthyl)amino]pyridine la 6-(3,4-dihydroxy-pyrrolidin-1-yl)-3-amino-2-[méthyl-(2-acétamidoéthyl)amino]pyridine
la 6-(2-carboxy-pyrrolidin-1-yl)-3-amino-2-[méthyl-(2-acétamidoéthyl)amino]pyridine la 6-(2-carboxy-3-hydroxy-pyrrolidin-1-yl)-3-amino-2-[méthyl-(2-acétamidoéthyl)amino]pyridine
la 6-(2-carboxy-4-hydroxy-pyrrolidin-1-yl)-3-amino-2-[méthyl-(2-acétamidoéthyl)amino]pyridine
la 6-(2-hydroxyméthyl-pyrrolidin-1-yl)-3-amino-2-[méthyl-(2-acétamidoéthyl)amino]pyridine
la 6-(2-hydroxyméthyl-4-hydroxy-pyrrolidin-1-yl)-3-amino-2-[méthyl-(2-acétamidoéthyl)amino]pyridine
la 6-(2-hydroxyméthyl-2-hydroxy-pyrrolidin-1-yl)-3-amino-2-[méthyl-(2-acetamidoéthyl)amino]pyridine
la 6-(2-carboxamido-pyrrolidin-1-yl)-3-amino-2-[méthyl-(2-acétamidoéthyl)amino]pyridine
la 6-(2-diméthylcarboxamido-pyrrolidin-1-yl)-3-amino-2-[méthyl-(2-acétamidoéthyl)amino]pyridine
la 6-(2-carboxamido-3-hydroxy-pyrrolidin-1-yl)-3-amino-2-[méthyl-(2-acétamidoethyl)amino]pyridine
la 6-(2-carboxamido-4-hydroxy-pyrrolidin-1-yl)-3-amino-2-[méthyl-(2-acétamtdoéthyl)amino]pyridine
la 6-(pyrrolidin-1-yl)-3-amino-2-méthoxypyridine
la 6-(3-hydroxypyrrolidin-1-yl)-3-amino-2-méthoxypyridine
la 6-(3-amino-pyrrolidin-1-yl)-3-amino-2-méthoxypyridine
la 6-(3-acétamido-pyrrolidin-1-yl)-3-amino-2-méthoxypyridine la 6-(3,4-dihydroxy-pyrrolidin-1-yl)-3-amino-2-méthoxypyridine la 6-(2-carboxy-pyrrolidin-1-yl)-3-amino-2-méthoxypyridine
la 6-(2-carboxy-3-hydroxy-pyrrolidin-1-yl)-3-amino-2-méthoxypyridine la 6-(2-carboxy-4-hydroxy-pyrrolidin-1-yl)-3-amino-2-méthoxypyridine la 6-(2-hydroxyméthyl-pyrrolidin-1-yl)-3-amino-2-méthoxypyridine la 6-(2-hydroxyméthyl-4-hydroxy-pyrrolidin-1-yl)-3-amino-2-méthoxypyridine la 6-(2-hydroxyméthyl-2-hydroxy-pyrrolidin-1-yl)-3-amino-2-méthoxypyridine la 6-(2-carboxamido-pyrrolidin-1-yl)-3-amino-2-méthoxypyridine la 6-(2-diméthylcarboxamido-pyrrolidin-1-yl)-3-amino-2-méthoxypyridine la 6-(2-carboxamido-3-hydroxy-pyrrolidin-1-yl)-3-amino-2-méthoxypyridine la 6-(2-carboxamido-4-hydroxy-pyrrolidin-1-yl)-3-amino-2-méthoxypyridine la 6-(pyrrolidin-1-yl)-3-amino-2-(2-hydroxyéthyloxy)pyridine la 6-(3-hydroxypyrrolidin-1 -yl)-3-amino-2-(2-hydroxyéthyloxy)pyridine la 6-(3-amino-pyrrolidin-1-yl)-3-amino-2-(2-hydroxyéthyloxy)pyridine la 6-(3-acétamido-pyrrolidin-1-yl)-3-amino-2-(2-hydroxyéthyloxy)pyridine la 6-(3,4-dihydroxy -pyrrolidin-1-yl)-3-amino-2-(2-hydroxyéthyloxy)pyridine la 6-(2-carboxy-pyrrolidin-1-yl)-3-amino-2-(2-hydroxyéthyloxy)pyridine la 6-(2-carboxy-3-hydroxy-pyrrolidin-1-yl)-3-amino-2-(2-hydroxyéthyloxy)pyridine la 6-(2-carboxy-4-hydroxy-pyrrolidin-1-yl)-3-amino-2-(2-hydroxyéthyloxy)pyridine la 6-(2-hydroxyméthyl-pyrrolidin-1-yl)-3-amino-2-(2-hydroxyéthyloxy)pyridine
la 6-(2-hydroxyméthyl-4-hydroxy-pyrrolidin-1-yl)-3-amino-2-(2-hydroxyéthyloxy)pyridine
la 6-(2-hydroxyméthyl-2-hydroxy-pyrrolidin-1-yl)-3-amino-2-(2-hydroxyéthyloxy)pyridine
la 6-(2-carboxamido-pyrrolidin-1-yl)-3-amino-2-(2-hydroxyéthyloxy)pyridine
la 6-(2-diméthylcarboxamido-pyrrolidin-1-yl)-3-amino-2-(2-hydroxyéthyloxy)pyridine
la 6-(2-carboxamido-3-hydroxy-pyrrolidin-1-yl)-3-amino-2-(2-hydroxyéthyloxy)pyridine
la 6-(2-carboxamido-4-hydroxy-pyrrolidin-1-yl)-3-amino-2-(2-hydroxyéthyloxy)pyridine
la 6-(pyrrolidin-1-yl)-3-amino-2-éthoxypyridine
la 6-(3-hydroxypyrrolidin-1-yl)-3-amino-2-éthoxypyridine
la 6-(3-amino-pyrrolidin-1-yl)-3-amino-2-éthoxypyridine
la 6-(3-acétamido-pyrrolidin-1-yl)-3-amino-2-éthoxypyridine
la 6-(3,4-dihydroxy-pyrrolidin-1-yl)-3-amino-2-éthoxypyridine
la 6-(2-carboxy-pyrrolidin-1-yl)-3-amino-2-éthoxypyridine
la 6-(2-carboxy-3-hydroxy-pyrrolidin-1-yl)-3-amino-2-éthoxypyridine
la 6-(2-carboxy-4-hydroxy-pyrrolidin-1-yl)-3-amino-2-éthoxypyridine
la 6-(2-hydroxyméthyl-pyrrolidin-1-yl)-3-amino-2-éthoxypyridine
la 6-(2-hydroxyméthyl-4-hydroxy-pyrrolidin-1-yl)-3-amino-2-éthoxypyridine
la 6-(2-hydroxymethyl-2-hydroxy-pyrrolidin-1-yl)-3-amino-2-éthoxypyridine
la 6-(2-carboxamido-pyrrolidin-1-yl)-3-amino-2-éthoxypyridine
la 6-(2-diméthylcarboxamido-pyrrolidin-1-yl)-3-amino-2-éthoxypyridine
la 6-(2-carboxamido-3-hydroxy-pyrrolidin-1-yl)-3-amino-2-éthoxypyridine
la 6-(2-carboxamido-4-hydroxy-pyrrolidin-1-yl)-3-amino-2-éthoxypyridine
ainsi que leurs sels d'addition.

9. Composition selon la revendication 8, dans laquelle la base d'oxydation de formule (I) est choisie parmi
la 2-méthoxy-6-(pyrrolidin-1-yl)-3-aminopyridine
la 5,6-diamino-2-pyrrolidin-1-yl-pyridine
la N-(5,6-diamino-pyridin-2-yl)-3-hydroxy-pyrrolidine
la 2,6-di-(pyrrolidin-1-yl)-5-aminopyridine,
ainsi que leurs sels d'addition.

10. Composition selon l'une quelconque des revendications 1 a 9, comprenant de plus une ou plusieurs bases d'oxydation additionnelles choisies parmi les paraphénylènediamines, les bis-phénylalkylènediamines, les para-aminophénols, les ortho-aminophénols, les bases hétérocycliques autres que celles définies dans les revendications 1 à 9 ; et leurs sels d'addition.

11. Composition selon l'une quelconque des revendications 1 à 10, dans laquelle la ou les bases d'oxydation de formule (I) ou la ou les bases additionnelles sont chacune présentes en quantité comprise entre 0,001 et 10 % en poids du poids total de la composition tinctoriale

12. Composition selon l'une quelconque des revendications précédentes comprenant un ou plusieurs coupleurs choisis parmi les métaphénylenediamines, les métaaminophénols, les métadiphénols, les coupleurs naphtaléniques et les coupleurs hétérocycliques et leurs sels d'addition.

13. Composition selon la revendication 12, dans laquelle le ou les coupleurs sont présents en quantité compnse entre 0,001 et 10 % en poids du poids total de la composition tinctoriale.

14. Composition selon l'une quelconque des revendications 1 à 13 comprenant un agent oxydant

15. Composition selon la revendication 14, dans laquelle l'agent oxydant est choisi parmi le peroxyde d'hydrogène, le peroxyde d'urée, les bromates de métaux alcalins, les persels, les peracides et les enzymes oxydases.

16. Procédé de teinture d'oxydation des fibres kératiniques **caractérisé en ce qu'**on applique sur les fibres au moins une composition telle que définie à l'une quelconque des revendications 1 à 13, et qu'on révèle la couleur à l'aide d'un agent oxydant.

17. Procédé selon la revendication 16, dans lequel l'agent oxydant est choisi parmi le peroxyde d'hydrogène, le peroxyde d'urée, les bromates de métaux alcalins, les persels, les peracides et les enzymes oxydases.

18. Procède selon l'une des revendications 16 et 17 dans lequel l'agent oxydant est mélangé au moment de l'emploi à la composition telle que définie selon l'une quelconque des revendications 1 à 13

19. Procédé selon l'une quelconque des revendications 16 et 17 dans lequel l'agent oxydant est appliqué sur les fibres sous forme de composition oxydante simultanément ou sequentiellement a la composition telle que définie selon l'une quelconque des revendications 1 à 13

20. Dispositif a plusieurs compartiments ou "kit" de teinture à plusieurs compartiments, dans lequel un premier compartiment tinctoriale contient une composition telle que définie à l'une quelconque des revendications 1 a 13 et un deuxième compartiment contient une composition oxydante.

21. Produit coloré susceptible d'être obtenu par condensation oxydative d'une composition telle que définie selon l'une quelconque des revendications 1 à 13

22. Utilisation de la composition telle que définie selon l'une quelconque des revendications 1 a 9 pour la teinture des fibres kératiniques.

23. Dérivé de 2,5-diaminopyridine de formule (I') suivante : 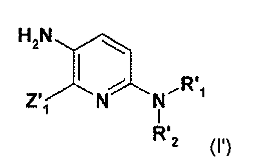 dans laquelle
Z'₁ représente un radical OR'₃ ou NR'₄R'₅ ;
R'₁ R'₂, R'₄ et R'₅ représentent, indépendamment l'un de l'autre, un atome d'hydrogène, un radical (poly)aminoalkyle en C₂-C₆, un radical (poly)alkylaminoalkyle en C₂-C₆, un radical amino-hydroxyalkyle en C₂-C₆, un radical acylaminoalkyle en C₂-C₆, un radical alcoxyalkyle en C₂-C₆, un radical hydroxyalcoxyalkyle en C₂-C₆, un radical aminoalcoxyalkyle en C₂-C₆ un radical (poly)hydroxyalkyle en C₂-C_{6,} un radical carboxyalkyle en C₂-C₆, un radical sulfonylalkyle en C₂-C₆,
R'₁ et R'₂ d'une part, et R'₄ et R'₅ d'autre part, indépendamment les uns des autres, forment avec l'atome d'azote auquel ils sont rattachés un hétérocycle saturé comportant 5 à 8 chaînons, éventuellement substitué par un ou plusieurs radicaux choisis dans le groupe constitué par les atomes d'halogène, les radicaux amino, (di)alkylamino, hydroxy, carboxy, carboxamido, alcoxy, les radicaux alkyle en C₁-C₆ éventuellement substitués par un ou plusieurs radicaux hydroxy, amino, (di)-alkylamino, alcoxy, hydroxyalcoxy, aminoalcoxy, carboxy, sulfonyl, l'hétérocycle pouvant contenir un ou plusieurs hétéroatomes additionnels choisis parmi l'oxygène, l'azote éventuellement substitue, le soufre ou un groupement SO₂, l'hétérocycle ne comportant pas de liaison peroxyde, ni de radicaux diazo ou nitroso ;
R'₃ représente un radical alkyle en C₁-C₆, linéaire ou ramifié, pouvant être substitué par un ou plusieurs radicaux choisis parmi les radicaux hydroxy, amino, un radical mono- ou di-alkylamino en C₁-C₃ dont la partie alkyle est éventuellement substituée par un ou plusieurs, groupements hydroxy, amino, alcoxyalcoxy en C₁-C₂, carboxy ; à l'exception de la 2,3,6-triaminopyridine, de la 6-(4-morpholinyl)-2,3-diaminopyridine, de la 6-(1-piperidinyl)-2,3-diaminopyridine et de la 2,6-di-(4-morphohnyl)-3-ammopyridine et leurs sels d'addition,
a l'exception de la 2,3-diamino-6-(4-méthyl-piperazinyl)-pyridine

24. Dérivé de 2,5-diaminopyridine de formule (I') selon la revendication 23 choisi parmi les composés suivants :
la 2-méthoxy-6-(pyrrolidin-1-yl)-3-aminopyridine
la 1-(5-amino-6-méthoxy-pyridin-2-yl)-pyrrolidin-3-ol
la 3-amino-2-méthoxy-6-(morpholin-4-yl) pyridine,
la 3-amino-2-méthoxy-6-(pipéridin-1-yl)-pyridine,
la 6-N,N-(2-hydroxyéthyl)-méthyl)amino)-3-amino-2-méthoxypyridine
la 5,6-diamino-2-pyrrolidin-1-yl-pyridine
la N-(5,6-diamino-pyridin-2-yl)-3-hydroxy-pyrrolidine
la 2,6-di-(pyrrolidin-1-yl)-5-aminopyridine
la 6-(pyrrolidin-1-yl)-2,3-diaminopyridine
la 6-(3-hydroxypyrrolidin-1-yl)-2,3-diaminopyridine
la 6-(3-amino-pyrrolidin-1-yl)-2,3-diaminopyridine
la 6-(3-acétamido-pyrrolidin-1-yl)-2,3-diaminopyridine
la 6-(3,4-dihydroxy-pyrrolidin-1-yl)-2,3-diaminopyridine
la 6-(2-carboxy-pyrrolidin-1-yl)-2,3-diaminopyridine
la 6-(2-carboxy-3-hydroxy-pyrrolidin-1-yl)-2,3-diaminopyridine
la 6-(2-carboxy-4-hydroxy-pyrrolidin-1-yl)-2,3-diaminopyridine
la 6-(2-hydroxyméthyl-pyrrolidin-1-yl)-2,3-diaminopyridine
la 6-(2-hydroxyméthyl-4-hydroxy-pyrrolidin-1-yl)-2,3-diaminopyridine
la 6-(2-hydroxyméthyl-2-hydroxy-pyrrolidin-1-yl)-2,3-diaminopyridine
la 6-(2-carboxamido-pyrrolidin-1-yl)-2,3-diaminopyridine
la 6-(2-diméthylcarboxamido-pyrrolidin-1-yl)-2,3-diaminopyridine
la 6-(2-carboxamido-3-hydroxy-pyrrohdin-1-yl)-2,3-diaminopyridine
la 6-(2-carboxamido-4-hydroxy-pyrrolidin-1-yl)-2,3-diaminopyridine
la 6-(pyrrolidin-1-yl)-3-amino-2-(2-hydroxyéthylamino)pyridine
la 6-(3-hydroxypyrrolidin-1-yl)-3-amino-2-(2-hydroxyéthylamino)pyridine
la 6-(3-amino-pyrrolidin-1-yl)-3-amino-2-(2-hydroxyéthylamino)pyridine
la 6-(3-acétamido-pyrrolidin-1-yl)-3-amino-2-(2-hydroxyéthylamino)pyridine
la 6-(3,4-dihydroxy-pyrrolidin-1-yl)-3-amino-2-(2-hydroxyéthylamino)pyridine
la 6-(2-carboxy-pyrrolidin-1-yl)-3-amino-2-(2-hydroxyéthylamino)pyridine
la 6-(2-carboxy-3-hydroxy-pyrrolidin-1-yl)-3-amino-2-(2-hydroxyéthylamino)pyridine
la 6-(2-carboxy-4-hydroxy-pyrrolidin-1-yl)-3-amino-2-(2-hydroxyéthylamino)pyridine
la 6-(2-hydroxyméthyl-pyrrolidin-1-yl)-3-amino-2-(2-hydroxyéthylamino)pyridine
la 6-(2-hydroxyméthyl-4-hydroxy-pyrrolidin-1-yl)-3-amino-2-(2-hydroxyéthylamino)pyridine
la 6-(2-hydroxyméthyl-2-hydroxy-pyrrolidin-1-yl)-3-amino-2-(2-hydroxyethylamino)pyridine
la 6-(2-carboxamido-pyrrohdin-1-yl)-3-amino-2-(2-hydroxyéthylamino)pyridine
la 6-(2-dimethylcarboxamido-pyrrolidin-1-yl)-3-amino-2-(2-hydroxyethylamino)pyridine
la 6-(2-carboxamido-3-hydroxy-pyrrolidin-1-yl)-3-amino-2-(2-hydroxyéthylamino)pyridine
la 6-(2-carboxamido-4-hydroxy-pyrrolidin-1-yl)-3-aminopyridinela 6-(pyrrolidin-1-yl)-3-amino-2-(2-aminoéthylammo)pyridine
la 6-(3-hydroxypyrrolidin-1-yl)-3-amino-2-(2-aminoéthylamino)pyridine
la 6-(3-amino-pyrrolidin-1-yl)-3-amino-2-(2-aminoéthylamino)pyridine
la 6-(3-acétamido-pyrrolidin-1-yl)-3-amino-2-(2-aminoéthylamino)pyridine
la 6-(3,4-dihydroxy-pyrrolidin-1-yl)-3-amino-2-(2-aminoéthylamino)pyridine
la 6-(2-carboxy-pyrrolidin-1-yl)-3-amino-2-(2-aminoéthylammo)pyridine
la 6-(2-carboxy-3-hydroxy-pyrrolidin-1-yl)-3-amino-2-(2-aminoéthylamino)pyridine
la 6-(2-carboxy-4-hydroxy-pyrrolidin-1-yl)-3-amino-2-(2-aminoethylamino)pyridine
la 6-(2-hydroxyméthyl-pyrrolidin-1-yl)-3-amino-2-(2-aminoéthylamirio)pyridine
la 6-(2-hydroxyméthyl-4-hydroxy-pyrrolidin-1-yl)-3-amino-2-(2-aminoéthylamino)pyridine
la 6-(2-hydroxyméthyl-2-hydroxy-pyrrolidin-1-yl)-3-amino-2-(2-aminoéthylamino)pyridine
la 6-(2-carboxamido-pyrrolidin-1-yl)-3-amino-2-(2-aminoéthylamino)pyridine la 6-(2-diméthylcarboxamido-pyrrolidin-1-yl)-3-amino-2-(2-aminoéthylamino)pyridine la 6-(2-carboxamido-3-hydroxy-pyrrolidin-1-yl)-3-amino-2-(2-amtnoéthylamino)pyridine la 6-(2-carboxamido-4-hydroxy-pyrrolidin-1-yl)-3-amino-2-(2-aminoéthylamino)pyridine la 6-(pyrrolidin-1-yl)-3-amino-2-(2-acétamidoethylamino)pyridine la 6-(3-hydroxypyrrolidin-1-yl)-3-amino-2-(2-acétamidoéthylamino)pyridine la 6-(3-amino-pyrrolidin-1-yl)-3-amino-2-(2-acetamidoéthylamino)pyridine la 6-(3-acétamido-pyrrolidin-1-yl)-3-amino-2-(2-acétamidoéthylamino)pyridine la 6-(3,4-dihydroxy -pyrrolidin-1-yl)-3-amino-2-(2-acétamidoéthylamino)pyridine la 6-(2-carboxy-pyrrolidin-1-yl)-3-amino-2-(2-acétamidoéthylamino)pyridine la 6-(2-carboxy-3-hydroxy-pyrrolidin-1-yl)-3-amino-2-(2-acétamidoéthylamino)pyridine la 6-(2-carboxy-4-hydroxy-pyrrolidin-1-yl)-3-amino-2-(2-acétamidoéthylamino)pyridine la 6-(2-hydroxyméthyl-pyrrolidin-1-yl)-3-amino-2-(2-acetamidoéthylamino)pyridine la 6-(2-hydroxyméthyl-4-hydroxy-pyrrolidin-1-yl)-3-amini-2-(2-acétamidoéthylamino)pyridine
la 6-(2-hydroxyméthyl-2-hydroxy-pyrrolidin-1-yl)-3-amino-2-(2-acétamidoéthylamino)pyridine
la 6-(2-carboxamido-pyrrolidin-1-yl)-3-amino-2-(2-acétamidoéthylamino)pyridine la 6-(2-diméthylcarboxamido-pyrrolidin-1-yl)-3-amino-2-(2-acétamidoéthylamino)pyridine
la 6-(2-carboxamido-3-hydroxy-pyrrolidin-1-yl)-3-amino-2-(2-acétamidoethylamino)pyridine
la 6-(2-carboxamido-4-hydroxy-pyrrolidin-1-yl)-3-amino-2-(2-acétamidoéthylamino)pyridine
la 6-(pyrrolidin-1-yl)-3-amino-2-(pyrrolidin-1-yl)-pyridine
la 6-(3-hydroxypyrrolidin-1-yl)-3-amino-2-(pyrrolidin-1-yl)-pyridine la 6-(3-amino-pyrrolidin-1-yl)-3-amino-2-(pyrrolidin-1-yl)-pyridine la 6-(3-acétamido-pyrrolidin-1-yl)-3-amino-2-(pyrrolidin-1-yl)-pyridine la 6-(3,4-dihydroxy-pyrrolidin-1-yl)-3-amino-2-(pyrrolidin-1-yl)-pyridine la 6-(2-carboxy-pyrrolidin-1-yl)-3-amino-2-(pyrrolidin-1-yl)-pyridine la 6-(2-carboxy-3-hydroxy-pyrrolidin-1-yl)-3-amino-2-(pyrrolidin-1-yl)-pyridine la 6-(2-carboxy-4-hydroxy-pyrrolidin-1-yl)-3-amino-2-(pyrrolodin-1-yl)-pyridine la 6-(2-hydroxyméthyl-pyrrolidin-1 -yl)-3-amino-2-(pyrrolidin-1-yl)-pyridine la 6-(2-hydroxyméthyl-4-hydroxy-pyrrolidin-1-yl)-3-amino-2-(pyrrolidin-1-yl)-pyridine la 6-(2-hydroxyméthyl-2-hydroxy-pyrrolidin-1-yl)-3-amino-2-(pyrrolidin-1-yl)-pyridine la 6-(2-carboxamido-pyrrolidin-1-yl)-3-amino-2-(pyrrolidin-1-yl)-pyridine la 6-(2-diméthylcarboxamido-pyrrolidin-1-yl)-3-amino-2-(pyrrolidin-1-yl)-pyridine la 6-(2-carboxamido-3-hydroxy-pyrrolidin-1-yl),3-amino-2-(pyrrolidin-1-yl)-pyridine la 6-(2-carboxamido-4-hydroxy-pyrrolidin-1-yl)-3-amino-2-(pyrrolidin-1-yl)-pyridine la 6-(pyrrolidin-1-yl)-3-amino-2-(3-hydroxy-pyrrolidin-1-yl)-pyridine la 6-(3-hydroxypyrrolidin-1-yl)-3-amino-2-(3-hydroxy-pyrrolidin-1-yl)-pyridine la 6-(3-amino-pyrrolidin-1-yl)-3-amino-2-(3-hydroxy-pyrrolidine-1-yl)-pyridine la 6-(3-acétamido-pyrrolidin-1-yl)-3-amino-2-(3-hydroxy-pyrrolidin-1-yl)-pyridine la 6-(3,4-dihydroxy-pyrrolidin-1-yl)-3-amino-2-(3-hydroxy-pyrrolidin-1-yl)-pyridine la 6-(2-carboxy-pyrrolidin-1-yl)-3-amino-2-(3-hydroxy-pyrrolidin-1-yl)-pyridine la 6-(2-carboxy-3-hydroxy-pyrrolidin-1-yl)-3-amino-2-(3-hydroxy-pyrrolodin-1-yl)-pyridine la 6-(2,carboxy-4-hydroxy-pyrrolodin-1-yl)-3-amino-2-(3-hydroxy-pyrrolidin-1-yl)-pyridine la 6-(2-hydroxyméthyl-pyrrolidin-1-yl)-3-amino-2-(3-hydroxy-pyrrolidin-1-yl)-pyridine
la6-(2-hydroxyméthyl-4-hydroxy-pyrrolidin-1-yl)-3-amino-2-(3-hydroxy-pyrrolidin-1-yl)-pyridine
la 6-(2-hydroxyméthyl-2-hydroxy-pyrrolidin-1-yl)-3-amino-2-(3-hydroxy-pyrrolidin-1-yl)-pyridine
la 6-(2-carboxamido-pyrrolodin-1-yl)-3-amino-2-(3-hydroxy-pyrrolidin-1-yl)-pyridine
la 6-(2-diméthylcarboxamido-pyrrolidin-1-yl)-3-amino-2-(3-hydroxy-pyrrolidin-1-yl)-pyridine
la 6-(2-carboxamido-3-hydroxy-pyrrolidin-1-yl)-3-amino-2-(3-hydroxy-pyrrolidin-1-yl)-pyridine
la 6-(2-carboxamido-4-hydroxy-pyrrolidin-1-yl)-3-amino-2-(3-hydroxy-pyrrolidin-1-yl)-pyridine
la 6-(pyrrolidin-1-yl)-3-amino-2-(2-carboxy-pyrrolidin-1-yl)-pyridine
la 6-(3-hydroxypyrrolidin-1-yl)-3-amino-2-(2-carboxy-pyrrolidin-1-yl)-pyridine
la 6-(3-amino-pyrrolidin-1-yl)-3-amino-2-(2-carboxy-pyrrolidin-1-yl)-pyridine
la 6-(3-acétamido-pyrrolidin-1-yl)-3-amino-2-(2-carboxy-pyrrolidin-1-yl)-pyridine
la 6-(3,4-dihydroxy-pyrrolidin-1-yl)-3-amino-2-(2-carboxy-pyrrolidin-1-yl)-pyridine
la 6-(2-carboxy-pyrrolidin-1-yl)-3-amino-2-(2-carboxy-pyrrolidin-1-yl)-pyridine
la 6-(2-carboxy-3-hydroxy-pyrrolidin-1-yl)-3-amino-2-(2-carboxy-pyrrolidin-1-yl)-pyridine
la 6-(2-carboxy-4-hydroxy-pyrrolidin-1-yl)-3-amino-2-(2-carboxy-pyrrolidin-1-yl)-pyridine
la 6-(2-hydroxyméthyl-pyrrolidin-1-yl)-3-amino-2-(2-carboxy-pyrrolidin-1-yl)-pyridine
la 6-(2-hydroxyméthyl-4-hydroxy-pyrrolidin-1-yl)-3-amino-2-(2-carboxy-pyrrolidin-1-yl)-pyridine
la 6-(2-hydroxyméthyl-2-hydroxy-pyrrolidin-1-yl)-3-amino-2-(2-carboxy-pyrrolidin-1-yl)-pyridine
la 6-(2-carboxamido-pyrrolidin-1-yl)-3-amino-2-(2-carboxy-pyrrolidin-1-yl)-pyridine
la 6-(2-diméthylcarboxamido-pyrrolidin-1-yl)-3-amino-2-(2-carboxy-pyrrolidin-1-yl)-pyridine
la 6-(2-carboxamido-3-hydroxy-pyrrolidin-1-yl)-3-amino-2-(2-carboxy-pyrrolidin-1-yl)-pyridine
la 6-(2-carboxamido-4-hydroxy-pyrrolidin-1-yl)-3-amino-2-(2-carboxy-pyrrolidin-1-yl)--pyridine
la 6-(pyrrolidin-1-yl)-3-amino-2-(2-hydroxyméthyl-pyrrolidin-1-yl)-pyridine
la 6-(3-hydroxypyrrolidin-1-yl)-3-amino-2-(2-hydroxyméthyl-pyrrolidin-1-yl)-pyridine
la 6-(3-amino-pyrrofidin-1-yl)-3-amino-2-(2-hydroxyméthyl-pyrrolidin-1-yl)-pyridine
la 6-(3-acétamido-pyrrolidin-1-yl)-3-amino-2-(2-hydroxyméthyl-pyrrolidin-1-yl)-pyridine
la 6-(3,4-dihydroxy-pyrrolidin-1-yl)-3-amino-2-(2-hydroxyméthyl-pyrrolidin-1-yl)-pyridine
la 6-(2-carboxy-pyrrolidin-1-yl)-3-amino-2-(2-hydroxyméthyl-pyrrolidin-1-yl)-pyridine
la 6-(2-carboxy-3-hydroxy-pyrrolidin-1-yl)-3-amino-2-(2-hydroxyméthyl-pyrrolidin-1-yl)-pyridine
la 6-(2-carboxy-4-hydroxy-pyrrolidin-1-yl)-3-amino-2-(2-hydroxyméthyl-pyrrolidin-1-yl)-pyridine
la 6-(2-hydroxyméthyl-pyrrolidin-1-yl)-3-amino-2-(2-hydroxyméthyl-pyrrolidin-1-yl)-pyridine
la 6-(2-hydroxyméthyl-4-hydroxy-pyrrolidin-1-yl)-3-amino-2-(2-hydroxyméthyl-pyrrolidin-1-yl)-pyridine
la 6-(2-hydroxyméthyl-2-hydroxy-pyrrolidin-1-yl)-3-amino-2-(2-hydroxyméthyl-pyrrolidin-1-yl)-pyridine
la 6-(2-carboxamido-pyrrolidin-1-yl)-3-amno-2-(2-hydroxyméthyl-pyrrolidin-1-yl)-pyridine
la 6-(2-diméthylcarboxamido-pyrrolidin-1-yl)-3-amino-2-(2-hydroxymethyl-pyrrolidin-1-yl)-pyridine
la 6-(2-carboxamido-3-hydroxy-pyrrolidin-1-yl)-3-amino-2-(2-hydroxyméthyl-pyrrolidin-1-yl)-pyridine
la 6-(2-carboxamido-4-hydroxy-pyrrolidin-1-yl)-3-amino-2-(2-hydroxyméthyl-pyrrolidin-1-yl)-pyridine
la 6-(pyrrolidin-1-yl)-3-amino-2-(2-carboxamido-pyrrolidin-1-yl)-pyridine
la 6-(3-hydroxypyrrolidin-1-yl)-3-amino-2-(2-carboxamido-pyrrolidin-1-yl)-pyridine
la 6-(3-amino-pyrrolidin-1-yl)-3-amino-2-(2-carboxamido-pyrrolidin-1-yl)-pyridine
la 6-(3-acétamido-pyrrohdm-1-yl)-3-amino-2-(2-carboxamido-pyrrolidin-1-yl)-pyridine
la 6-(3,4-dihydroxy -pyrrolidin-1-yl)-3-amino-2-(2-carboxamido-pyrrolidin-1-yl)-pyridine
la 6-(2-carboxy-pyrrolidin-1-yl)-3-amino-2-(2-carboxamido-pyrrolidin-1-yl)-pyridine
la 6-(2-carboxy-3-hydroxy-pyrrolidin-1-yl)-3-amino-2-(2-carboxamido-pyrrodin-1-yl)-pyridine
la 6-(2-carboxy-4-hydroxy-pyrrolidin-1-yl)-3-amino-2-(2-carboxamido-pyrrolidin-1-yl)-pyridine
la 6-(2-hydroxyméthyl-pyrrolidin-1-yl)-3-amino-2-(2-carboxamido-pyrrolidin-1-yl)-pyridine
la 6-(2-hydroxyméthyl-4-hydroxy-pyrrolidin-1-yl)-3-amino-2-(2-carboxamido-pyrrolidin-1-yl)-pyridine
la 6-(2-hydroxyméthyl-2-hydroxy-pyrrolidin-1-yl)-3-amino-2-(2-carboxamido-pyrrolidin-1-yl)-pyridine
la 6-(2-carboxamido-pyrrolidin-1-yl)-3-amino-2-(2-carboxamido-pyrrolidin-1-yl)-pyridine
la 6-(2-dimethylcarboxamido-pyrrolidin-1-yl)-3-amino-2-(2-carboxamido-pyrrolidin-1-yl)-pyridine
la 6-(2-carboxamido-3-hydroxy-pyrrolidin-1-yl)-3-amino-2-(2-carboxamido-pyrrolidin-1-yl)-pyridine
la 6-(2-carboxamido-4-hydroxy-pyrrolidin-1-yl)-3-amino-2-(2-carboxamido-pyrrolidn-1-yl)-pyridine
la 6-(pyrrolidin-1-yl)-3-amino-2-[méthyl-(2-hydroxyéthyl)amino]pyridine
la 6-(3-hydroxypyrrohdin-1-yl)-3-amino-2-[méthyl-(2-hydroxyéthyl)amino]pyridine
la 6-(3-amino-pyrrolidn-1-yl)-3-amino-2-[méthyl-(2-hydroxyéthyl)amino]pyridine
la 6-(3-acétamido-pyrrolidin-1-yl)-3-amino-2-[méthyl-(2-hydroxyéthyl)amino]pyridine
la 6-(3.4-dihydroxy-yrrolidin-1-yl)-3-amino-2-[méthyl-(2-hydroxyéthyl)amino]pyridine
la 6-(2-carboxy-pyrrolidin-1-yl)-3-amino-2-[méthyl-(2-hydroxyéthyl)amino]pyridine
la 6-(2-carboxy-3-hydroxy-pyrrolidin-1-yl)-3-amino-2-[méthyl-(2-hydroxyéthyl)amino]pyridine
la 6-(2-carboxy-4-hydroxy-pyrrolidin-1-yl)-3-amino-2-[méthyl-(2-hydroxyethyl)amino]pyridine
la 6-(2-hydroxyméthyl-pyrrolidin-1-yl)-3-amino-2-[méthyl-(2-hydroxyéthyl)amino]pyridine
la 6-(2-hydroxyméthyl-4-hydroxy-pyrrolidin-1-yl)-3-amino-2-[méthyl-(2-hydroxyéthyl)amino]pyridine
la 6-(2-hydroxyméthyl-2-hydroxy-pyrrolidin-1-yl)-3-amino-2-[méthyl-(2-hydroxyéthyl)amino]pyridine
la 6-(2-carboxamido-pyrrolidin-1-yl)-3-amino-2-[méthyl-(2-hydroxyéthyl)amino]pyridine
la 6-(2-diméthylcarboxamido-pyrrolidin-1-yl)-3-amino-2-[méthyl-(2-hydroxyéthyl)amino]pyridine
la 6-(2-carboxamido-3-hydroxy-pyrrolidin-1-yl)-3-amino-2-[méthyl-(2-hydroxyéthyl)amino]pyridine
la 6-(2-carboxamido-4-hydroxy-pyrrolidin-1-yl)-3-amino-2-[méthyl-(2-hydroxyéthyl)amino]pyridine
la 6-(pyrrolidm-1-yl)-3-amino-2-[méthyl-(2-aminoéthyl)amino]pyridine
la 6-(3-hydroxypyrrolidin-1-yl)-3-amino-2-[méthyl-(2-aminoéthyl)ammo]pyridine
la 6-(3-amino-pyrrolidin-1-yl)-3-amino-2-[méthyl-(2-aminoéthyl)amino]pyridine
la 6-(3-acétamido-pyrrolidin-1-yl)-3-amino-2-[méthyl-(2-aminoéthyl)amino]pyridine
la 6-(3,4-dihydroxy-pyrrolidin-1-yl)-3-amino-2-[méthyl-(2-aminoéthyl)amino]pyridine
la 6-(2-carboxy-pyrrolidn-1-yl)-3-amino-2-[méthyl-(2-aminoéthyl)amino]pyridine
la 6-(2-carboxy-3-hydroxy-pyrrolidin-1-yl)-3-amino-2-[méthyl-(2-aminoéthyl)amino]pyridine
la 6-(2-carboxy-4-hydroxy-pyrrolidin-1-yl)-3-amino-2-[méthyl-(2-aminoéthyl)amino]pyridine
la 6-(2-hydroxyméthyl-pyrrolidin-1-yl)-3-amino-2-[méthyl-(2-aminoéthyl)amino]pyridine
la 6-(2-hydroxyméthyl-4-hydroxy-pyrrolidin-1-yl)-3-amino-2-[méthyl-(2-aminoéthyl)amino]pyridine
la 6-(2-hydroxyméthyl-2-hydroxy-pyrrolidin-1-yl)-3-amino-2-[méthyl-(2-aminoéthyl)amino]pyridine
la 6-(2-carboxamido-pyrrolidin-1-yl)-3-amino-2-[méthyl-(2-aminoéthyl)amino]pyridine
la 6-(2-diméthylcarboxamido-pyrrolidin-1-yl)-3-amino-2-méthyl-(2-ammoéthyl)amino]pyridine
la 6-(2-carboxamido-3-hydroxy-pyrrolidin-1-yl)-3-amino-2-[méthyl-(2-aminoéthyl)amino]pyridine
la 6-(2-carboxamido-4-hydroxy-pyrrolidin-1-yl)-3-amino-2-[méthyl-(2-aminoéthyl)amino]pyridine
la 6-(pyrrolidin-1-yl)-3-amino-2-[méthyl-(2-acétamidoéthyl)amino]pyridine
la 6-(3-hydroxypyrrolidn-1-yl)-3-ammo-2-[méthyl-(2-acétamidoéthyl)amino]pyridine
la 6-(3-amino-pyrrolidin-1-yl)-3-amino-2-[méthyl-(2-acétamidoéthyl)amino]pyridine
la 6-(3-acétamido-pyrrolidin-1-yl)-3-amino-2-[méthyl-(2-acétamidoéthyl)amino]pyridine
la 6-(3,4-dihydroxy -pyrrolidin-1-yl)-3-amino-2-[méthyl-(2-acétamidoéthyl)amino]pyridine
la 6-(2-carboxy-pyrrolidin-1-yl)-3-amino-2-[méthyl-(2-acétamidoéthyl)amino]pyridine
la 6-(2-carboxy-3-hydroxy-pyrrolidin-1-yl)-3-amino-2-[méthyl-(2-acétamidoéthyl)amino]pyridine
la 6-(2-carboxy-4-hydroxy-pyrrolidin-1-yl)-3-amino-2-[méthyl-(2-acétamidoéthyl)amino]pyridine
la 6-(2-hydroxyméthyl-pyrrolidin-1-yl)-3-amino-2-[méthyl-(2-acétamidoéthyl)ammo]pyridine
la 6-(2-hydroxyméthyl-4-hydroxy-pyrrolidin-1-yl)-3-amino-2-[méthyl-(2-acétamidoéthyl)amino]pyridine
la 6-(2-hydroxyméthyl-2-hydroxy-pyrrolidin-1-yl)-3-amino-2-[méthyl-(2-acétamidoethyl)amino]pyridine
la 6-(2-carboxamido-pyrrolidin-1-yl)-3-amino-2-[méthyl-(2-acétamidoéthyl)amino]pyridine
la 6-(2-diméthylcarboxamido-pyrrolidin-1-yl)-3-amino-2-[méthyl-(2-acétamidoéthyl)amino]pyridine
la 6-(2-carboxamido-3-hydroxy-pyrrolidin-1-yl)-3-amino-2-[méthyl-(2-acétamidoéthyl)amino]pyridine
la 6-(2-carboxamido-4-hydroxy-pyrrolidin-1-yl)-3-amino-2-[méthyl-(2-acétamidoéthyl)amino]pyridine
la 6-(pyrrolidin-1-yl)-3-amino-2-méthoxypyridine
la 6-(3-hydroxypyrrolidin-1-yl)-3-amino-2-méthoxypyridine
la 6-(3-amino-pyrrolidin-1-yl)-3-amino-2-méthoxypyridine
la 6-(3-acétamido-pyrrolidin-1-yl)-3-amino-2-méthoxypyridine
la 6-(3,4-dihydroxy-pyrrolidin-1-yl)-3-amino-2-méthoxypyridine
la 6-(2-carboxy-pyrrolidin-1-yl)-3-amio-2-méthoxypyridine
la 6-(2-carboxy-3-hydroxy-pyrrolidin-1-yl)-3-amino-2-méthoxypyridine
la 6-(2-carboxy-4-hydroxy-pyrrolidin-1-yl)-3-amino-2-méthoxypyridine
la 6-(2-hydroxyméthyl-pyrrolidin-1-yl)-3-amino-2-méthoxypyridine
la 6-(2-hydroxyméthyl-4-hydroxy-pyrrolidin-1-yl)-3-amino-2-méthoxypyridine
la 6-(2-hydroxyméthyl-2-hydroxy-pyrrolidin-1-yl)-3-amino-2-méthoxypyridine
la 6-(2-carboxamido-pyrrolidin-1-yl)-3-amino-2-méthoxypyridine
la 6-(2-diméthylcarboxamido-pyrrolidin-1-yl)-3-amino-2-méthoxypyridine
la 6-(2-carboxamido-3-hydroxy-pyrrolidin-1-yl)-3-amino-2-méthoxypyridine
la 6-(2-carboxamido-4-hydroxy-pyrrolidin-1-yl)-3-amino-2-méthoxypyridine
la 6-(pyrrolidin-1-yl)-3-amino-2-(2-hydroxyéthyloxy)pyridine
la 6-(3-hydroxypyrrolidin-1-yl)-3-amino-2-(2-hydroxyéthyloxy)pyridine
la 6-(3-amino-pyrrolidin-1-yl)-3-amino-2-(2-hydroxyéthyloxy)pyridine
la 6-(3-acétamido-pyrrolidin-1-yl)-3-amino-2-(2-hydroxyéthyloxy)pyridine
la 6-(3,4-dihydroxy -pyrrolidin-1-yl)-3-amino-2-(2-hydroxyéthyloxy)pyridine
la 6-(2-carboxy-pyrrolidin-1-yl)-3-amino-2-(2-hydroxyéthyloxy)pyridine
la 6-(2-carboxy-3-hydroxy-pyrrolidin-1-yl)-3-amino-2-(2-hydroxyéthyloxy)pyridine
la 6-(2-carboxy-4-hydroxy-pyrrolidin-1-yl)-3-amino-2-(2-hydroxyéthyloxy)pyridine
la 6-(2-hydroxyméthyl-pyrrolidin-1-yl)-3-amino-2-(2-hydroxyéthyloxy)pyridine
la 6-(2-hydroxyméthyl-4-hydroxy-pyrrolidin-1-yl)-3-amino-2-(2-hydroxyéthyloxy)pyridine
la 6-(2-hydroxymethyl-2-hydroxy-pyrrolidin-1-yl)-3-amino-2-(2-hydroxyéthyloxy)pyridine
la 6-(2-carboxamido-pyrrolidin-1-yl)-3-amino-2-(2-hydroxyéthyloxy)pyridine
la 6-(2-diméthylcarboxamido-pyrrolidin-1-yl)-3-amino-2-(2-hydroxyéthyloxy)pyridine
la 6-(2-carboxamido-3-hydroxy-pyrrolidin-1-yl)-3-amino-2-(2-hydroxyéthyloxy)pyridine
la 6-(2-carboxamido-4-hydroxy-pyrrolidin-1-yl)-3-amino-2-(2-hydroxyéthyloxy)pyridine
la 6-(pyrrolidin-1-yl)-3-amino-2-éthoxypyridine
la 6-(3-hydroxypyrrolidin-1-yl)-3-amino-2-éthoxypyridine
la 6-(3-amino-pyrrolidin-1-yl)-3-amino-2-éthoxypyridine
la 6-(3-acétamido-pyrrolidin-1-yl)-3-amino-2-éthoxypyridine
la 6-(3,4-dihydroxy-pyrrolidin-1-yl)-3-amino-2-éthoxypyridine
la 6-(2-carboxy-pyrrolidin-1-yl)-3-amino-2-éthoxypyridine
la 6-(2-carboxy-3-hydroxy-pyrrolidin-1-yl)-3-amino-2-ethoxypyridine
la 6-(2-carboxy-4-hydroxy-pyrrolidin-1-yl)-3-amino-2-éthoxypyridine
la 6-(2-hydroxyméthyl-pyrrolidin-1-yl)-3-amino-2-éthoxypyridine
la 6-(2-hydroxyméthyl-4-hydroxy-pyrrolidin-1-yl)-3-amino-2-éthoxypyridine
la 6-(2-hydroxyméthyl-2-hydroxy-pyrrolidin-1-yl)-3-amino-2-éthoxypyridine
la 6-(2-carboxamido-pyrrolidin-1-yl)-3-amino-2-éthoxypyridine
la 6-(2-diméthylcarboxamido-pyrrolidn-1-yl)-3-amino-2-éthoxypyridine
la 6-(2-carboxamido-3-hydroxy-pyrrolidin-1-yl)-3-amino-2-ethoxypyridine
la 6-(2-carboxamido-4-hydroxy-pyrrolidin-1-yl)-3-amino-2-éthoxypyridine
ainsi que leurs sels d'addition.

## Claims

1. Dyeing compositions comprising, in an appropriate dyeing medium, as oxidation base, at least one 2,5-diaminopyridine derivative of formula (T) or the corresponding addition salts: 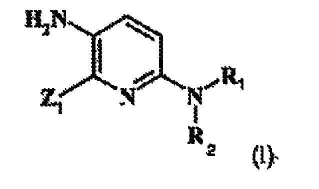 in which
Z₁ represents an OR₃ or NR₄R₅ radical;
R₁, R₂, R₁ and R₅ represent, independently of one another, a hydrogen atom, a C₁-C₆ alkyl, a C₂-C₆ (poly)aminoalkyl radical, a C₃-C₆: (poly)alkylaminoalkyl radical, a C₂-C₆, aminohydroxyalkyl radical, a C₁-C₆ acylaminoalkyl radical, a C₁-C₆ alkoxyalkyl radical, a C₂-C₆ hydroxyalkoxyalkyl radical, a C₂-C₆ aminoalkoxyalkyl radical, a C₂-C₆ (poly)hydroxyalkyl radical, a C₂-C₆ carboxyalkyl radical or a C₃-C₆ sulphoalkyl radical;
R₁ and R₃, on the one hand, and R₄ and R₆, on the other hand, independently of one another, form, with the nitrogen atom to which they are attached, a saturate heterocycle comprising 5 to 8 ring members which is optionally substituted by one or more radical chosen from the group consisting of halogen atoms, amino, (di)alkylamino, hydroxy, carboxyl, carboxamido, alkylsulphonylamino, acetamido and alkoxy radical, and C₁-C₆ alkyl radical optionally substituted by one or more hydroxyl, amino, (di)alkylamino, alkoxy, hydroxyalkoxy, aminoalkoxy, carboxyl, carboxamido, sulphonamido or sulpho radicals, it being possible for the heterocycle to comprise one or more additional heteroatoms chosen from oxygen, nitrogen (optionally substituted) or sulphur or an SO₂ group, the heterocycle comprising neither a peroxide bond nor diazo or nitroso radicals;
R₃ represents a linear or branched C₁-C₆ alkyl radical which can be substituted by one or more radicals chosen from the radicals of the type hydroxy, amino, a C₁-C₃ mono- or dialkylamino radical, the alkyl part of which is optionally substituted by one or more groups of the type hydroxyl, amino, C₁-C₂ alkoxyalkoxy, carboxyl, with the exception of 2,3,6-triaminopyridine and its addition salts.

2. Composition according to claim 1, in which the oxidation base of formula (I) is such that R₃ denotes a methyl, ethyl, hydroxyethyl, carboxymethyl or carboxyethyl radical.

3. Composition according to Claim 1 or 2, in which the oxidation base of formula (I) is such that R₃ denotes ethyl radicals

4. Composition according to any one of Claims 1 to 3, in which the oxidation base of formula (I) is such that R₁, R₂, R₄ and R₅ represents, independently of one another, a C₁-C₄ (di)alkylaminoalkyl radical, a C₂-C₄ carboxyalkyl radical, a C₃-C₄ sulphoalkyl radical, a C₂-C₄ (poly)hydroxyalkyl radical or a C₂-C₄ (poly)aminoalkyl radical.

5. Composition according to any one of Claims 1 to 3, in which the oxidation base cf formula (I) is such that R₁ and R₂, on the one hand, and R₄ and R₅, on the other hand, independently of one another, form, with the nitrogen atom to which they are attached, a 5- to a-membered heterocycle chosen from the heterocycles pyrrolidine, piperidine, homopiperidine, piperazine, homopiperazine or diazepane, it being possible for the said rings to be substituted by one or more C₁-C₄ alkyl radicals optionally substituted by one or more hydroxyl, amino, C₁-C₂ (di)alkylamino, carboxyl, carboxamido or sulphonamido.

6. Composition according to Claim 5, in which the oxidation base of formula (I) is such that R₁ and R₂, on the one hand, and R₄ and R₅, on the other hand, independently of one another, form, with the nitrogen atom to which they are attached, a heterocycle chosen from 2,5-dimethylpyrrolidine, pyrrolidine-2-carboxylic acid, 3-hydroxypyrrolidine-2-carboxylic acid, 4-hydroxypyrrolidine-2-carboxylic acid, 2,4-dicarboxypyrrolidine, 3-hydroxy-2-hydroxymethylpyrrolidine, 2-carboxamidopyrrolidine, 3-hydroxy-2-carboxamidopyrrolidine, 2-(diethylcarboxamido; pyrrolidine, 2-(hydroxymethyl)pyrrolidine, 3,4-dihydroxy-2-(hydroxymethyl)pyrrolidine, 3-hydroxypyrrolidine, 3,4-dihydroxypyrrolidine, 3-aminopyrrolidine, 3-(methylamino)pyrrolidine, 4-amino-3-hydroxypyrrolidine, 3-hydroxy-4-[(2-hydroxyethyl)-amino]pyrrolidine, 2,6-dimethylpiperidine, 2-carboxypiperidine, 2-carboxamidopiperidine, 2-hydroxymethypiperidine, 3-hydroxy-2-hydroxymethylpiperidine, 3-hydroxypiperidine, 4-hydroxypiperidine, 3-hydroxymethylpiperidine, homopiperidine, 2-carboxyhomopiperidine, 2-carboxamidohomopiperidine, diazepane, N-.methyldiazepane or N-(2-hydroxethyl)diazepane.

7. Composition according to Claim. 6, in which the oxidation base of formula (I) is such thar R₁ and R₂, on the one hand, and R₄ and R₅, on the other hand, independently of one another, together form a heterocycle chosen from pyrrolidine, 3-hydroxypyrrolidine, 3-aminopyrrolidine, 3-acetamidopyrrolidine, 3-(methylsulphonylamino)pyrrolidine, pyrrolidine-2-carboxylic acid, 3-hydroxypyrrolidine-2-carboxylic acid, piperidine, hydroxypiperidine, homopiperidine, diazepane, N-methylhomopiperazine or N-(β-hydroxyethyl)homopiperazine.

8. Composition according to any one of Claims 1 to 7, in which the oxidation base of formula (I) is chosen from:
2-methoxy-5- (pyrrolidin-1-yl)-3-aminopyridine
1-(5-amino-6-methoxypyridin-2-yl)pyrrolidin-3-ol
3-amino-2-methoxy-6-(morpholin-4-yl)pyridine,
3-amino-2-methoxy-6-(piperidin-1-yl)pyridine,
6-N,N-(2-hydroxyethyl)methyl)amino)-3-amino-2-methoxypyridine
5,6-diamino-2-(pyrrolidin-1-yl)pyridine
N-(5,6-diaminopyridin-2-yl)-3-hydroxypyrrolidine
2,6-di(pyrrolidin-1-yl)-5-aminopyridine
6-(pyrrolidin-1-yl)-2,3-diaminopyridine
6 (3-hydroxypyrrolidin-1-yl)-2,3-diaminopyridine
6-(3-aminopyrrolidin-1-yl) -2,3-diaminopyridine
6-(3-acetamidopyrrolidin-1-yl)-2,1-diaminopyridine
6-(3,4-dihydroxypyrrolidin-1-yl)-2,3-diaminopyridine
6-(2-carboxypyrrolidin-1-yl)-2,3-diaminopyridine
6-(2-carboxy-3-hydroxypyrrolidin-1-yl)-2,3-diaminopyridine
6-(2-carboxy-4-hydroxypyrrolidin-1-yl)-2,3-diaminopyridine
6-{3-(hydroxymethyl)pyrrolidin-1-yl}-2,3-diaminopyridine
6-(2-hydroxymethyl-4-hydroxypyrrolidin-1-yl)-2,3-diaminopyridine
6-(2-hydroxymethyl-2-hydroxypyrrolidin-1-yl) 2,3-diaminopyridine
6-(2-carboxamidopyrrolidin-1-yl)-2,3-diaminopyridine
6-(2-dimethylcarboxamidopyrrolidin-1-yl)-2,3-diaminopyridine
6-(2-carboxamido-3-hydroxypyrrolidin-1-yl)-2,3-diaminopyridine
6-(2-carboxamido-4-hydroxypyrrolidin-1-yl)-2,3-diaminopyridine
6-(pyrralidin-1-yl)-3-amino-2-methylaminepyridine
6-(3-hydroxypyrrolidin-1-yl)-3-amino-2-methylaminopyridine
6-(3-aminopyrrolidin-1-yl)-3-amino-2-methylaminopyridine
6-(3-acetamidopyrrolidin-1-yl)-3-amino-2-methylaminopyridine
6-(3,4-dihydroxypyrrolidin-1-yl)-3-amino-2-methylaminopyridine
6-(2-carboxypyrrolidin-1-yl)-3-amino-2-methylaminopyridine
6-(2-carboxy-3-hydroxypyrrolidin-1-yl)-3-amino-2-methylaminopyridine
6-(2-carboxy-4-hydroxypyrrolidin-1-yl)-3-amino-2-methylaminopyridine
6-(2-(hydroxymethyl)pyrolidin-1-yl)-3-amino 2-methylaminopyridine
6-(2-hydroxymethyl-4-hydroxypyrrolidin-1-yl)-3-amino-2-methylaminopyridine
6-(2-hydroxymethyl-2-hydroxypyrrolidin-1-yl)-3-amino-2-methylaminopyridine
6-(2-carboxamidopyrrolidin-1-yl)-3-amino-2-methylaminopyridine
6-(2-dimethylcaroxamidopyrrolidin-1-yl)-3-amino-2-methylaminopyridine
6-(2-carboxamido-3-hydroxypyrrolidin-1-yl)-3-amino-2-methylaminopyridine
6-(2-carboxamido-4-hydroxypyrrolidin-1-yl)-3-amino-2-methylaminopyridine
6-(pyrrolidin-1-yl)-3-amino-2-ethylaminopyridine
6-(3-hydroxypyrrolidin-1-yl)-3-amino-2-ethylaminopyridine
6-(3-aminopyrrolidin-1-yl)-3-amino-2-ethylaminopyridine
6-(3-acetamidopyrrolidin-1-yl)-3-amino-2-ethylaminopyridine
6-(3,4-dihydroxypyrrolidin-1-yl)-3-amino-2-ethylaminopyridine
6-(2-carboxypyrrolidin-1-yl)-3-amino-2-ethyl-aminopyridine
6-(2-carboxy-3-hydroxypyrrolidin-1-yl)-3-amino-2-ethylaminopyridine
6-(2-carboxy-4-hydroxypyrrolidin-1-yl)-3-amino-2-ethyl aminopyridine
6-(2-(hydroxymethyl)pyrrolidin-1-yl)-3-amino-2-ethylaminopyridine
6-(2-hydroxymethyl-4-hydroxypyrrolidin-1-yl)-3-amino-2-ethylaminopyridine
6-(2-hydroxymethyl-2-hydroxypyrrolidin-1-yl)-3-amino. 2-ethylaminopyridine
6-(2-carboxamidopyrrolidin-1-yl)-3-amino-2-ethylamino, pyridine
6-(2-dimethylcarboxamidopyrrolidin-1-yl)-3-amino-2-ethylaminopyridine
6-(3-carboxamido-3-hydroxypyrrolidin-1-yl)-3-amino-2-ethylaminopyridine
6-(2-carboxamide-4-hydroxypyrrolidin-1-yl)-3-amino-2-ethylaminopyridine
6-(pyrrolidin-1-yl)-3-amino-2-(2-hydroxyethylamino)-pyridine
6-(3-hydroxypyrrolidin-1-yl)-3-amino-3-(2-hydyoxyethylamino)pyridine
6-(3-aminopyrrolidin-1-yl)-3-amino-2-(2-hydroxyethylamino)pyridine
6-(3-acetamidopyrrolidin-1-yl)-3-amino-2-(2-hydroxyethylamino)pyridine
6-(3,4-dihydroxypyrrolidin-1-yl)-3-amino-2-(2-hydroxyethylamino)pyridine
6-(2-carboxypyrrolin-1-yl)-3-amino-2-(2-hydroxyethylamino)pyridine
6-(2-carboxy-3-hydroxypyrrolidin-1-yl)-3-amino-2-(2-hydroxyethylamino)pyridine
6-(2-carboxy-4-hydroxypyrrolidin-1-yl)-3-amino-2-(2-hydixoxyethylamino)pyridine
6-(2-(hydroxymethyl)pyrrolidin-1-yl)-3-amino-2-(2-hydroxyethylamino)pyridine
6-(2-hydroxymethyl-4-hydroxypyrrolidin-1-yl)-3-amino-2-(2-hydroxyethylamino)pyridine
6-(2-hydroxynethyl-2-hydroxypyrrolidin-1-yl)-3-amino-2-(2-hydroxyethylamino)pyridine
6-(2-carboxanidopyrrolidin-1-yl)-3-amino-2-(2-hydroxy ethylamino)pyridine
6-(3-dimethylcarboxamidopyrrolidin-1-yl)-3-amino-2-(2-hydroxyethylamino)pyridine
6-(2-carboxamido-3-hydroxypyrrolidin-1-yl)-3-amino 2-(2-hydroxyethylamino)pyridine
6-(2-carboxamido-4-hydroxypyrrolidin-1-yl)-3-aminopyridine
6-(pyrrolidin-1-yl)-3-amino-2-(2-aminoethylamine)-pyridine
6-(3-hydroxypyrrolidin-1-yl)-3-amino-2-(2-aminoethylamino)pyridine
6-(3-aminopyrrolidin-1-yl)-3-amino-2-(2-aminoethylamino)pyridine
6-(3-acetamidopyrrolidin-1-yl)-3-amino-2-(2-aminoethylamino)pyridine
6-(3,4-dihydroxypyrrolidin-1-yl)-3-amine-2-(2-aminoethylamino)pyridine
6-(2-carboxypyrrolidin-1-yl)-3-amino-2-(2-aminoethylamino)pyridine
6-(2-carboxy-3-hydroxypyrrolidin-1-yl)-3-amino-2-(2-aminoethylamino)pyridne
6-(2-carboxy-4-hydroxypyrrolidin-1-yl)-3-amino-2-(2-aminoethylamino)pyridine
6-(2-(hydroxymethyl)pyrrolidin-1-yl)-3-amino 2-(2-aminoethylamino)pyridine
5-(2-hydroxymethyl-4-hydroxypyrrolidin-1-yl)-3-amino-2-(2-aminoethylamino)pyridine
6-(2-hydroxymethyl-2-hydroxypyrrolidin-1-yl)-3-amino-2-(2-aminoethylamino)pyridine
6-(2-carboxamidopyrrolidin-1-yl)-3-amino-2-(2-aminoethylamino)pyridine
6-(2-dimethylcarboxamidopyrrolidin-1-yl)-3-amino-2-(2-aminoethylamino)pyridine
6-(2-carboxamido-3-hydroxypyrrolidin-1-yl)-3-amino-2-(2-aminoethylamino)pyridine
6-(2-carboxamido-4-hydroxypyrrolidin-1-yl)-3-amino-2-(2-aminoethylamino)pyridine
6-(pyrrolidin-1-yl)-3-amino-2 (2-acetamidoethylamino) pyridine
6-(3-hydroxypyrrolidin-1-yl)-3-amino-2-(2-acetamidoethylamino)pyridine
6-(3-aminopyrrolidin-1-yl)-3-amino-2-(2-acetamidoethylamino)pyridine
6-(3-acetamidopyrrolidin-1-yl)-3-amino-2-(2-acetamidoethylamino)pyridine
6-(3,4-dihydroxypyrrolidin-1-yl)-3-amino-2-(2-acetatmidoethylamino)pyridine
6-(2-carboxypyrrolidin-1-yl)-3-amino-2-(2-acetamidoethylamino}pyridine
6-(2-carboxy-3-hydroxypyrrolidin-1-yl)-3-amino-2-(2-acetamidoethylamino)pyridine
6-(2-carboxy-4-hydroxypyrrolidin-1-yl)-3-amino-2-(2-aceramidoethylamino)pyridine
6-(2-(hydroxymethyl)pyrrolidin-1-yl)-3-amino-2-(2-acetamidoethyl-amino)pyridine
6-(2-hydroxmethyl-4-hydroxypyrrolidin-1-yl)-3-amino-2-(2-acetamidoethhylamino)pyridine
6-(2-hydroxymethyl-2-hydroxypyrrolidin-1-yl)-3-amino-2-(2-acetamidoethylamino)pyridine
6-(2-carboxamidopyrrolidin-1-yl)-3-amino-2-(2-acetamidoethylamino)pyridine
6-(2-dimethylcarboxamidopyrrolidin-yl)-3-amino-2-(2-acetanidoethylamino)pyridine
6-(2-carboxamido-3-hydroxypyrrolidin-1-yl)-3-amino-2-(2-acetamidoethylamino) pyridine
6-(2-carboxamido-4-hydroxypyrrolidin-1-yl)-3-amino-2-(2-acetamidoethylamino)pyridine
6-(pyrrolidin-1-yl)-3-amino-2-(pyrrolidin-1-yl)pyridine
6-(3-hydroxypyrrolidin-1-yl)-3-amino-2-(pyrrolidin-1-yl)pyridine
6-(3-aminopyrrolidin-1-yl)-3-amino-2-(pyrrolidin-1-yl)pyridine
6-(3-acetamidopyrrolidin-1-yl)-3-amino-2-(pyrrolidin-1-yl)pyridine
6-(3,4-dihydroxypyrrolidin-1-yl)-3-amino-2-(pyrrolidin-1-yl)pyridine
6-(2-carboxypyrrolidin-1-yl)-3-amino-2-(pyrrolidin-1-yl)pyridine
6-(2-carboxy-3-hydroxypyrrolidin-1-yl)-3-amino-2-(pyrrolidin-1-yl)pyridine
6-(2-carboxy-4-hydroxypyrrolidin-1-yl)-3-amino-2-(pyrrolidin-1-yl)pyridine
6-(2-(hydroxymethyl)pyrrolidin-1-yl)-3-amino-2-(pyrrolidin-1-yl)pyridine
6-(2-hydroxymethyl-4-hydroxypyrrolidin-1-yl)-3-amino-2-(pyrrolidin-1-yl)pyridine
6-(2-hydroxymethyl-2-hydroxypyrrolidin-1-yl)-3-amino-2-(pyrrolidin-1-yl)pyridine
6-(2-carboxamidopyrrolidin-1-yl)-3-amino-2-(pyrrolidin-1-yl)pyridine
6-(2-dimethylcarboxamidopyrrolidin-1-yl)-3-amino-2-(pyrrolidin-1-yl)pyridine
6-(2-carboxamido-3-hydyoxypyrrolidin-1-yl)-3-amino-2-(pyrrolidin-1-yl)pyridine
6-(2-carbaxamido-4-hydroxypyrrolidin-1-yl)-3-amino-2-(pyrrcidin-1-yl)pyridine
5-(pyrrolidin-1-yl)-3-amino-2-(3-hydroxypyrrolidin-1-yl)pyridine
6-(3-hydroxypyrrolidin-1-yl)-3-amino-2-(3-hydroxpyrrolidin-1-yl)pyridine
6-(3-aminopyrrolidin-1-yl)-3-amino-2-(3-hydroxypyrrolidin-1-yl)pyridine
6-(3-acetamidopyrrolidin-1-yl)-3-amino-2-(3-hydroxypyrrolidin-1-yl)pyridine
6-(3,4-dihydroxypyrrolidin-1-yl)-3-amino-2-(3-hydroxypyrrolidin-1-yl)pyridine
6-(2-carboxypyrrolidin-1-yl)-3-amino-2-(3-hydroxypyrrolidin-1-yl)pyridine
6-(2-carboxy-3-hydroxypyrrolidin-1-yl)-3-amino-2-(3-hydroxypyrrolidin-1-yl)pyridine
6-(2-carboxy-4-hydroxypyrrolidin-1-yl)-3-amino-2-(3-hydroxypyrrolidin-1-yl)pyridine
6-(2-(hydroxymethyl)pyrrilidin-1-yl)-3-amino-2-(3-hydroxypyrrolidin-1-yl)pyridine
6-(2-hydroxymethyl-4-hydroxypyrrolidin-1-yl)-3-amino-2-(3-hydroxypyrrolidin-1-yl)pyridine
6-(2-hydroxytrethyl-2-hydroxypyrrolidin-1-yl)-3-amino-2-(3-hydroxypyrrolidin-1-yl)pyridine
6-(2-carboxamidopyrrolidin-1-yl)-3-amino-2-(3-hydroxypyrrolidin-1-yl)pyridine
6-(2-dimethylcarboxamidopyrrolidin-1-yl)-3-amino-2-(3-hydroxypyrrolidin-1-yl)pyridine
6-(2-carboxamido-3-hydroxypyrralidin-1-yl)-3-amino-2-(3-hydroxypyrrolidin-1-yl)pyridine
6-(2-carboxamido-4-hydroxypyrrolidin-1-yl)-3-amino-2-(3-hydroxypyrrolidin-1-yl)pyridine
6-(pyrrolidin-1-yl)-3-amino-2-(2-carboxypyrrolidin-1-yl)pyridine
6-(3-hydroxypyrrolidin-1-yl)-3-amino-2-(2-carboxypyrrolidin-1-yl)pyridine
6-(3-aminopyrrolidin-1-yl)-3-amino-2-(2-carboxypyrrolidin-1-yl)pyridine
6-(3-acetamidopyrrolidin-1-yl)-3-amino-2-(2-carboxypyrrolidin-1-yl)pyridine
6-(3,4-dinydroxypyrrolidin-1-yl)-3-amino-2-(2-carboxypyrrolidin-1-yl)pyridine
6-(2-carboxypyrrolidin-1-yl)-3-amino-2-(2-carboxypyrrolidin-1-yl)pyridine
6-(2-carboxy-3-hydroxypyrrolidin-1-yl)-3-amino-2-(2-carboxypyrrolidin-1-yl)pyridine
6-(2-carbxy-4-hydroxypyrrolidin-1-yl)-3-amino-2-(2-carboxypyxrolid-n-1-yl)pyridine
6-(2 (hydxoxymethyl)pyrrolidin-1-yl)-3-amino-2-(2-carboxypyrrolidin-1-yl)pyridine
6-(2-hydroxymethyl-4-hydroxypyrrolidin-1-yl)3-amino-2-(2-carboxypyrrolidin-1-yl)pyridine
6-(2-hydroxymethyl-2-hydroxypyrrolidin-1-yl)-3-amino-2-(2-carboxypyrrolidin-1-yl)pyridine
6-(2-carboxamidopyrrolidin-1-yl)-3-amino-2-(2-carboxypyrrolidin-1-yl)pyridine
6-(2 dimethylcarboxamidopyrrolidin-1-yl)-3-amino-2-(2-carboxypyrrolidin-1-yl)pyridine
6-(2-carboxamido-3-hydroxypyrrolidin-1-yl)-3-amino-2-(2-carboxypyrrolidin-1-yl)pyridine
6-(2-carboxamido-4-hydroxypyrrolidin-1-yl)-3-amino-2-(2-carboxypyrrolidin-1-yl)pyrrodine
6-(pyrrolidin-1-yl)-3-amino-2-(2-(hydroxymethyl)-pyrrolidin-1-yl)pyridine
6-(3-hydroxypyrrolidin-1-yl)-3-amino-2-(2-(hydroxymethyl)pyrrolidin-1-yl)pyridine
6-(3-aminopyrrolidin-1-yl)-3-amino-2-(2-(hydroxymethyl)pyrrolidin-1-yl)pyridine
6-(3-acetamidopyrrolidin-1-yl)-3-amino-2-(2-(hydroxymethyl)pyrrolidin-1-yl)pyridine
6-(3,4-dihydroxypyrrolidin-1-yl)-3-amino-2-(2-(hydroxymethyl)pyrrolidin-1-yl)pyridine
6-(2-carboxypyrrolidin-1-yl)-3-amino-2-(2-(hydroxymethyl)pyrrolidin-1-yl)pyridine
6-(2-carboxy-3-hydroxypyrrolidin-1-yl)-3-amino-2-(2-(hydroxymethyl)pyrrolidin-1-yl)pyridine
6-(2-carboxy-4-hydroxypyrrolidin-1-yl)-3-amino-2-(2-(hydroxymethyl)pyrrolidin-1-yl)pyridine
6-(2-(hydroxymethyl)pyrrolidin-1-yl)-3-amino-2-(2-(hydroxymethyl)pyrrolidin-1-yl)pyridine
6-(2-hydroxymethyl-4-hydroxypyrrolidin-1-yl)-3-amino-2-(2-(hydroxymethyl)pyrrolidin-1-yl)pyridine
6-(2-hydroxymethyl-2-hydroxypyrrolidin-1-yl)-3-amino-2-(2-(hydroxymethyl)pyrrolidin-1-yl)pyrridine
6-(2-carboxamidopyrrolidin-1-yl)-3-amino-3-(2-(hydroxymethyl)pyrrolidin-1-yl)pyridine
6-(2-dimethylcarboxamidopyrrolidin:1-1-yl)-3-amino-2-(2-(hydroxymethyl)pyrrolidin-1-yl)pyridine
6-(2-carboxamido-3-hydroxypyrrolidin-1-yl)-3-amino-2-(2-(hydroxymethyl)pyrrolidin-1-yl)pyridine
6-(2-carboxamido-4-hydroxypyrrolidin-1-yl)-3-amino-2-(2-(hydroxymethyl)pyrrolidin-1-yl)pyridine
6-(pyrrolidin-1-yl)-3-amino-2-(2-carboxamidopyrrolidin-1-yl)pyridine
6-(3-hydroxypyrrolidin-1-yl)-3-amino-2-(2-carboxamidopyrrolidin-1-yl)pyridine
6-(3-aminopyrrolidin-1-yl)-3-amino-2-(2-carboxamidopyrrolidin-1-yl)pyridine
6-(3-acetamidopyrrolidin-1-yl)-3-amino-2-(2-carboxamidopyrrolidin-1-yl)pyridine
6-(3,4-dihydroxypyrrolidin-1-yl)-3-amino-2-(2-carboxamidopyrrolidin-1-yl)pyridine
6-(2-carboxypyrrolidin-1-yl)-3-amino-2-(2-carboxamidopyrrolidin-1-yl)pyridine
6-(2-carboxy-3-hydroxypyrrolidin-1-yl)-3-amino-2-(2-carboxamidopyrrolidin-1-yl)pyridine
6-(2-carboxy-4-hydroxypyrrolidin-1-yl)-3-amino-2-(2-carboxamidopyrrolidin-1-yl)pyridine
6-(3-(hydroxymethyl)pyrrolidin-1-yl)-3-amino-(2-carboxamidopyrrolidin-1-yl)pyridine
6-(2-hydroxymethyl-4-hydroxypyrrolidin-1-yl)-3-amino-2-(2-carboxamidopyrrolidin-1-yl)pyridine
6-(2-hydroxymethyl-2-hydroxypyrrolidin-1-yl)-3-amino-2-(2-carboxamidopyrrolidin-1-yl)pyridine
6-(2-carboxamidopyrrolidin-1-yl)-3-amino-2-(2-carboxamidopyrrolidin-1-yl)pyridine
6-(2-dimethylcarboxamidopyrrolidin-1-yl)-3-amino-2-(2-carboxamidopyrcolidin-1-yl)pyridine
6-(2-carboxamido-3-hydroxypyrrolidin-1-yl)-3-amino-2-(2-carboxamidopyrrolidin-1-yl)pyridine
6-(2-carboxamido-4-hydroxypyrrolidin-1-yl)-3-amino-2-(2-carboxamidopyrrolidin-1-yl)pyridine
6-(pyrrolidin-1-yl)-3-amino-2-dimethylaminopyridine
6-(3-hydroxypyrrolidin-1-yl)-3-amino-2-dimethylaminopyridine
6-(3-aminopyyrolidin-1-yl)-3-amino-2-dimethylaminopyridine
6-(3-acetamidopyrrolidin-1-yl)-3-amino-2-dimethylaminopyridine
6-(3,4-dihydroxypyrrolidin-1-yl)-3-amino-2-dimenthylaminopyridine
6-(2-carboxypyrrolidin-1-yl)-3-amino-2-dimethylaminopyridine
6-(2-carboxy-3-hydroxypyrrolidin-1-yl)-3-amino-2-dimethylaminopyridine
6-(2-carboxy-4-hydroxypyrrolidin-1-yl)-3-amino-2-dimethylaminopyridine
6-(2-(hydroxymethyl)pyrrolidin-1-yl)-3-amino-2-dimethylaminopyridine
6-(2-hydroxymethyl-4-hydroxypyrrolidin-1-yl)-3-amino-2-dimethylaminopyridine
6-(2-hydroxymethyl-2-hydroxypyrrolidin-1-yl)3-amino-2-dimethylaminopyridine
6-(2-carboxamidopyrrolidin-1-yl)-3-amino-2-dimethylaminopyridine
6-(2-dimethylcarboxamidopyrrolidin-1-yl)-3-amino-2-dimethylaminopyridine
6-(2-carboxamido-3-hydroxypyrrolidin-1-yl)-3-amino-2-dimethylaminopyridine
6-(2-carboxamido-4-hydroxypyrrolidin-1-yl)-3-amino-2-dimethylaminopyridine
6-(pyrrolidin-1-yl)-3-amino-2-[methyl(2-hydroxyethyl)-amino]pyridine
6-(3-hydroxypyrrolidin-1-yl)-3-amino-2-[methyl(2-hydroxyethyl)amino]pyridine
6-(3-aminopyrrolidin-1-yl)-3-amino-2-[methyl(2-hydroxyethyl)amino]pyridine
6-(3-acetamidopyrrolidin-1-yl)-3-amino-2-[methyl(2-hydroxyethyl)amino]pyridine
6-(3,4-dihydraxypyrrolidin-1-yl)-3-amino-2-[methyl(2-hydroxyethyl)amino]pyridine
6-(2-carboxypyrrolidin-1-yl)-3-amino-2-[methyl(2-hydroxyethyl)amino]pyridine
6-(2-carboxy-3-hydroxypyrrolidin-1-yl)-3-amino-2-[methyl(2-hydroxyethyl)amino]pyridine
6-(2-carboxy-4-hydroxypyrrolidin-1-yl)-3-amino-2-[methyl(2-hydroxyethyl)amino]pyridine
6-(2-(hydroxymethyl)pyrrolidin-1-yl)-3-amino-2-[methyl(2-hydroxyethyl)amino]pyridine
6-(2-hydroxymethyl-4-hydroxypyrrolidin-1-yl)-3-amino-2-[methyl(2-hydroxyethyl)amino]pyridine
6-(2-hydroxymethyl-2-hydroxypyrrolidin-1-yl)-3-amino-2-[methyl(2-hydroxyethyl)amino]pyridine
6-(2-carboxamidopyrrolidin-1-yl)-3-amino-2-[methyl(2-hydroxyethyl)amino]pyridine
6-(2-dimethylcarboxamidopyrrolidin-1-yl)-3-amino-2-[methyl(2-hydroxyethyl)amino]pyridine
6-(2-carboxamido-3-hydroxypyrrolidin-1-yl)-3-amino-2-[methyl(2-hydroxyethyl)amino]pyridine
6-(2-carboxamido-4-hydroxypyrrolidin-1-yl)-2-amino-2-[methyl(2-hydroxyethyl)amino]pyridine
6-(pyrrolidin-1-yl)-3-amino-2-[methyl(2-aminoethyl)-amino]pyridine
6-(3-hydroxypyrolidin-1yl)-3-amino-2-[methyl(2-aminoethyl)amino]pyridine
6-(3-aminopyrrolidin-1-yl)-3-amino-2-[methyl(2-aminoethyl)amino]pyridine
6-(3-acetamidopyrrolidin-1-yl)-3-amino-2-methyl(2 aminoethyl)amino pyridine
6-(3,4-dihydroxypyrrolidin-1-yl)-3-amino-2-[methyl(2-aminoethyl)amino]pyridine
6-(2-carboxypyrrolidin-1-yl)-3-amino-2-[methyl(2-aminoethyl)amino]pyridine
6-(3-carboxy-3-hydroxypyrrolidin-1-yl)-3-amino-2-[methyl(2-aminoethyl)amino]pyridine
6-(2-carboxy-4-hydroxypyrrolidin-1-yl)-3-amino-2-[methyl(2-aminoethyl)amino]pyridine
6-(2-(hydroxymethyl)pyrrolidin-1-yl)-3-amino-2-[methyl(2-aminoethyl)amino]pyridine
6-(2-hydroxymethyl-4-hydroxypyrrolidin-1-yl)-3-amino-2-[methyl(2-aminoethyl)amino]pyridine
6-(2-hydroxymethyl-2-hydroxypyrrolidin-1-yl)-3-amino-2-[methy-(2-aminoethyl)amino]pyridine
6-(2-carboxamidopyrrolidin-1-yl)-3-amino-2-[methyl(2-amioethyl)amino]pyridine
6-(2-dimethylcarboxanidopyrrolidin-1-yl)-3-amino-2-[methyl(2-aminoethyl)amino]pyridine
6-(2-carboxamido-3-hydroxypyrrolidin-1-yl)-3-amino-2-[methyl(2-aminoethyl)amino]pyridine
6-(2-carboxamido-4-hydroxypyrrolidin-1-yl)-3-amino-2-[Methyl(2-aminoethyl)amino]pyridine
6-(pyrrolidin-1-yl)-3-amino-2-[methyl(2-acetamidoethyl)amino]pyridine
6-(3-hydroxypyrrolidin-1-yl)-3-amino-2-[methyl(2-acetamidoethyl)amino]pyridine
6-(3-aminopyrrolidin-1-yl)-3-amino-2-[methyl(2-acetamidoethyl)amino]pyridine
6-(3-acetamidopyrrolidin-1-yl)-3-amino-2-[methyl(2-acetamidoethyl)amino]pyridine
6-(3,4-dihydroxypyrrolidin-1-yl)-3-amino-2-[methyl(2-acetamidoethyl)amino]pyridine
6-(2-carboxypyrrolidin-1-yl)-3-amino-2-[methyl(2-acetamidoethyl)amino]pyridine
6-(2-carboxy-3-hydroxypyrrolidin-1-yl)-3-amino-2-[methyl(2-acetamidoethyl)amino]pyridine
6-(2-carboxy-4-hydroxypyrrolidin-1-yl)-3-amino-2-[methyl(2-acetamidoethyl)amino]pyridine
6-(2-hydroxymethyl)pyrrolidin-1-yl)-3-amino-2-[methyl(2-acetamidoethyl)amino]pyridine
6-(2-hydroxymethyl-4-hydroxypyrrolidin-1-yl)-3-amino-2-[methyl(2-acetamidoethyl)amino]pyridine
6-(2-hydroxymethyl-3-hydroxypyyrolidin-1-yl)-3-amino-2-[methyl(2-acetamidoethyl)amino]pyridine
6-(2-carboxamidopyrrolidin-1-yl)-3-amino-2-[methyl(2-acetamidoethyl)amino]pyridine
6--(2-dimethylcarboxamidopyrrolidin-1-yl)-3-amino-2-[methyl(2-acetamidoethyl)amino]pyridine
6-(2-carboxamido-3-hydroxypyrrolidin-1-yl)-3-amino-2-[methyl(2-azetamidoerthyl)amino]pyridine
6-(2-carboxamido-4-hydroxypyrrolidin-1-yl)-3-amino-2-[methyl-2-acetamidoethyl)amino]pyridine
6-(pyrrolidin-1-yl)-3-amino-2-methoxypyridine
6-(3-hydroxypyrrolidin-1-yl)-3-amino-2-methoxypyridine
6-(3-aminopyrrolidin-1-yl)-3-amino-2-methoxypyridine
6-(3-acetamidopyrrolidin-1-yl)-3-amino-2-methoxypyridine
6-(3,4-dihydroxypyrrolidin-1-yl)-3-amino-2-methoxypyridine
6-(2-carboxypyrrolidin-1-yl)-3-amino-2-methoxypyridine 6-(2-carboxy-3-hydroxypyrrolidin-1-yl)-3-amino-2-methoxypyridine
6-(2-carboxy-4-hydroxypyrrolidin-1-yl)-3-amino-2-methoxypyridine
6-(2-(hydroxymethyl)pyrrolidin-1-yl)-3-amino-2-methoxypyridine
6-(2-hydroxymethyl-4-hydroxypyrrolidin-1-yl)-1-amino-2-methoxypyridine
6-(2 hydroxymethyl-2-hydroxypyrrolidin-1-yl)-3-amino-2-methoxypyridine
6-(2-carboxamidopyrrolidin-1-yl)-1-amino-2-methoxypyridine
6-(2-dimethylcarboxamidopyrrolidin-1-yl)-3-amino-2-methoxypyridine
6-(2-carboxamido-3-hydroxypyrrolidin-1-yl)-3-amino-2 methoxypyridine
6-(2-carboxamido-4-hydroxypyrrolidin-1-yl)-3-amino-2-methoxypyridine
6-(pyrrolidin-1-yl)-3-amino-2-(2-hydroxyethyloxy)-pyridine
6-(3-hydroxypyrrolidin-1-yl)-3-amino-2-(2-hydroxyethyloxy)pyridine
6-(3-aminopyrrolidin-1-yl)-3-amino-2-(2-hydroxyethyloxy)pyridine
6-(3-acetamidopyrolidin-1-yl)-3-amino-2-(2-hydroxyethyloxy)pyridine
6-(3,4-dihydroxypyrrolidin-1-yl)-3-amino-2-(2-hydroxyethyloxy)pyridine
6-(2-carboxypyrrolidin-1-yl)-3-amino-2-(2-hydroxyethyloxy)pyridine
6-(2-carboxy-3-hydroxypyrrolidin-1-yl-)-3-amino-2-(2-hydroxyethyloxy)pyridine
6-(2-carboxy-4-hydroxypyrrolidin-1-yl)-3-amino-2-(2-hydroxyethyloxy)pyridine
6-(2-hydroxymethyl)pyrrolidin-1-yl)-3-amino-2-(2-hydroxyethyylox)pyridine
6-(2-hydroxymethyl-4-hydroxypyrrolidin-1-yl)-3-amino-2-(3-hydroxyethyloxy)pyridine
6-(2-hydroxymethyl-2-hydroxypyrrolidin-1-yl)-3-amino-2-(2-hydroxyethyoxy)pyridine
6-(2-carboxamidopyrrolidin-1-yl)-3-amino-2-(2-hydroxyethyloxy)pyridine
6-(2-dimethylcarboxamidopyrrolidin-1-yl)-3-amino-2-(2-hydroxyethyloxy)pyridine
6-(2-carboxamido-3-hydroxypyrrolidin-1-yl)-3-amino-2-(2-hydroxyethyloxy)pyridine
6-(2-carboxamido-4-hydroxypyrrolidin-1-yl)-3-amino-2-(2-hydroxyethyloxy) pyridine
6-(pyrrolidin-1-yl)-3-amino-2-ethoxypyridine
6-(3-hydroxypyrrolidin-1-yl)-3-amino-2-ethoxyridine
6-(3-hypyrolidin-1-yl)-3-amino-3-ethoxypyridine
6-(3-acetamidopyrrolidin-1-yl)-3-amino-2-ethoxypyridine
6-(3,4-dihydroxypyrrolidin-1-yl)-3-amino-3-ethoxypyridine
6-(2-carboxypyrrolidin-1-yl)-3-amino-2-ethoxypyridine
6-(2-carboxy-3-hydroxypyrrolidin-1-yl)-3-amino-2-ethoxypyridine
6-(2-carboxy-4-hydroxypyrrolidin-1-yl)-3-amino-2-ethoxypyridine
6-(2-(hydroxymethyl)pyrrolidin-1-yl)-3-amino-2-ethoxypyridine
6-(2-hydroxymethyl-4-hydroxypyrrolidin-1-yl)-3-amino-2-ethoxypyridine
6-(2-hydroxymethyl-2-hydroxypyrrolidin-1-yl)-3-amino-2-ethoxypyridine
6-(2-carboxamidopyrrolidin-1-yl)-3-amino-2-ethoxypyridine
6-(2-dimethylcarboxamidopyrrolidin-1-yl)-3-amino-2-ethoxypyridine
6-(2-carboxamido-3-hydroxypyrrolidin-1-yl)-3-amino-2-ethoxypyridine
6-(2-carboxamido-4-hydroxypyrralidin-1-yl)-3-amino 2-ethoxypyridine
and their addition salts.

9. Composition according to claim 8, in which the oxidation base of formule (I) is chosen frow:
2-methoxy-6-(pyrrolidin-1-yl)-3-aminopyridine
5,6-diamino-2-(pyrrolidin-1-yl)pyridine
N-(5,6-diaminopyridin-2-yl)-3-hydroxypyrrolidine
2,6-di(pyrrolidin-1-yl)-5-aminopyridine
and their addition salts.

10. Composition according to any one of Claims 1 to 9, furthermore comprising one or more additional oxidation bases chosen from para-phenylenediamines, bisphenylalkylenediamines, para-aminophenols, ortho-aminophenols, heterocyclic bases, other than these defined in Claims 1 to 9, and their addition salts.

11. Composition according to any one of Claims 1 to 10, in which the oxidation base or bases of formula (1) or the additional base or bases are each present in an amount of between 0.001 and 10% by weight of the total weight of the dyeing composition.

12. Composition according to any one of the preceding claims, comprising one or more couplers chosen from meta-phenylenediamines, meta-aminophenols, meta-diphenols, naphtalene couplers and heterocyclic couplers, and their addition salts.

13. Composition according to Claim 12, in which the coupler or couplers are present in an amount of between 0,001 and 10% by weight of the total weight of the dyeing composition.

14. Composition according to any one of Claims 1 to 13, comprising an oxidizing agent.

15. Composition according to Claim 14, in which the oxidizing agent is chosen from hydrogen peroxide, urea hydrogen peroxide, alkali metal bromates, persalts, peracids and oxidase enzymes.

16. Process for the oxidation dyeing of keratinous fibres, characterize in that at least one composition as defined in any one of Claims 1 to 13 is applied to the fibres and in that the colour is developed using an oxidizing agent.

17. Process according to Claim 16, in which the oxidizing agent is chosen from hydrogen peroxide, urea hydrogen peroxide, alkali metal bromates, persalts, peracids and oxidase enzymes.

18. Process according to either of Claims 16 and 17, in which the oxidizing agent is mixed at the time of use with the composition as defined according to any one of Claims 1 to 13.

19. Process according to either one of Claims 16 and 17, in which the oxidizing agent is applied to the fibres in the form of an oxidizing composition simultaneously with or sequentially to the composition as defined according to any one of Claims 1 to 13.

20. Dyeing multi-compartment device or dyeing "kit" comprising several compartments, in which a first dyeing compartment comprises a composition as defined in any one of Claims 1 to 13 and a second compartment comprises an oxidizing composition.

21. Coloured product capable of being obtained by oxidative coupling of a composition as defined according to any one of Claims 1 to 13.

22. Use of the composition as defined according to any one of Claims 1 to 9 for the dyeing of keratinous fibres.

23. 2,5-Diaminopyridine derivative of following formula (I'): 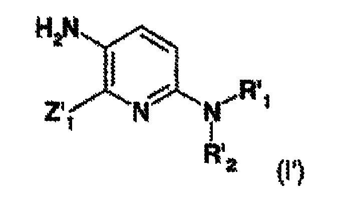 in which:
Z'₁ represents an OR'₃ or NR'₄R'₅ radical;
R'_{1,} R'₂, R'₄ and R'₅ represent, independently of one another, a hydrogen atom, a C₁-C₆ (poly)aminoalkyl radical, a C₂-C₆ (poly)alkylaminoalkyl radical, a C₂-C₆ aminohydroxyalkyl radical, a C₂-_{C6} acylaminoalkyl radical, a C₁-C₆ alkoxyalkyl radical, a C₂-C₆ hydroxyalkoxyalkyl radical, a C₁-C₆ aminoalkoxyalkyl radical, a C₁-C₆ (polyl)hydroxyalkyl radical, a C₂-C₆ carboxyalkyl radical or a C₂-C₆ sulphoalkyl radical;
R'₁, and R'₂, on the one hand, and R'₄ and R'₅, on the other hand, independently of one another, form, with the nitrogen atom to which they are attached, a saturated heterocycle comprising 5 to 8 ring members which is optionally substituted by one or more radicals chosen from the group consisting of halogen atoms, amino, (di) alkylamino, hydroxyl, carboxyl, carboxamido and alkoxy radicals, and C₁-C₆ alkyl radicals optionally substituted by one or more hydroxy, amino, (di) alkylamino, alkoxy, hydroxyalkoxy, aminoalkoxy, carboxyl or sulpho radicals, it being possibly for the heterocycle to comprise one or more additional heteroatoms chosen from oxygen, nitrogen (optionally substituted) or sulphur or an SO₂ group, the heterocycle comprising neither a peroxide bond nor diazo or nitroso radicals;
R'₄ represents a linear or branched C₁-C₆ alkyl radical which can be substituted by one or more radicals chosen from the radical of the type hydroxyl, amino, a C₁-C₃ mono- or dialkylamino radical, the alkyl part of which is optionally substituted by one or more groups of the type hydroxy, amino, C₁-C₂ alkoxyalkoxy, carboxyl; with the exception of 2,3,6-triaminopyridine, of 6-(4-morpholinyl)-2,3-diaminopyridine, of 6-(1-piperidinyl)-2,3-diaminopyridine and of 2,6-di(4-morpholinyl)-3-aminopyridine, and their addition salts;
with the exception of 2,3-diamino-6-(4-methylpiperazinyl)pyridine.

24. 2, 5-Diaminopyridine derivative of formula (I') according to Claim 23, chosen from the following compounds:
2-methoxy-6-(pyrrolidin-1-yl)-3-aminopyridine
1-(5-amino-6-methoxypyridin-2-yl)pyrrolidin-3-ol
3-amino-2-methoxy-6-(morpholin-4-y')pyridine,
3-amino-2 -methoxy-6-(piperidin-1-yl)pyridine,
6-N,N-(2-hydroxyethyl)-methyl}amino)-3-amino-2-methoxypyridine
5,6-diamino-2-(pyrrolidin-1-yl)pyridine
N-(5,6-diaminopyridin-2-yl;-3-hydroxypyrrolidine
2,6-di(pyrrolidin-1-yl)-5-aminopyridine
6-(pyrrolidin-1-yl)-2,3-diaminopyridine
6-(3-hydroxypyrrolidin-1-yl)-2,3-diaminopyridine
6-(3-aminopyrrolidin-1-yl)-2,3-diaminopyridine
6-(3-acetamidopyrrolidin-1-yl)-2,3-diaminopyridine
6-(3,4-dihydroxypyrrolidin-1-yl) -2,3-diaminopyridine
6-(2-carboxypyrrolidin-1-yl)-2,3-diaminopyridine
6-(2-carboxy-3-hydroxypyrrolidin-1-yl)-2,3-diaminopyridine
6-(2-carboxy-4-hydroxypyrrolidin-1-yl)-2,3-diaminopyridine
6-(2-(hydroxymethyl)pyrrolidin-1-yl)-2,3-diaminopyridine
6-(2-hydroxymethyl-4-hydroxypyrrolidin-1-yl)-2,3-diaminopyridine
6-(2-hydroxymethyl-2-hydroxypyrrolidin-1-yl) 2,3-diaminopyridine
6-(2-carboxamidopyrrolidin-1-yl)-2,3-diaminopyridine 6-(2-dimethylcarboxamidopyrrolidin-1-yl)-2,3-diaminopyridine
6-(2-carboxamido-3-hydroxypyrrolidin-1-yl)-2,3-diaminopyridine
6-(2-carboxamido-4-hydroxypyrrolidin-1-yl)-2,3-diaminopyridine
6-(pyrrolidin-1-y)-3-amino-2-(2-hydroxyethylamino) pyridine
6-(3-hydroxypyrrolidin-1-yl)-3-amino-2-(2-hydroxyethylamino)pyridine
6-(3-aminopyrrolidin-1 yl)-3-amino-2-(2-hydroxyethylamino)pyridine
6-(3-acetamidopyrrolidin-1-yl)-3-amino-2-(2-hydroxyethylamino)pyridine
6-(3,4-dihydroxypyrrolidin-1-yl)-3-amino-2-(2-hydroxyethylamino)pyridine
6-(2-carboxypyrrolidin-1-yl)-3-amino-2-(2-hydroxyethylamino)pyridine
6-(2-carboxy-3-hydroxypyrrolidin-1-yl)-3-amino-2-(2-hydroxyethylamino)pyridine
6-(2-carboxy-4-hydroxypyrrolidin-1-yl)-amino-2-(2-hydroxyethylamino)pyridine
6-(2-(hydroxymethyl)pyrrolidin-1-yl)-3-amino-2-(2-hydroxyethylamino)pyridine
6-(2-hydroxymethyl-4-hydroxypyrrolidin-1-yl)-3-amino-2-(2-hydroxyethylamino)pyridine
6-(2-hydroxymethyl-2-hydroxypyrrolidin-1-yl)-3-amino-2-(2-hydroxyethylamino)pyridine
6-(2-carboxamidopyrrolidin-1-yl)-3-amino-2-(2-hydroxyethylamino)pyridine
6-(2-dimethylcarboxamidopyrrolidin-1-yl)-3-amino 2-(2-hydroxyethylamino)pyridine
6-(2-carboxamido-3-hydroxypyrrolidin-1-yl)-3-amino-2-(2-hydroxyethylamino)pyridine
6-(2-carboxamido-4-hydroxypyrrolidin-1-yl)-3-aminopyridine
6-(pyrrolidin-1-yl)-3-amino-2-(2-aminoethylamino)-pyridine
6-(3-hydroxypyrrolidin-1-yl)-3-amino-2-(2-aminoethylamino)pyridine
6-(3-aminopyrrolidin-1-yl)-3-amino-2-(2-aminoethylamino)pyridine
6-(3-acetamidopyrrolidin-1-yl)-3-amino-2-(2-aminoethylamino)pyridine
6-(3,4-dihydroxypyrrolidin-1-yl)-3-amino-2-(2-aminoethylamino)pyridine
6-(2-carboxypyrrolidin-1-yl)-3-amino-2-(2-aminoethylamino)pyridine
6-(2-carboxy-3-hydroxypyrrolidin-1-yl)-3-amino-2-(2-aminoethylamino)pyridine
6-(2-carboxy-4-hydroxypyrrolidin-1-yl)-3-amino-2-(2-aminoethylamino)pyridine
6-(2-(hydroxymethyl)pyrrolidin-1-yl)-3-amino-2-(2-aminoethylamino)pyridine
6-(2-hydroxymethyl-4-hydroxypyrrolidin-1-yl)-3-amino-2-(2-aminoethylamino)pyridine
6-(2-hydroxymethyl-2-hydroxypyrrolidin-1-yl)-3-amino-2-(2-aminoethylamino)pyridine
6-(2-carboxamidopyrrolidin-1-yl)-3-amino-2-(2-aminoethylamino)pyridine
6-(2-dimethylcarboxamidopyrrolidin-1-yl)-3-amino-2-(2-aminoethylamino)pyridine
6-(2-carboxamido-3-hydroxypyrrolidin-1-yl)-3-amino-2-(2-aminoethylamino)pyridine
6-(2-carboxamido-4-hydroxypyrrolidin-1-yl)-3-amino-2-(2-aminoethylamino)pyridine
6-(pyrrolidin-1-yl)-3-amino-2-(2-acetamidoethylamino)-pyridine
6-(3-hydroxypyrrolidin-1-yl)-3-amino-2-(2-acetamidoethylamino)pyridine
6-(3-aminopyrrolidin-1-yl)-3-amino-2-(2-acetamidoethylamino) pyridine
6-(3-acetamidopyrrolidin-1-yl)-3-amino-2-(acetamidoethylamino)pyridine
6-(3,4-dihydroxypyrrolidin-1-yl)-3-amino-2-(2-acetamidoethylamino)pyridine
6-(2-carboxypyrrolidin-1-yl)-3-amino-2-(2-acetamidoethylamino)pyridine
6-(2-carboxy-3-hydroxypyrrolidin-1-yl)-3-amino-2-(2-acetamidoethylamino)pyridine
6-(2-carboxy-4-hydroxypyrrolidin-1-yl)-3-amino-2-(2-acetamidoethylamino)pyridine
6-(2-(hydroxymethyl)pyrrolidin-1-yl)-3-amino-2-(2-acetamidoethylamino)pyridine
6-(3-hydyoxymethyl-4-hydroxypyrrolidin-1-yl)-3-amino-2-(2-acetamidoethylamino)pyridine
5-(2-hydroxymethyl-2-hydroxypyrrolidin-1-yl)-3-amino-2-(2-aceramidoethylamino)-pyridine
6-(2-carboxamidopyrrolidin-1-yl)-3-amino-2-(2-acetamidoethylamino)pyridine
6-(2-dimethylcarboxamidopyrrolidin-1-yl)-3-amino-2-(2-acetamidoethylamino)pyridine
6-(2-carboxamido-3-hydroxypyrrolidin-1-yl)-3-amino-2-(2-acetamidoethylamino)pyridine
6-(2-carboxamido-4-hydroxypyrrolidin-1-yl)-3-amino-2-(2-acetamidoethylamino)pyridine
6-(pyrrolidin-1-yl)-3-amino-2-(pyrrolidin-1-yl)pyridine 6-(3-hydroxypyrrolidin-1-yl)-3-amino-2-(pyrrolidin-1-yl)pyridine
6-(3-aminopyrrolidin-1-yl)-3-amino-2-(pyrrolidin-1-yl)pyridine
6-(3-acetamidopyrrolidin-1-yl)-3-amino-2-(pyrrolidin-1-yl)pyridine
6-(3,4-dihydroxypyrrolidin-1-yl)-3-amino-2-(pyrrolidin-1-yl)pyridine
6-(2-carboxypyrrolidin-1-yl)-3-amino-2-(pyrrolidin-1-yl)pyridine
6-(2-carboxy-3-hydroxypyrrolidin-1-yl)-3-amino-2-(pyrrolidin-1-yl)pyridine
6-(2-carboxy-4-hydroxypyrrolidin-1-yl)-3-amino-2-(pyrrolidin-1-yl)pyridine
6-(2-(hydroxymethyl)pyrrolidin-1-yl)-3-amino-2-(pyrrolidin-1-yl)pyridine
6-(2-hydroxymethyl-4-hydroxypyrrolidin-1-yl)-3-amino-2-(pyrrolidin-1-yl)pyridine
6-(2-hydroxymethyl-2-hydroxypyrrolidin-1-yl)-3-amino-2-(pyrrolidin-1-yl)pyridine
6-(2-carboxamidopyrrolidin-1-yl)-3-amino-2-(pyrrolidin-1-yl)pyridine
6-(2-dimethylcarboxamidopyrrolidin-1-yl)-3-amino-2-(pyrrolidin-1-yl)pyridine
6-(2-carboxamido-3-hydroxypyrrolidin-1-yl)-3-amino-2-(pyrrolidin-1-yl)pyridine
6-(2-carboxamido-4-hydroxypyrrolidin-1-yl)-3-amino-2-(pyrrolidin-1-yl)-pyridine
6-(pyrrolidin-1-yl)-3-amino-2-(3-hydroxypyrrolidin-1-yl)pyridine
6-(3-hydroxypyrrolidin-1-y)-3-amino-2-(3-hydroxypyrrolidin-1-yl)pyridine
6-(3-aminopyrrolidin-1-yl)-3-amino-2-(3-hydroxypyrrolidin-1-yl)pyridine
6-(3-acetamidopyrrolidin-1-yl)-3-amino-2-(3-hydroxypyrrolidin-1-yl)pyridine
6-(3,4-dihydroxypyrrolidin-1-yl)-3-amino-2-(3-hydroxypyrrolidin-1-yl)pyridine
6-(2-carboxypyrrolidin-1-yl)-3-amino-2-(3-hydroxypyrrolidin-1-yl)pyridine
6-(2-carboxy-3-hydroxypyrrolidin-1-yl)-3-amino-2-(3-hydroxypyrrolidin-1-yl)pyridine
6-(2-carboxy-4-hydroxypyrrolidin-1-yl)-3-amino-2-(3-hydroxypyrrolidin-1-yl)pyridine
6-(2-(hydroxymethyl)pyrrolidin-1-yl)-3-amino-2-(3-hydroxypyrrolidin-1-yl)pyridine
6-(2-hydroxymethyl-4-hydroxypyrrolidin-1-yl)-3-amino-2-(3-hydroxypyrrolidin-1-yl)pyridine
6-(2-hydroxymtethyl-2-hydroxypyrrolidin-1-yl)-3-amino-2-(3-hydroxyrrolidin-1-yl)pyridine
6-(2-carboxamidopyrrolidin-1-yl)-3-amino-2-(3-hydroxypyrrolidin-1-yl)pyridine
6-(2-dimethylcarboxamidopyrroldin-1-yl)-3-amino-2-(3-hydroxypyrrolidin-1-yl)pyridine
6-(2-carboxamido-3-hydroxypyrrolidin-1-yl)-3-amino-2-(3-hydroxypyrrolidin-1-yl)pyridine
6-(2-carboxamido-4-hydroxypyrrolidin-1-yl)-3-amino-2-(3-hydroxypyrrolidin-1-yl)pyridine
6-(pyrrolidin-1-yl)-3-amino-2-(2-carboxypyrrolidin-1-yl)pyridine
6-(3-hydroxypyrrolidin-1-yl)-3-amino-2-(2-carboxypyrrolidin-1-yl)pyridine
6-(3-aminopyrrolidin-1-yl)-3-amino-2-(2-carboxypyrrolidin-yl)pyridine
6-(3-acetamidopyrrolidin-1-yl)-3-amino-2-(2-carboxypyrrolidin-1-yl)pyridine
6-(3,4-dihydroxypyrrolidin-1-yl)-3-amino-2-(2-carboxypyrrolidin-1-yl)pyridine
6-(2-carboxypyrrolidin-1-yl)-3-amino-2-(2-carboxypyrrolidin-1-yl)pyridine
6-(2-carboxy-3-hydroxypyrrolidin-1-yl)-3-amino-2-(2-carboxypyrrolidin-1-yl)pyridine
6-(2-carboxy-4-hydroxypyrrolidin-1-yl)-3-amino-2-(2-carboxypyrrolidin-1-yl)pyridine
6-(2-(hydroxymethyl)pyrrolidine-1-yl)-3-amino-2 (2-carboxypyrrolidin-1-yl)pyridine
6-(2-hydroxymethyl-4-hydroxypyrrolidin-1-yl)-3-amino-2-(2-carboxyrrolidin-1-yl)pyridine
6-(2-hydroxymethyl-2-hydroxypyrrolidin-1-yl)-3-amino-2-(2-carboxypyrrolidin-1-yl)pyridine
6-(2-carboxamidopyrrolidin-1-yl)-3-amino-2-(2-carboxypyrrolidin-1-yl)pyridine
6-(2-dimethylcarboxamidopyrrolidin-1-yl)-3-amino-3-(2-carboxypyrrolidin-1-yl)pyridine
6-(2-carboxamido-3-hydroxypyrrolidin-1-yl)-3-amino-2-(2-carboxypyrrolidin-1-yl)pyridine
6-(2-carboxamido-4-hydroxypyrrolidin-1-yl)-3-amino-2-(2-carboxypyrrolidin-1-yl)pyridine
6-(pyrrolidin-1-yl)-3-amino-2-(2-(hydroxymethyl)-pyrrolidin-1yl)pyridine
6-(3-hydroxypyrrolidin-1-yl)-3-amino-2-(2-(hydroxymethyl)pyrrolidin-1-yl)pyridine
6-(3-aminopyrrolidin-1-yl)-3-amino-2-(2-(hydroxymethyl)pyrrolidin-1-yl)pyridine
6-(3-acetamidopyrrolidin-1-yl)-3-amino-2-(2-hydroxymethyl)pyrrolidin-1-yl)pyridine
6-(3,4-dihydroxypyrrolidin-1-yl)-3-amino-2-(2-(hydroxymethyl)pyrrolidin-1-yl)pyridine
6-(2-carboxypyrrolidin-1-yl)-3-amino-2-(2-(hydroxymethyl)pyrrolidin-1-yl)pyridine
6-(2-carboxy-3-hydroxypyrrolidin-1-yl)-3-amino-2-(2-(hydroxymethyl)pyrrolidin-1-yl)pyridine
6-(2-carboxy-4-hydroxypyrrolidin-1-yl)-3-amino-2-(2-(hydroxymethyl)pyrrolidin-1-yl)pyridine
6-(2-(hydroxymethyl)pyrrolidin-1-yl)-3-amino-2-(2-(hydroxymethyl)pyrrolidin-1-yl)pyridine
6-(2-hydroxymethyl-4-hydroxypyrrolidin-1-yl) 3-amino-2-(2-(hydroxymethyl)pyrrolidin-1-yl)pyridine
6-(2-hydroxymethyl-2-hydroxypyrrolidin-1-yl)-3-amino-2-(2-(hydroxymethyl)pyrrolidin-1-yl)pyridine
6-(2-carboxamidopyrrolidin-1-yl)-3-amino-2-(2-(hydroxymethyl)pyrrolidin-1-yl)pyridine
6-(3-dimethylcarboxamidopyrrolidin-1-yl)-3-amino-2-(2-(hydroxymethythyl)pyrrolidin-1-yl)pyridine
6-(2-carboxamido-3-hydroxypyrrolidin-1-yl)-3-amino-2-(2-(hydroxymethyl)pyrrolidin-1-yl)pyridine
6-(2-carboxamido-4-hydroxypyrrolidin-1-yl)-3-amino-2-(2-(hydroxymethyl)pyrrolidin-1-yl)pyridine
6-(pyrrolidin-1-yl)-3-amino-2-(2-carboxamidopyrrolidin-1-yl)pyridine
6-(3-hydroxypyrrolidin-1-yl)-3-amino-2-(2-carboxamidopyrrolidin-1-yl)pyridine
6-(3-aminopyrrolidin-1-yl)-3-amino-2-(2-carboxamidopyrrolidin-1-yl)pyridine
6-(3-acetamidopyrrolidin-1-yl)-3-amino-2-(2-carboxamidopyrrolidin-1-yl)pyridine
6-(3,4-dihydroxypyrrolidin-1-yl)-3-amino-2-(carboxamidopyrrolidin-1-yl)pyridine
6-(2-carboxypyrrolidin-1-yl)-3-amino-2-(2-carboxamidopyrrolidin-1-yl)pyridine
6-(2-carboxy-3-hydroxypyrrolidin-1-yl)-3-amino-2(2-carboxamidopyrrolidin-1-yl)pyridine
6-(2-carboxy-4-hydroxypyrrolidin-1-yl)-3-amino-2-(2-carboxamidopyrrolidin-1-yl)pyridine
6-(2-(hydroxymethyl)pyrrolidin-1-yl)-3-amino-2-(2-carboxamidopyrrolidin-1-yl)pyridine
6-(2-hydroxymethyl-4-hydroxypyrrolidin-1-yl)-3-amino-2-(2-carboxamidopyrrolidin-1-yl)pyridine
6-(2-hydroxymethyl-2-hydroxypyrrolidin-1-yl)-3-amino-2-(2-carboxamidopyrrolidin-1-yl)pyridine
6-(2-carboxamidopyrrolidin-1-yl)-3-amino-2-(2-carboxamidopyrrolidin-1-yl)pyridine
6-(2-dimethylcarboxamidopyrrolidin-1-yl)-3-amino-2-(2-carboxamidopyrrolidin-1-yl)pyridine
6-(2-carboxamido-3-hydroxypyrrolidin-1-yl)-3-amino-2-(2-carboxamidopyrrolidin-1-yl)pyridine
6-(2-carboxamido-4-hydroxypyrrolidin-1-yl)-3-amino-2-(2-carboxamidopyrrolidin-1-yl)pyridine
-(pyrrolidin-1-yl)-3-amino-2-methyl(2-hydroxyethyl)-amino]pyridine
6-(3-hydroxypyrrolidin-1-yl)-3-amino-2-[methyl(2-hydroxyethyl)aminon]pyridine
6-(3-aminopyrrolidin-1-yl)-3-amino-2-[methyl(2-hydroxyethyl)amino]pyridine
6-(3-acetamidopyrrolidin-1-yl)-3-amino-2-[methyl(2-hydroxyethyl)amino]pyridine
6-(3,4-dihydroxypyrrol_din-1-yl)-3-amino-2-[methyl(2-hydroxyethyl)amino]pyridine
6-(2-carboxypyrrolidin-1-yl)-3-amino-2-[methyl(2-hydroxyethyl)amino]pyridine
6-(2-carboxy-3-hydroxypyrrolidin-1-yl)-3-amno-2-[methyl(2-hydroxyethyl)amino]pyridine
6-(2-carboxy-4-hydroxypyrrolidin-1-yl)-3-amino-2-[methyl(2-hydroxyethyl)amino]pyridine
6-(2-hydroxymethyl)pyrrolidin-1-yl)-3-amino-2-[methyl(2-hydroxyethyl)amino]pyridine
6-(2-hydroxymethyl-4-hydroxypyrrolidin-1-yl)-3-amino-2-[methyl(2-hydroxyethyl)amino]pyridine
6-(2-hydroxymethyl-2-hydroxypyrrolidin-1-yl)-3-amino-2-[methyl(2-hydroxyethyl)amino]pyridine
6-(2-carboxamidopyrrolidin-1-yl)-3-amino-2-[methyl (2-hydroxyethyl)amino]pyridine
6-(2-dimethylcarboxamidopyrrolidin-1-yl)-3-amino-2-[methyl(2-hydroxyethyl)amino]pyridine
6-(2-carboxamido-3-hydroxypyrrolidin-1-yl)-3-amino-2-[methyl(2-hydroxyethyl)amino]pyridine
6-(2-carboxamido-4-hydroxypyrrolidin-1-yl)-3-amino-2-[methyl(2-hydroxyethyl)amino]pyridine
6-(pyrrolidin-1-yl)-3-amino-2-[methyl(2-aminoethyl)-amino]pyridine
6-(3-hydroxypyrrolidin-1-yl)-3-amino-2-[methyl(2-aminoethyl)amino]pyridine
6-(3-aminopyrrolidin-1-yl)-3-amino-2-[methyl(2-aminoethyl)amino]pyridine
6-(3-acetamidopyrrolidin-1-yl)-3-amino-2-[methyl(2-aminoethyl)amino]pyridine
6-(3,4-dihydroxypyrrolidin-1-yl)-3-amino-2-[methyl(2-aminonethyl)amino]pyridine
6-(2-carboxypyrolidin-1-yl)-3-amino-2-[methyl(2-aminoethyl)amino]pyridine
6-(2-carboxy-3-hydroxypyrrolidin-1-yl)-3-amino-2-[methyl(2-aminoethyl)amino]pyridine
6-(2-carboxy-4-hydroxypyrrolidin-1-yl)-3-amino-2-[methyl(2-aminoethyl)amino]pyridine
6-(2-(hydroxymethyl)pyrrolidin-1-yl)-3-amino-2-[methyl(2-aminoethyl)amino]pyridine
6-(2-hydroxymethyl-4-hydroxypyrrolidin-1-yl)-amino-2-[methyl(2-aminoethyl)amino]pyridine
6-(2-hydroxymethyl-2-hydroxyrrolidin-yl)-3-amino-2-[methyl(2-aminoethyl)amino]pyridine
6-(2-carboxamidopyrrolidin-1-yl)-3-amino-2-[methyl (2-aminoethyl)amino]pyridine
6-(2-dimethylcarboxamidopyrrolidin-1-yl)-3-amino-2-[methyl(2-aminoethyl)amino]pyridine
6-(2-carboxamido-3-hydroxypyrrolidin-1-yl)-3-amino-2-[methyl(2-aminoethyl)amino]amino]pyridine
6-(2-carboxamido-4-hydroxypyrrolidin-1-yl)-3-amino-2-[methyl(2-aminoethyl)amino]pyridine
6-(pyrrolidin-1-yl)-3-amino-2- [methyl(2-acetamidoethyl)amino]pyridine
6-(3-hydroxypyrrolidin-1-yl)-3-amino-2-[methyl(2-acetamidoethyl)amino]pyridine
6-(3-aminopyrrolidin-1-yl)-3-amino-2-[methyl(2-acetamidoethyl)amino]pyridine
6-(3-acetamidopyrrolidin-1-yl)-3-amino-2-[methyl(2-acetamidoethyl)amino]pyridine
6-(3,4-dihydroxypyrrolidin-1-yl)-3-amino-2- [methyl (2-acetamidoetyl)amino]pyrrodine
6-(2-carboxypyrrolidin-1-yl)-3-amino-2-[methyl(2-acetamidoethyl)amino]pyridine
6-(2-carboxy-3-hydroxypyrrolidin-1-yl)-3-amino-2-[methyl(2-acetamidoethyl)amino]pyridine
6-(2-carboxy-4-hydroxypyrrolidin-1-yl)-3-amino-2-[methyl(2-acetamidoethyl)amino]pyridine
6-(2-(hydroxymethyl)pyrrolidin-1-yl)-3-amino-2-[methyl(2-acetamidoethyl)amino]pyridine
6-(2-hydroxymethyl-4-hydryoxypyrrolidin-1-yl)-3-amino-2-[methyl(2-acetamidoethyl)amino]pyridine
6-(2-hydroxymethyl-2-hydroxypyrrolidin-1-yl)-3-amino-2-[methyl(2-acetamidoethyl)amino]pyridine
6-(2-carboxamidopyrrolidin-1-yl)-3-amino-2-[methyl (2-acetamidoethyl)amino]pyridine
6-(2-dimethylcarboxamidopyrrolidin-1-yl)-3-amino-2-[methyl(2-acetamidoethyl)amino]pyridine
6-(2-carboxamido-3-hydroxypyrrolidin-1-yl)-3-amino-2-[methyl(2-acetamidoethyl)amino] pyridine
6-(2-carboxamido-4-hydroxypyrrolidin-1-yl)-3-amino-2-[methyl(2-acetamidoethyl)amino]pyridine
6-(pyrrolidin-1-yl)-3-amino-2-methoxypyridine
6-(3-hydroxypyrrolidin-1-yl)-3-amino-2-methoxypyridine
6-(3-aminopyrrolidin-1-yl)-3-amino-2-methoxypyridine
6-(3-acetamidopyrrolidin-1-yl)-3-amino-2-methoxypyridine
6-(3,4-dihydroxypyrrolidin-1-yl)-3-amino-2-methoxypyridine
6-(2-carboxypyrrolidin-1-yl)-3-amino-2-methoxypyridine
6-(2-carboxy-3-hydroxypyrrolidin-1-yl)-3-amino-2-methoxypyridine
6-(2-carboxy-4-hydroxypyrrolidin-1-yl)-3-amino-2-methoxypyridine
6-(2-(hydroxymethyl)pyrrolidin-1-yl)-3-amino-2-methoxypyridine
6-(2-hydroxymechyl)-4-hydroxypyrrolidin-1-yl)-3-amino-2-methoxypyridine
6-(2-hydroxymethyl-2-hydroxypyrrolidin-1-yl)-3-amino-2-methoxypyridine
6-(2-carboxamidopyrrolidin-1-yl)-3-amino-2-methoxypyridine
6-(2-dimethylcarboxamidopyrrolidin-1-yl)-3-amino-2-methoxypyridine
6-(2-carboxamido-3-hydroxypyrrolidin-1-yl)-3-amino-2-methoxypyridine
6-(2-carboxamido-4-hydroxypyrrolidin-1-yl)-3-amino-2-methoxypyridine
6-(pyroolidin-1-yl)-3-amino-2-(2-hydroxyethyloxy)-pyridine
6-{3-hydroxypyrrolidin 1-yl)-3-amino-2-(2-hydroxyethyloxy}pyridine
6-(3-aminopyrrolidin-1-yl)-3-amino-2-(2-hydroxyethyloxy)pyridine
6-(3-acetamidopyrrolidin-1-yl)-3-amino-2-(2-hydroxyethyloxy)pyridine
6-(3,4-dihydroxypyrrolidin-1-yl)-3-amino-2-(2-hydroxyethyloxy)pyridine
6-(2-carboxypyrrolidin-1-yl)-3-amino-2-(2-hydroxyethyloxy)pyridine
6-(2-carboxy-3-hydroxypyrrolidin-1-yl)-3-amino 2 (2-hydroxyethyloxy)pyridine
6-(2-carboxy-4-hydroxypyrrolidin-1-yl)-3-amino-2-(2-hydroxyethyloxy)pyridine
6-(2-(hydroxymethyl)pyrrolidin-1-yl)-3-amino-2-(2-hydroxyethyloxy)pyridine
6-(2-hydroxymethyl-4-hydroxypyrrolidin-1-yl)-3-amino-2-(2-hydroxyethyloxy)pyridine
6-(2-hydroxymethyl-2-hydroxypyrrolidin-1-yl)-3-amino-2-(2-hydroxyethyloxy)pyridine
6-(2-carboxamidopyrrolidin-1-yl)-3-amino-2-(2-hydroxyethyloxy)pyridine
6-(2-dimethylcarboxamidopyrrolidin-1-yl)-3-amino-2-(2-hydroxyethyloxy)pyridine
6-(2-carboxyethyloxamido-3-hydroxypyrrolidin-1-yl)-1-amino-2-(2-hydroxyethyloxy)pyridine
6-(2-carboxamido-4-hydroxypyrrolidin-1-yl)-3-amino-2-(2-hydroxyethyloxy)pyridine
6-(pyrrolidin-1-yl)-3-amino-2-ethoxypyridine
6-(3-hydroxypyrrolidin-1-yl)-3-amino-2-ethoxypyridine
6-(3-aminopyrrolidin-1-yl)-3-amino-2-ethoxypyridine
6-(3-acetamidopyrrolidin-1-yl)-3-amino-2-ethoxypyridine
6-(3,4-dihydroxypyrrolidin-1-yl)-3-amino-2-ethoxypyridine
6-(2-carboxypyrrolidin-1-yl)-3-amino-2-ethoxypyridine
6-(2-carboxy-3-hydroxypyrrolidin-1-yl)-3-amino-2-ethoxypyridine
6-(2-carboxy-4-hydroxypyrrolidin-1-yl)-3-amino-2-ethoxypyridine
6-(2-(hydroxethyl)pyrrolidin-1-yl)-3-amino-2-ethoxypyridine
6-(2-hydroxymethyl-4-hydroxypyrrolidin-1-yl)-3-amino-2-ethoxypyridine
6-(2-hydroxymethyl-2-hydroxypyrrolidin-1-yl)-3-amino-2-ethoxypyridine
6-(2 carboxamidopyrrolidin-1-yl)-3-amino-2-ethoxypyridine
6-(2-dimethylcarboxamidoxyrrolidin-1-yl)-3-amino-2-ethoxypyridine
6-(2-carboxamido-3-hydroxypyrrolidin-1-yl)-3-amino-2-ethoxypyridine
6-(2-carboxamido-4-hydroxypyrrolidin-1-yl)-3-amino-2-ethoxypyridine and their addition salts.

## Patentansprüche

1. Färbezusammensetzung, die in einem geeigneten Färbemedium als Oxidationsbase mindestens ein 2,5-Diaminopyridinderivat der Formel (I) oder die entsprechenden Additionssalze umfaßt : 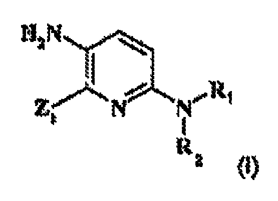 worin
Z₁ für einen OR₃- oder NR₃R₅-Rest steht;
R₁, R₂, R₄ und R₅ unabhängig voneinander für ein Wasserstoffatom, einen C₁-C₆-Alkylyest, einen C₂-C₆-(Poly)aminoalkylrest, einen C₂-C₆-(Poly)-alkylaminoalkylrest, einen C₂-C₆-Aminohydroxyalkylrest, einen C₂-C₆-Acylaminoalkylrest, einen C₂-C₆-Alkoxyalkylrest, einen C₂-C₆-Hydroyalkoxyalkylrest, einen C₂-C₆-Aminoalkoxyalkylrest, einen C₂-C₆-(Poly)hydroxyalkylrest, einen C₂-C₆-Carboxyalkylrest oder einen C₂-C₆-Sulfalkylrest stehen;
R₁ und R₂ einerseits und R₁ und R₃ andererseits unabhängig voneinander mit dem Stickstoffatom, an das sie gebunden sind, einen gesättigten Heterocyclus mit 5 bis 8 Gliedern, der gegebenenfalls durch einen oder mehrere Reste aus der Gruppe bestehend aus Halogenatomen, Amino-, (Di)alkylamino-, Hydroxy, Carboxy-, Carboxamido-, Alkylsulfonylamino-, Acetamido- und Alkoxyresten und C₁-C₆-Alkylresten, die gegebenenfalls durch einen oder mehrere Hydroxy-, Amino-, (Di)alkylamino-, Alkoxy-, Hydroxyalkoxy-, Aminoalkoxy-, Carboxy-, Carboxamido-, Sulfonamido- oder Sulforeste substituiert sind, substituiert ist, bilden, wobei der Heterocyclus ein oder mehrere zusätzliche Heteroatome, die unter Sauerstoff, gegebenenfalls substituiertem Stickstoff, Schwefel oder einer SO₂-Gruppe ausgewählt sind, enthalten kann, wobei der Heterocyclus weder eine Peroxidbindung noch Diazo- oder Nitroreste enthält;
R₃ für einen linearen oder verzweigten C₁-C₆-Alkylrest, gegebenenfalls substituiert durch einen oder mehrere Reste, ausgewählt unter Resten des Typs Hydroxy, Amino, Mono- oder Di-C₁-C₃-alkylanino, deren Alkylteil gegebenenfalls substituiert ist durch eine oder mehrere Gruppen des Typs Hydroxy, Amino, C₁-C₂-Alkoxyalkoxy, Carboxy, steht; mit Ausrahme von 2,3,6-Triaminopyridin und Additionssalzen davon.

2. Zusammensetzung nach Anspruch 1, in der die Oxidationsbase der Formel (I) so beschaffen ist, daß R₁ für einen Methyl-, Ethyl-, Hydroxyethyl-, carboxymethyl- oder Carboxyethylrest steht.

3. Zusammensetzung nach Anspruch 1 oder 2, in der die Oxidationsbase der Formel (I) so beschaffen ist, daß R₃ für einen Methylrest steht.

4. Zusammensetzung nach einem der Anspruche 1 bis 3, in der die Oxidationsbase der Formel (I) so beschaften ist, daß R₁, R₂, R₄, und R₅ unabhängig voneinander für einen C₁-C₄-(Di)alkylaminoalkylrest, einen C₂-C₄-Carboxyalkylrest, einen C₂-C₄-Sulfalkylrest, einen C₁-C₄-(Poly)hydroxyalkylrest oder einen C₂-C₄-(Poly)aminoalkylrest stehen.

5. Zusammensetzung nach einem der Ansprüche1 bis 3, in der die Oxidationsbase der Formel (I) so beschaffen ist, daß R₁ und R₂ einerseits und R₃ und R_{b} andererseits unabhängig voneinander mit dem Stickstoffatom, an das sie gebunden sind, einen Heterocyclus mit 5 bis 8 Gliedern, der unter Pyrrolidin-, Piperidin-, Homopiperidin-, Piperazin-, Homopiperazin- und Diazepan-Heterocyclen ausgewählt ist, bilden, wobei die Ringe durch einen oder mehrere C₁-C₂-Alkylreste, die gegebenenfalls durch ein oder mehrere Hydroxy, Amino, (Di)-C₁-C₂-alkylamino, Carboxy, Carboxamido oder Sulfonamido substituiert sind, substituiert sein könnten.

6. Zusammensetzung nach Anspruch 5, in der die Oxidationsbase der Formel (I) so beschaffen ist, daß R₁ und R₂ einerseits und R₄ und R₅ andererseits unabhängig voneinander mit dem Stickstoffatom, an das sie gebunden sind, einen Heterocyclus bilden, der unter 2,5-Dimethylpyrrolidin, Pyrrolidin-2-carbonsäure, 3-Hydroxypyrrolidin-2-carbonäure, 4-Hydroxypyrrolidin-2-carbonsäure, 2,4-Dicarboxypyrrolidin, 3-Hydroxy-2-hydroxymethylpyrrolidin, 2-Carboxamidopyrrolidin, 3-Hydroxy-2-carboxamidopyrrolidin, 2-(Diethylcarboxamido)pyrrolidin, 2-(Hydroxymethyl)pyrrolidin, 3,4-Dihydroxy-2-(hydroxymethyl)pyrrolidin, 3-Hydroxypyrrolidin, 3,4-Dihydroxypyrrolidin, 3-Aminopyrrolidin, 3-(Methylamino)pyrrolidin, 4-Amino-3-hydroxypyrrolidin, 3-Hydroxy-4-[(2-hydroxyethyl)amino]-pyrrolidin, 2,6-Dimethylpiperidin, 2-carhoxypiperidin, 2-Carboxamidopiperidin, 2-Hydroxymethylpiperidin, 3-Hydroxy-2-hydroxymethylpiperidin, 3-Hydroxypiperidin, 4-Hydroxypiperidin, 3-Hydroxymethylpiperidin, Homopiperidin, 2-Carboxyhomopiperidin, 2-Carboxamidchomopiperidin, Diazepan, N-Methyldiazepan und N-(2-Hydroxyethyl) - diazepan ausgewählt ist.

7. Zusammensetzung nach Anspruch 6, in der die Oxidationsbase der Formel (I) so beschaffen ist, daß R₁ und R₂ einerseits und R₄ und R₅ andererseits unabhängig voneinander einen Heterocyclus bilden, der unter Pyrrolidin, 3-Hydroxypyrrolidin, 3-Aminopyrrolidin, 3-Acetamidopyrrolidin, 3-(Methylsulfonylamino)pyrrolidin, Pyrrolidin-2-carbonsäure, 3-Hydroxypyrrolidin-2-carbonsäure, Piperidin, Hydroxypiperidin, Homopiperidin, Diazepan, N-Methylhomopiperazin oder N-(β-Hydroxyethyl)homopiperazin ausgewählt ist.

8. Zusammensetzung nach einem der Ansprüche 1 bis 7, in der die Oxidationsbase der Formel (I) ausgewählt ist unter:
2-Methoxy-6-(pyrrolidin-1-yl)-3-aminopyridin
1-(5-Amino-6-methoxypyridin-2-yl)pyrrolidin-3-ol
3-Amino-2-methoxy-6-(morpholin-4-yl)pyridin,
3-Amino-2-methoxy-6-(piperidin-1-yl)pyridin,
6-N,N-(2-hydroxyethyl)methyl)amino)-3-amino-2-methoxypyridin
5,6-Diamino-2-(pyrrolidin-1-yl)pyridin
N-(5,6-Diaminopyridin-2-yl)-3-hydroxypyrrolidin
2,6-Di(pyrrolidin-1-yl)-5-aminopyridin
6-(Pyrrolidin-1 yl) 2,3-diaminopyridin
6-(3-Hydroxypyrrolidin-1-yl)-2,3-diaminopyridin
6-(3-Aminopyrrolidin-1-yl)-2,3-diaminopyridin
6-(3-Acetamidopyrrolidin-1-yl)-2,3-diaminopyridin
6-(3,4-Dihydroxypyrrolidin-1-yl)-2,3-diaminopyridin
6-(2-Carboxypyrrolidin-1-yl)-2,3-diaminopyridin
6-(2-Carboxy-3-hydroxypyrrolidin-1-yl)-2,3-diaminopyridin
6-(2-Carboxy-4-hydroxypyrrolidin-1-yl)-2,3-diaminopyridin
6-(2-(Hydroxymethyl)pyrrolidin-1-yl)-2,3-diaminopyridin
6-(2-Hydroxymethyl-4-hydroxypyrrolidin-1-yl)-2 ,3-diaminopyridin
6-(2-Hydroxymethyl-2-hydroxypyrrolidin-1-yl)-2,3-diaminopyridin
6-(2-Carboxamidopyrrolidin-1-yl)-2,3-diaminopyridin
6-(2-Dimethylcarboxamidopyrrolidin-1-yl)-2,3-diaminopyridin
6-(2-Carboxamido-3-hydroxypyrrolidin-1-yl)-2,3-diaminopyridin
6-(2-Carboxamido-4-hydroxypyrridin-1-yl)-2,3-di aminopyridin
6-(Pyrrolidin-1-yl)-3-amino-2-methylaminopyridin
6-(3-Hydroxypyrrolidin-1-yl)-3-amino-2-methylaminopyridin
6-(3-Aminopyrrolidin-1-yl)-3-amino-2-methylaminopyridin
6-(3-Acetamidopyrrolidin-1-yl)-3-amino-3-methylaminopyridin
6-(3,4-Dihydroxypyrrolidin-1-yl)-3-amino-2-methylaminopyridin
6-(2-Carboxypyrrolidin-1-y)-3-amino-2-methylaminopyridin
6-(2-Carboxy-3-hydroxypyrrolidin-1-yl)-3-amino-2-methylaminopyridin
6-(2-Carboxy-4-hydroxypyrrolidin-1-yl)-3-amino-2-methylaminopyridin
6-(2-(Hydroxymethyl)pyrrolidin-1-yl)-3-amino-2-methylaminopyridin
6-(2-Hydroxymethyl-4-hydroxypyrrolidin-1-yl)-3-amino-2-methylaminopyridin
6-(2-Hydroxymethyl-2-hydroxypyrrolidin-1-yl)-3-amino-2-methylaminopyridin
6-(2-Carboxamidopyrrolidin-1-yl)-3-amino-2-methylaminopyridin
6-(2-Dimethylcarboxamidopyrrolidin-1-yl)-3-amino-2-methylaminopyridin
6-(2-carboxamido-3-hydroxypyrrolidin-1-yl)-3-amino-2-methylaminopyridin
6-(2-Carboxamido-4-hydroxypyrrolidin-1-yl)-3-amino-2-methylaminopyridin
6-(Pyrrolidin-1-yl)-3-amino-2-ethylaminoidin
6-(3-Hydroxypyrrolidin-1-yl)-3-amino-2-ethylaminopyridine
6-(3-Aminopyrrolidin-1-yl)-3-amino-2-ethylaminopyridin
6-(3-Acetamidopyrrolidin-1-yl)-3-amino-2-ethylaminopyridin
6-(3,4-Dihydroxypyrrolidin-1-yl)-3-amino-2-ethylaminopyridin
6-(2-Carboxypyrrolidin-1-yl)-3-amino-2-ethylaminopyridin
6-(2-Carboxy-3-hydroxypyrrolidin-1-yl)-3-amino-2-ethylaminopyridin
6-(2-Carboxy-4-hydroxypyrrolidin-1-3-amino-2-ethylaminopyridin
6-(2-(Hydroxymethyl)pyrrolidin-1-yl)-3-amino-2-ethylaminopyridin
6-(2-Hydroxymethyl-4-hydroxypyrrolidin-2-yl)-3-amino-2-ethylaminopyridin
6-(2-Hydroxymethyl-2-hydroxypyrolidin-1-yl)-3-amino-2-ethylaminpyridin
6-(2-Carboxamidopyrrolidin-1-yl)-3-amino-2-ethylaminopyridin
6-(2-Dimethylcarboxamidopyrrolidin-1-yl)-3-amino-2-ethylaminopyridin
6-(2-Carboxamido-3-hydyoxypyrrolidin-1-yl)-3-amino-2-ethylaminopyridin
6-(2-Carboxamido-4-hydroxypyrrolidin-1-yl)-3-amino-2-ethylaminopyridin
6-(Pyrrolidin-1-yl)-3-amino-2-(2-hydroxyethylamino)pyridin
6-(3-Hydroxypyrrolidin-1-yl)-3-amino-2-(2-hydroxyethylamino)pyridin
6-(3-Aminopyrrolidin-1-yl)-3-amino-2-(2-hydxoxyethylamino)pyridin
6-(3-Acetamidopyrrolidin-1-yl)-3-amino-2-(2 hydroxyethylamino)pyridin
6-(3,4-Dihydroxypyrrolidin-1-yl)-3-amino-2-(2-hydroxyethylamino)pyridin
6-(2-Carboxypyrrolidin-1 yl)-3-amino-2-(2-hydroxyethylamino)pyridin
6-(2-Carboxy-3-hydroxypyrrolidin-1-yl) -3-amino-2-(2-hydroxyethylamino)pyridin
6-(2-Carboxy-4-hydroxypyrrolidin-1-yl)-3-amino-2-(2-hydroxyethylamino)pyridin
6-(2-(Hydroxymethyl)pyrrolidin-1-yl)-3-amino-2-(2-hydroxyethylamino)pyridin
6-(2-Hydroxymethyl-4-hydroxypyrrolidin-1-yl)-3-amino-2-(2-hydroxyethylamino)pyridin
6-(2-Hydroxymethyl-2-hydroxypyrridin-1-yl)-3-amino-2-(2-hydroxyethylamino)pyridin
6-(2-Carboxamidopyrrolidin-1-yl)-3-amino-2-(2-hydroxyethylamino)pyridin.
6-(2-Dimethylcarboxamidopyrrolidin-1-yl)-3-amino-2-(2-hydroxyethylamino)pyridin
6-(2-Carboxamido-3-hydroxypyrrolidin-1-yl)-3-amino-2-(2-hydroxyethylamino)pyridin
6-(2-Carboxamido-4-hydroxypyrrolidin-1-yl)-3-aminopyridin
6-(Pyrrolidin-1-yl)-3-amino-2-(2-aminoethylamino)-pyridin
6-(3-Hydroxypyrrolidin-1-yl)-3-amino-2-(2-aminoethylamino)pyridin
6-(3-Aminopyrrolidin-1-yl)-3-amino-2-(2-aminoethylamino)pyridin
6-(3-Acetamidopyrrolidin-1-yl)-3-amino-2-(2-aminoethylamino)pyridin
6-(3,4-Dihydroxypyrrolidin-1-yl)-3-amino-2-(2-aminoethylamino)pyridin
6-(2-Carboxypyrrolidin-1-yl)-3-amino-2-(2-aminoethylamino)pyridin
6-(2-Carboxy-3-hydroxypyrrolidin-1-yl)-3-amino-2-(2-aminoethylamino)pyridin
6-(2-Carboxy-4-hydroxypyrrolidin-1-yl)-3-amino-2-(2-aminoethylamino)pyridin
6-(2-(Hydroxymethyl)pyrrolidin-1-yl)-3-amino-2-(2-aminoethylamino)pyridin
6-(2-Hydroxymethyl-4-hydroxypyrrolidin-1-yl)-3-amino-2-(2-aminoethylamino)pyridin
6-(2-Hydroxymethyl-2-hydroxypyrrolidin-1-yl)-3-amino-2-(2-aminoethylamino)pyridin
6-(2-Carboxamidopyrrolidin-1-yl)-3-amino-2-(2-aminoethylamino)pyridin
6-(2-Dimethylcarboxamidopyrrolidin-1-yl)-3-amino-2-(2-aminoethylamino)pyridin
6-(2-Carboxamido-3-hydroxypyrrolidin-1-yl)-3-amino-2-(2-aminoethylamino)pyridin
6-(2-Carboxamido-4-hydroxamid-4-hydroxypyrrolidin-1-yl)-3-amino-2-(2-aminoethylamino)pyridin
6-(Pyrrolidin-1-yl)-3-amino-2-(2-acetamidoethylamino)pyridin
6-{3-Hydroxypyrrolidin-1-yl}-3-amino-2-(2-acetamidoethylamino)pyridin
6-(3-Aminopyrrolidin-1-yl)-3-amino-2-(2-acetamidoethylamino)pyridin
6-(3-Acetamidopyrrolidin-1-yl)-3-amino-2-(2-acetamidoethylamino)pyridin
6-(3,4-Dihydroxypyrrolidin-1-yl)-3-amino-2-(2-acetamidoethylamino)pyridin
6-(2-Carboxypyrrolidin-1-yl)-3-amino-2-(2-acetamidoethylamino)pyridin
6-(2-Carboxy-3-hydroxypyrrolidin-1-yl)-3-amino-2-(2-acetamidoethylamino)pyridin
6-(2-Carboxy-4-hydroxypyrrolidin-1-yl)-3-amino-2-(2-acetamidoethylamino)pyridin
6-(2-(Hydroxymethyl)pyrrolidin-1-yl)-3-amino-2-(2-acetamidoethylamino)pyridin
6-(2-Hydroxymethyl-4-hydroxypyrrolidin-1-yl)-3-amino-2-(2-acetamidoethylamino)pyridin
6-(2-Hydroxymethyl-2-hydroxypyrrolidin-1-yl)-3-amino-2-(2-acetamidoethylamino)pyridin
6-(2-Carboxamidopyrrolidin-1-yl)-3-amino-2-acetamidoethylamino)pyridin
6-(2-Dimethylcarboxamidopyrrolidin-1-yl)-3-amino-2-(2-acetamidoethylamino)pyridin
5-(2-Carboxamido-3-hydroxypyrrolidin-1-yl)-3-amino-2-(2-acetamidoethylamino)pyridin
6-(2-Carboxamido-4-hydroxypyrrolidin-1-yl)-3-amino-2-(2-acetamidoethylamino)pyridin
6-(Pyrrolidin-1-yl)-3-amino-2-(pyrrolidin-1-yl)-pyridin
6 (3-Hydroxypyrrolidin-1-yl)-3-amino-2-(pyrrolidin-1-yl)pyridin
6-(2-Aminopyrrolidin-1-yl)-3-amino-2-(pyrrolidin-1-yl)pyridin
6-(3-Acetamidopyrrolidin-1-yl)-3-amino-2-(pyrrolidin-1-yl)pyridin
6-(3,4-Dihydroxypyrrolidin-1-yl)-3-amino-2-(pyrrolidin-1-yl)pyridin
6-(2-Carboxypyrrolidin-1-yl)-3-amino-2-(pyrrolidin-1-yl)pyridin
6-(2-Carboxy-3-hydroxypyrrolidin-1-yl)-3-amino-1-(pyrrolidin-1-yl)pyridin
6-(2-Carboxy-4-hydroxypyrolidin-1-yl) -3-amino-2-(pyrrolidin-1-yl)pyridin
6-(2 (Hydroxymethyl)pyrrolidin-1-yl)-3-amino-2-(pyrrolidin-1-yl)pyridin
6-(2-Hydroxymethyl-1-hydroxypyrrolidin-1-yl)-3-amino-2-(pyrrolidin-1-yl)pyridin
6-(2-Hydroxymethyl-2-hydroxypyrrolid-1-yl)-3-amino-2-(pyrrolidin-1-yl)pyridin
6-(2-Carboxamidopyrrolidin-1-yl)-3-amino-2-(pyrrolidin-1-yl)pyridin
6-(2-Dimethylcarboxamidopyrrolidin-1-yl)-3-amino-2-(pyrrolidin-1-yl)pyridin
6-(2-Carboxamido-3-hydroxypyrrolidin-1-yl)-3-amino-2-(pyrrolidin-1-yl)pyridin
6-(2-Carboxamido-4-hydropypyrrolidin-1-yl)-3-amino-2-(pyrrolidin-1-yl)pyridin
6-(Pyrrolidin-1-yl)-3-amino-2-(3-hydroxypyrrolidin-1-yl)pyridin
6-(3-Hydroxypyrrolidin-1-yl) 3-amino-2-(3-hydroxypyrrolidin-1-yl)pyridin
6-(3-Aminopyrrolidin-1-yl)-3-amino-2-(3 hydroxypyrrolidin-1-yl)pyridin
6-(3-Acetamidopyrrolidin-1-yl)-3-amino-2-(3-hydroxypyrrolidin-1-yl)pyridin
6-(3,4-Dihydroxypyrrolidin-1-yl)-3-amino-2-(3-hydroxypyrrolidin-1-yl)pyridin
6-(2-Carboxypyrrolidin-1-yl)-3-amino-2-(3-hydroxypyrrolidin-1-yl)pyridin
6-(2-Carboxy-3-hydroxypyrrolidin-1-yl)-3-amino-2-(3-hydroxypyrrolidin-1-yl)pyridin
6-(2-Carboxy-4-hydroxypyrrolidin-1-yl)-3-amino-2-(3-hydroxypyrolidin-1-yl)pyridin
6-(2-(Hydroxymethyl)pyrrolidin-1-yl)-3-amino-2-(3-hydroxypyrrolidin-1-yl)pyridin
6-(2-Hydroxymethyl-4-hydroxypyrrolidin-1-yl)-3-amino-2-(3-hydroxypyrrolidin-1-yl)pyridin
6-(2-Hydroxymethyl-2-hydroxypyrrolidin-1-yl)-3-amino-2-(3-hydroxypyrrolidin-1-yl)pyridin
6-(2-Carboxamidopyrrolidin-1-yl)-3-amino-2-(3-hydroxypyrrolidin-1-yl)pyridin
6-(2-Dimethylcarboxamidopyrrolidin-1-yl)-3-amino-2-(3-hydroxypyrrolidin-1-yl)pyridin
6-(2-Carboxamido-3-hydroxypyrrolidin-1-yl)-3-amino-2-(3-hydroxypyrrolidin-1-yl)pyridin
6-(2-Carboxamido-4-hydroxypyrrolidin-1-yl)-3-amino-2-(3-hydroxypyrrolidin-1-yl)pyridin
6-(Pyrrolidin-1-yl)-3-amino-2-(2-carboxypyrrolidin-1-yl)pyridin
6-(3-hydroxypyrrolidin-1-yl)-3-amino-2-(2-carboxypyrrolidin-1-yl)pyridin
6-(3-Aminopyrrolidin-1-yl)-3-amino-2-(2-carboxypyrrolidin-1-yl)pyridin
6-(3-Acetamidopyrrolidin-1-yl)-3-amino-2-(2-carboxypyrrolidin-1-yl)pyridin
6-(3,4-Dihydroxypyrrolidin-1-yl)-3-amino-2-(2-carboxypyrrolidin-1-yl)pyridin
6-(2-Carboxypyrrolidin-1-yl)-3-amino-2-(2-carboxypyrrolidin-1-yl)pyridin
6-(2-Carboxy-3-hydroxypyrrolidin-1-yl)-3-amino-2-(2-carboxypyrrolidin-yl)pyridin
6-(2-Carboxy-4-hydroxypyrrolidin-1-yl)-3-amino-2-(2-carboxypyrrolidin-1-yl)pyridin
6-(2-(Hydroxymethyl)pyrrolidin-1-yl)-3-amino-2-(2-carboxypyrrolidin-1-y)pyridin
6-(2-Hydroxymethyl-4-hydroxypyrrolidin-3-yl)-3-amino-2-(2-carboxypyrrolidin-1-yl)pyridin
6-(2-Hydroxymethyl-2-hydroxypyrrolidin-1-yl)-3-amino-2-(2-carboxypyrrolidin-1-yl)-pyridin
6-(2-Carboxamidopyrrolidin-1-yl)-3-amino-2-(2-carboxypyrrolidin-1-yl)pyridin
6-(2-Dimethylcarboxamidopyrrolidin-1-yl)-3-amino-2-(2-carboxypyrrolidin-1-yl)pyridin
6-(2-Carboxamido-3-hydropypyrrolidin-1-yl)-3-amino-2-(2-carboxypyrrolidin-1-yl)pyridin
6-(2-Carboxamido-4-hydroxypyrrolidin-1-yl)-3-amino-2-(2-carboxypyrrolidin-1-yl)pyridin
6-(Pyrrolidin-1-yl)-3-amino-2-(2-(hydroxyethyl)-pyrrolidin-1-yl)pyridin
6-(3-Hydroxypyrrolidin-1-yl)-3-amino-2-(2-(hydroxmethyl)pyrrolidin-1-yl)pyridin
6-(3-aminopyrrolidin-1-yl)-3-amino-2-(2-(hydroxymethyl)pyrrolidin-1-yl)pyridin
6-(3-Acetamidopyrrolidin-1-yl)-3-amino-2-(2-(hydroxymethyl)pyrrolidin-1-yl)pridin
6-(3,4-Dihydroxypyrrolidin-1-yl)-3-amino-2-(2-(hydroxymethyl)pyrrolidin-1-yl)pyridin
6-(2-Carboxyrrolidin-1-yl)-3-amino-2-(2-(hydroxymethyl)pyrrolidin-1-yl)pyridin
6-(2-Carboxy-3-hydroxypyrrolidin-1-yl)-3-amino-2-(2-hydroxymethyl)pyrrolidin-1-yl)pyridin
6-(2-Carboxy-4-hydroxypyrrolidin-1-yl)-3-amino-2-(2-(hydroxymethyl)pyrrolidin-1-yl)pyridin
6-(2-(Hydroxymethyl)pyrrolidin-1-yl)-3-amino-2-(2-(hydroxymethyl)pyrrolidin-1-yl)pyridin
6-(2-Hydroxymethyl-4-hydroxypyrrolidin-1-yl)-3-amino-2-(2-(hydroxymethyl)pyrrolidin-1-yl)pyridin
6-(2-Hydroxymethyl-2-hydroxypyrrolidin 1 yl)-3-amino-2-(2-hydroxymethyl)pyrrolidin-1-yl)pyridin
6-(2-Carboxamidopyrrolidin-1-yl)-3-amino-2-(2-(hydroxymethyl)pyrrolidin-1-yl)pyridin
6-(2-Dimethylcarboxamidopyrrolidin-1-yl)-3-amino-2-(2-(hydroxymethyl)pyrrolidin-1-yl)pyridin
6-(2-Carboxamido-3-hydroxypyrrolidin-1-yl)-3-amino-2-2-(hydroxymethyl)pyrrolidin-1-yl)pyridin
6-(3-Carboxamido-4-hydroxypyrrolidin-1-yl)-3-amino-2-(2-hydroxymethyl)pyrrolidin-1-yl)pyridin
6-(Pyrrolidin-1-yl)-3-(2-carboxamidopyrrolidin-1-yl)pyridin
6-(3-Hydroxypyrolidin-1-yl)-3-amino-2-(2-carboxamidopyrrolidin-1-yl)pyridin
6-(3-Aminopyrrolidin-1-yl)-3-amino-2-(2-carboxamidopyrrolidin-1-yl)pyridin
6-(3-Acetamidopyrrolidin-1-yl)-3-amino-2-(2-carboxamidopyrralidin-1-yl)pylidin
6-(3,4-Dihydroxypyrrolidin-1-yl)-3-amino-2-(2-carboxamidopyrrolidin-1-yl)pyridin
6-(2-Carboxypyrrolidin-1-yl)-3-amino-2-(2-carboxamidopyrrolidin-1-yl)pyridin
6-(2-Carboxy-3-hydroxypyrrolidin-1-yl)-3-amino-2-(2-carboxamidopyrrolidin-1-yl)pyrridin
6-(2-Carboxy-4-hydroxypyrrolidin-1-yl)-3-amino-2-(2-carboxamidopyrrolidin-1-yl)pyridin
6-(2-(Hydroxymethyl)pyrrolidin-1-yl)-3-amino-2-(2 carboxamidopyrrolidin-1-yl)pyridin
6-(2-Hydromethyl-4-hydroxypyrrolidin-1-yl)-3-amino-2-(2-carboxamidopyrrolidin-1-yl)pyridin
6-(2-Hydroxymethyl-2-hydroxypyrrolidin-1-yl)-3-amino-2-(2-carboxamidopyrrolidin-1-yl)pyridin
6-(2-Carboxamidopyrrolidin-1-yl)-3-amino-1-(2-carboxamidopyrrolidin-1-yl)pyridin
6-(2-Dimethylcarboxamidopyrrolidin-1-yl)-3-amino-2-(2-carboxamidopyrrolidin-1-yl)pyridin
6-(2-Carboxamido-3-hydroxypyrrolidin-1-yl)-3-amino-2-(2-carboxamidopyrrolidin-1-yl)pyridin
6-(2-Carboxamido-4-hydroxypyrrolidin-1-yl) 3-amino-2-(2-carboxamidopyrrolidin-1-yl)pyridin
6-(Pyrrolidin-1-yl)-3-amino-2-dimethylaminopyridin 6-(3-Hydroxypyrrolidin-1-yl)-3-amino-2-dimethylaminopyridin
6-(3-Aminopyrrolidin-1-yl)-3-amino-2-dimethylaminopyridin
6-(3-Acetamidopyrrolidin-1-yl)-3-amino-2-dimethylaminopyridin
6-(3,4-Dihydroxypyrrolidin-1-yl)-3-amino-2-dimethylaminopyridin
6-(2-Carboxypyrrolidin-1-yl)-3-amino-2-dimethylaminopyridin
6-(2-Carboxy-3-hydroxypyrrolidin-1-yl)-3-amino-2-dimethylaminopyridin
6-(2-Carboxy-4-hydroxypyrrolidin-1-yl)-3-amino-2-dimethylaminopyridin
6-(2-(Hydroxymethyl)pyrrolidin-1-yl)-3-amino-2-dimethylaminopyridin
6-(2-Hydroxymethyl-4-hydroxyrrolidin-1-yl)-3-amino-1-dimethylaminopiridin
6-(2-Hydroxymethyl-2-hydroxypyrrolidin-1-yl)-3-amino-2-dimethylaminopyridin
6-(2-Carboxamidopyrrolidin-1-yl)-3-amino-2-dimethylaminopyridin
6-(2-Dimethylcarboxamidopyrrolidin-1-yl)-3-amino-2-dimethylaminopyridin
6-(2-Carboxamido-3-hydroxypyrrolidin-1-yl)-3-amino-2-dimetylaminopyridin
6-(2-Carboxamido-4-hydroxypyrrolidin-1-yl)-3-amino-2-dimethylaminopyridin
6-(Pyrrolidin-1-yl)-3-amino-2-[methyl(2-hydroxyethyl)amino]pyridin
6-(3-Hydroxypyrrolidin-1-yl)-3-amino-2-[methyl(2-hydroxyethyl)amino]pyridin
6-(3-Aminopyrrolidin-1-yl)-3-amino-2-[methyl(2-hydroxyehyl)amino]pyridin
6-(3-Acetamidopyrrolidin-1-yl)-3-amino-2-[methyl-(2-hydroxyethyl)amino]pyridin
6-(3,4-Dihydroxypyrolidin-1-yl-)-3-amino-2-[methyl(2-hydroxyethyl)amino]pyridin
6-(2-Carboxypyrrolidin-1-yl)-3-amino-2-[methyl(2-hydroxyethyl)amino]pyridin
6-(2-Carboxy-3-hydroxypyrrolidin-1-yl)-3-amino-2-[methyl(2-hydroxyethyl)amino]pyridin
6-(2-Carboxy-4-hydroxypyrrolidin-1-yl)-3-amino-2-[methyl(2-hydroxyehyl)amino]pyridin
6-(2-(Hydroxymethylpyrrolidin-1-yl)-3-amino-2-[methyl(2-hydroxyethyl)amino]pyridin
6-(2-Hydroxymethyl-4-hydroxypyrrolidin-1-yl)-3-amino-2-[methyl(2-hydroxyethyl)amino]pyridin
6-(2-Hydroxymethyl-2-hydroxypyrrolidin-1-yl)-3-amino-2-[methyl(2-hydroxyethyl)amino]pyridin
6-(2-Carboxaminopyrrolidin-1-yl)-3-amino-2-[methyl(-2-hydroxyethyl)amino]pyridin
6-(2-Dimethylcarboxamidopyrrolidin-1-yl)-3-amino-2-[methyl(2-hydroxyethyl)amino]pyridin
6-(2-Carboxamido-3-hydroxypyrrolidin-1-yl)-3-amino-2-[methyl(2-hydroxyethyl)amino]pyridin
6-(2-Carboxamido-4-hydroxypyrrolidin-1-yl)-3-amino-2-[methyl(2-hydroxyethyl)amino]pyridin
6-(pyrrolidin-1-yl)-3-amino-2-[methyl(2-aminoethyl)amino]pyridin
6-(3-Hydroxypyrrolidin-1-yl)-3-amino-2-[methyl(2-aminoethyl)amino]pyridin
6-(3-Aminopyrrolidin-1-yl)-3-amino-2-[methyl(2-aminoethyl)amino]pyridin
6-(3-Acetamidopyrrolidin-1-yl)-3-amino-2-[methyl-(2-aminoethyl)amino]pyridin
6-(3,4-Dihydroxypyrrolidin-1-yl)-3-amino-2-[methyl(2-aminoethyl)amino]pyridin
6-(2-Carboxypyrrolidin-1-yl)-3-amino-2-[methyl(2-aminoethyl)amino]pyridin
6-(2-Carboxy-3-hydroxypyrrolidin-1-yl)-3-amino-2-[methyl(2-aminoethyl-1-yl)amino]pyridin
6-(2-carboxy-4-hydroxypyrrolidin-1-yl)-3-amino-2-[methyl(2-aminoethyl)amino]pyridin
6-(2-(Hydroxymethyl)pyrrolidin-1-yl)-3-amino-2-[methyl(2-aminoethyl)amino]pyridin
6-(2-Hydroxymethyl-4-hydroxypyrrolidin-1-yl)-3-amino-2-[methyl(2-aminoethyl)amino]pyridin
6-(2-Hydroxymethyl-2-hydroxypyrrolidin-1-yl)-3-amino-2-[methyl(2-aminoethyl)amino]pyridin
6-(2-Carboxamidopyrrolidin-1-yl)-3-amio-2-[methyl(2-aminoethyl)amino]pyridin
4-(2-Dimethylcarboxamidopyrrolidin-1-yl)-3-amino-2-[methyl(2-aminoethyl)amino]pyridin
6-(2-Carboxamido-3-hydroxypyrrolidin-3-yl)-3-amino-2-[methyl(2-aminoethyl)amino]pyridin
6-(2-carboxamido-4-hydroxypyrrolidin-1-yl)-3-amino-2-[methyl(2-aminoethy)amino]pyridin
6-(Pyrrolidin-1-yl)-3-amino-2-[methyl(2-acetamidoethyl)amino]pyridin
6-(3-Hydroxypyrrolidin-1-yl)-3-amino-2-[methyl(2-acetamidoethyl)amino]pyridin
6-(3-Aminopyrrolidin-1-yl)-3-amino-2-[methyl(2-acetamidoethyl)amino]pyridin
6-(3-Acetamidopyrrolidin-1-yl)-3-amino-2-[methyl-(2-acetamidoethyl)amino]pyridin
6-(3,4-Dihydroxypyrrolidin-1-yl)-3-amino-2-(methyl(2-acetamidoethyl)amino)pyridin
6-(2-Carboxypyrrolidin-1-yl)-3-amino-2-[methyl(2-acetamidoethyl)amino]pyridin
6-(2-Carboxy-3-hydroxypyrrolidin-1-yl)-3-amino-2-[methyl(2-acetamidoethyl)amino]pyridin
6-(2-Carboxy-4-hydroxypyrrolidin-1-yl)-3-amino-2-[methyl(2-acetamidoethyl)amino]pyridin
6-(2-(Hydroxymethyl)pyrrolidin-1-yl)-3-amino-2-[methyl(2-acetamidoethyl)amino]pyridin
6-(2-Hydroxymethyl-4-hydroxypyrrolidin-1-yl)-3-amino-2-[methyl-(2-acetamidoethyl)amino]pyridin
6-(2-Hydroxymethyl-2-hydroxypyrrolidin-1-yl)-3-amino-2-[methyl(2-acetamidoethyl)amino]pyridin
6-(2-Carboxamidopyrrolidin-1-yl)-3-amino-2-[methyl(2-acetamidoethyl)amino]pyridin
6-(2-Dimethylcarboxamidopyrrolidin-1-yl)-3-amino 2-[methyl(2-acetamidoethyl)amino]pyridin
6-(2-Carboxamido-3-hydroxypyrrolidin-1-yl)-3-amino-2-[methyl(2-acetamidoethyl)amino]pyridin
6-(2-Carboxamido-4-hydroxypyrrolidin-1-yl)-3-amino-2-[methyl(2-acetamidoethyl)amino]pyridin
6-(Pyrrolidin-1-yl)-3-amino-2-methoxypyridin
6-(3-Hydroxypyrrolidin-1-yl)-3-amino-2-methoxypyridin
6-(3-Aminopyrrolidin-1-yl)-3-amino-2-methoxypyridin
6-(3-Acetamidopyrrolidin-1-yl)-3-amino 2-methoxypyridin
6-(3,4-Dihydroxypyrrolidin-1-yl)-3-amino-2-methoxypyridin
6-(2-Carboxypyrrolidin-1-yl)-3-amino-2-methoxypyridin
6-(2-Carboxy-3-hydroxypyrrolidin-2-yl)-2-amino-2-methoxypyridin
6-(2-Carboxy-4-hydroxypyrrolidin-1-yl)-3-amino-2-methoxypyridin
6-(2-(Hydroxymethyl)pyrrolidin-1-yl)-3-amino-2-methoxypyridin
6-(2-Hydroxymethyl-4-hydroxypyrrolidin-1-yl)-3-amino-2-methoxypyridin
6-(2-Hydroxymethyl-2-hydroxypyrrolidin-yl)-3-amino-2-methoxypyridin
6-(2-Carboxamidopyrrolidin-1-yl)-3-amino-2-methoxypyridin
6-(2-Dimethylcarboxamidopyrrolidin-1-yl)-3-amino-2-methoxypyridin
6-(2-Carboxamino-3-hydroxypyrrolidin-1-yl)-3-amino-2-methoxypyridin
6-(2-Carboxamido-4-hydroxypyrrolidin-1-yl)-3-amino-2-methoxypyridin
6-(Pyrrolidin-1-yl)-3-amino-2-(2-hydroxyethyloxy)-pyridine
6 (3-Hydroxypyrrolidin-1-yl)-3-amino-3-(2-hydroxyethyloxy)pyridin
6-(3-Aminopyrrolidin-1-yl)-3-amino-2-(2-hydroxyethyloxy)pyridin
6-(3-acetamidopyrrolidin-1-yl)-3-amino-2-(2-hydroxyethyloxy)pyridin
6-(3,4-Dihydroxypyrrolidin-1-yl)-3-amino-2-(2-hydroxyethyloxy)pyridin
6-(2-Carboxypyrrolidin-1-yl)-3-amino-2-(2-hydroxyethyloxy)pyridin
6-(2-Carboxy-3-hydroxypyrrolidin-1-yl)-3-amino-2-(2-hydroxyethyloxy)pyridin
6-(2-Carboxy-4-hydroxypyrrolidin-1-yl)-3-amino-2-(2-hydroyethyloxy)pyridin
6-(2-(Hydroxymethyl)pyrrolidin-1-yl)-3-amino-2-(2-hydroxyethyloxy)pyridin
6-(2-Hydroxymethyl-4-hydroxypyrolidin-1-yl)-3-amino-2-(2-hydroxyethyloxy)pyridin
6-(2-Hydroxymethyl-2-hydroxypyrrolidin-1-yl)-3-amino-2-(2-hydroxyethyloxy)pyridin
6-(2-Carboxamidopyrrolidin-1-yl)-1-amino-2-(2-hydroxyethyloxy)pyridin
6-(2-Dimethylcarboxamidopyrrolidin-1-yl)-3-amino-2-(2-hydroxyethyloxy)pyridin
6-(2-carboxamido-3-hydroxypyrrolidin-1-yl)-3-amino-2-(2-hydroxyethyloxy)pyridin
6-(2-Carboxamido-4-hydroxypyrrolidin-1-yl)-3-amino-2-(2-hydroxyethyloxy)pyridin
6-(Pyrrolidin-1-yl)-3-amino-2-ethoxypyridin
6-(3-Hydroxypyrrolidin-1-yl)-3-amino-2-ethoxy pyridin
6-(3-Aminopyrrolidin-1-yl)-3-amino-2-ethoxypyridin
6-(3-Acetamidopyrrolidin-1-yl)-3-amino-2-ethoxypyridin
6-(3,4-Dihydroxypyrrolidin-1-yl)-3-amino-2-ethoxy pyridin
6-(2-Carboxypyrolidin-1-yl)-3-amino-2-ethoxy" pyridin
6-(2-Carboxy-3-hydroxypyrrolidin-1-yl)-3-amino-2-ethoxypyridin
6-(2-Carboxy-4-hydroxypyrrolidin-1-yl)-3-amino-2-ethoxypyridin
6-(2-(Hydroxymethyl)-pyrrolidin-1-yl)-3-amino-2-ethoxypyridin
6-(2-Hydroxymethyl-4-hydroxypyrrolidin-1-yl)-3-amino-2-ethoxypyridin
6-(2-Hydroxyethyl-2-hydroxypyrrolidin-1-yl)-3-amino-2-ethylpyridin
6-(2-Carboxamidopyrrolidin-1-yl)-3-amino-2-ethoxypyridin
6-(2-Dimethylcarboxamidopyrrolidin-2-yl)-3-amino-2-ethoxypyridin
6-(2-Carboxamido-3-hydroxypyrrolidin-1-yl)-3-amino-2-ethoxypyridin
6-(2-Carboxamido-4-hydroxypyrrolidin-1-yl)-3-amino-2-ethoxypyridin
und Additionssalzen davon.

9. Zusammensetzung nach Anspruch 8, in der die Oxidationsbase der Formel (I) ausgewählt ist unter:
2-Methoxy-6-(pyrrolidin-1-yl)-3-aminopyridin
5,6-Diamino-2-(pyrrolidin-1-yl)pyridin
N-(5,6-Diaminopyridin-2-yl)-3-hydroxypyrrolidin
2,6-Di(pyrrolidin-1-yl)-5-aminopyridin
und Additionssalzen davon.

10. Zusammensetzung nach einem der Ansprüche 1 bis 9, die außerdem eine oder mehrere zusätzliche Oxidationsbasen umfaßt, die unter para-Phenylenaminen, Bisphenylalkylendiaminen, para-Aminophenolen ortho-Aminophenolen, anderen heterocyclischen Basen als den in den Ansprüchen 1 bis 9 definierten und Additionssalzen davon ausgewählt sind.

11. Zusammensetzung nach einer, der Ansprüche 1 bis 10, in der die Oxidationsbase der Formel (I) bzw. die Oxidationsbasen der Formel (I) oder die zusätzliche Base bzw. die zusätzlichen Basen jeweils in einer Menge zwischen 0,001 und 10 Gew.-%, bezogen auf das Gesamtgewicht der Färbezusammensetzung, vorliegen.

12. Zusammensetzung nach einem der vorhergehenden Ansprüche, die einen oder mehrere Kuppler umfaßt, die unter meta-Phenylendiaminen, meta-Aminophenolen, meta-Diphenolen, Naphthalin-Kupplern, heterocyclischen Kupplern und Additionssalzen davon ausgewählt sind.

13. Zusammensetzung nach Anspruch 12, in der der Kuppler bzw. die Kuppler in einer Menge zwischen 0,031 und 10 Gew.-%, bezogen auf das Gesamtgewicht der Färbezusammensetzung, vorliegen.

14. Zusammensetzung nach einem der Ansprüche 1 bis 13, die ein Oxidationsmittel umfaßt.

15. Zusammensetzung nach Anspruch 14, in der das Oxidationsmittel unter Wasserstoffperoxid, Harnstoffperoxid, Alkalimetallbromaten, Persalzen, Persäuren und Oxydaseenzymen ausgewählt ist.

16. Verfahren zum Färben von Keratinfasern, **dadurch gekennzeichnet, daß** man auf die Fasern eine Zusammensetzung gemäß einem der Ansprüche 1 bis 13 aufbringt und die Farbe mit Hilfe eines Oxidationsmittels entwickelt.

17. Verfahren nach Anspruch 16, bei dem man das Oxidationsmittel unter Wasserstoffperoxid, Harnstoffperoxid, Alkalimetallbromaten, Persalzen, Persäuren und Oxydaseenzymen auswählt,

18. Verfahren nach einem der Ansprüche 16 und 17, bei dem man das Oxidationsmittel zum Zeitpunkt der Verwendung mit der Zusammensetzung gemäß einem der Ansprüche 1 bis 13 mischt,

19. Verfahren nach einem der Ansprüche 16 und 17, bei dem man das Oxidationsmittel gleichzeitig mit oder nach der Zusammensetzung gemäß einem der Ansprüche 1 bis 13 auf die Fasern aufbringt.

20. Vorrichtung mit mehreren Kompartimenten oder Färbe-"Kit" mit mehreren Kompartimenten, wobei ein erstes Färbekompartiment eine Zusammensetzung gemäß einem der Ansprüche 1 bis 13 enthält und ein zweites Kompartiment eine oxidierende Zusammensetzung enthält.

21. Gefärbtes Produkt, das durch oxidative Kondensation einer Zusammensetzung gemäß einem der Ansprüche 1 bis 13 erhältlich ist.

22. Verwendung der Zusammensetzung gemäß einem der Anspruche 1 bis 9 zum Färben von Keratinfasern.

23. 2,5-Diaminopyridinderivat der folgenden Formel (I'): 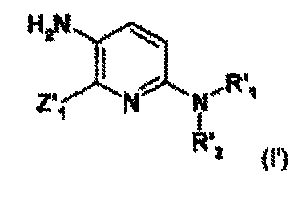 worin
Z'₁ für einen OR'₃- oder NR'₄R'₅-Rest steht;
R'_{1,} R'₂, R'₄ und R'₅ unabhängig voneinander für ein Wasserstoffatom, einen C₂-C₆-(poly)aminoalkylrest, einen C₂-C₆-(Poly)alkylaminoalkylrest, einen C₂-C₆-Aminohydroxyalkylrest, einen C₂-C₆-Acylaminoalkylrest, einen C₂-C₆-Alkoxyalkylrest, einen C₂-C₆-Hydroxyalkoxyalkylrest, einen C₂-C₆-Aminoalkoxyalkylrest, einen C₂-C₆-(Poly)hydroxylkylrest, einen C₂-C₆-Carboxyalkylrest oder einen C₂-C₆-Sulfalkylrest stehen;
R'₁ und R'₂ einerseits und R'₄ und R'₅ andererseits unabhängig voneinander mit dem Stickstoffatom, an das sie gebunden sind, einen gesättigten Heterocyclus mit 5 bis 8 Gliedern, der gegebenenfalls durch einen oder mehrere Reste aus der Gruppe bestehend aus Halogenatomen, Amino-, (Di)alkylamino-, Hydroxy-, Carboxy-, Carboxamido- und Alkoxyreste und C₁-C₆-Alkylrester, die gegebenenfalls durch einen oder mehrere Hydroxy-, Amino-, (Di)alkylamino-, Alkoxy-, Hydroxyalkoxy-, Aminoalkoxy-, Carboxy- oder Sulforeste substituiert sind, substituiert ist, bildern, wobei der Heterocyclus ein oder mehrere zusätzliche Heteroatome, die unter Sauerstoff, gegebenenfalls substituiertem Stickstoff, Schwefel oder einer SO₂-Gruppe ausgewählt sind, enthalten kann, wobei der Heterocyclus weder eine Peroxidbindung noch Diazo- oder Nitroreste enthält;
R'₃ für einen linearen oder verzweigten C₁-C₆-Alkylrest, gegebenenfalls substituiert durch einen oder mehrere Reste, ausgewählt unter Resten des Typs Hydroxy, Amino, Mono- oder Di-C₁-C₃-alkylamino, deren Alkylteil gegebenenfalls substituiert ist durch eine oder mehrere Gruppen des Typs Hydroxyl Amino, C₁-C₂-Alkoxyalkoxy, Carboxy, steht; mit Ausnahme von 2,3,6-Triaminopyridin, 6-(4-Morpholinyl)-2,3-diaminopyridin, 6-(1-Piperidinyl)-2,3-diaminopyridin und 2,6-Di(4-morpholinyl)-3-aminopyridin und Additionssalzen davon;
mit Ausnahme von 2,3 6-(4-methylpiperazinyl)pyridin.

24. 2,5-Diaminopyridinderivat der Formel (I') nach Anspruch 23, ausgewählt unter den folgenden Verbindungen:
2-Methoxy-pyrrolidin-1-yl)-3-aminopyridin
1-(5-amino-6-methoxypyridin-2-yl)pyrrolidin-3-ol
3-Amino-2-methoxy-6-(morpholin-4-yl)pyridin,
3-Amino-2-methoxy-6-(piperidin-1-yl)pyridin,
6-N,N-(2-hydroxyethyl)methyl)amino)-3-amino-2-methoxypyridin
5,6-Diamino-2-(pyrrolidin-1-yl)pyridin
N-(5,6-Diaminopyridin-2-yl)-3-hydroxypyrrolidin
2,6-Di(pyrrolidin-1-yl)-5-aminopyridin
6-(Pyrrolidin-1-yl)-2,3-diaminopyridin
6-(3-Hydroxypyrrolidin-1-yl)-2,3-diaminopyridin
6-(3-Aminopyrrolidin-1-yl)-2,3-diaminopyridin
6-(3-Acetamidopyrrolidin-1-yl)-2,3-diaminopyridin
6-(3,4-Dihydroxypyrrolidin-1-yl)-2,3-diaminopyridin 6-(2-Carboxypyrrolidin-1-yl)-2,3-diaminopyridin
6-(2-Carboxy-3-hydroxypyrrolidin-1-yl)-2,3-diaminopyridin
6-(2-Carboxy-4-hydroxypyrrolidin-1-yl)-2,3-diaminopyridin
6-(2-(Hydroxymethyl)pyrrolidin-1-yl)-2,3-diaminopyridin
6-(2-Hydroxymethyl-4-hydroxypyrrolidin-1-yl)-2,3-diaminopyridin
6-(2-Hydroxyethyl-2-hydroxypyrrolidin-1-yl)-2,3-diamopyridin
6-(2-Carboxamidopyrrolidin-1-yl)-2,3-diaminopyridin
6-(2-Dimethylcarboxamidopyrrolidin-1-yl)-2,2-diaminopyridin
6-(2-Carboxamido-3-hydroxypyrrolidin-1-yl)-2,3-diaminopyridin
6-(2-carboxamido-4-hydroxypyrrolidin-1-yl)-2,3-diaminopyridin
6-(Pyrrolidin-1-yl)-3-amino-2-(2-hydroxyethylamino)pyridin
6-(3-Hydroxypyrrolidin-1-yl)-3-amino-2-(2-hydroxy ethylamino)pyridin
6-(3-Aminopyrrolidin-1-yl)-3-amino-2-(2-hydroxy ethylanino)pyridin
6-(3-Acetamidopyrrolidin-1-yl-)-3-amino-2-(2-hydroxyethylamino)pyridin
6-(3,4-Dihydroxypyrrolidin-1-yl)-3-amino-2-(2-hydroxyethylamino)pyridin
6-(2-Carboxypyrrolidin-1-yl)-3-amino-2-(2-hydroxyethylamino)pyridin
6-(2-Carboxy-3-hydroxypyrrolidin-1-yl)-3-amino-2-(2-hydroxyethylamino)pyridin
6-(2-Carboxy-4-hydroxypyrrolidin-1-yl)-3-amino-2-(2-hydroxyethylamino)pyridin
6-(2-(Hydroxymethyl)pyrrolidin-1-yl)-3-amino-2-(2-hydroxyethylamino)pyridin
6-(2-Hydroxyethyl-4-hydroxypyrrolidin-1-yl)-3-amino-2-(2-hydroxyethylamino)pyridin
6-(2-Hydroxymethyl-2-hydroxypyrrolidin-1-yl)-3-amino-2-(2-hydroxyethylamino)pyridin
6-(2-Carboxamidopyrrolidin-1-yl)-3-amino-2-(2-hydroxyethylamino)pryridin
6-(2-Dimethylcarboxamidopyrrolidin-1-yl)-3-amino-2-(2-hydroxyethylamino)pyridin
6-(2-Carboxamido-3-hydroxypyrrolidin-1-yl)-3-amino-2-(2-hydroxyethylamino)pyridin
6-(2-Carboxamido-4-hydroxypyrrolidin-1-yl)-3-aminopyridin
6-(Pyrrolidin-1-yl)-3-amino-2-(2-aminoethylamino)-pyridin
6-(3-Hydroxypyrrolidin-1-yl)-3-amino-2-(2-aminoethylamino)pyridin
6-(3-Aminopyrrolidin-1-yl)-3-amino-2-(2-aminoethylamino)pyridin
6-(3-Acetamidopyrrolidin-1-yl)-3-amino-2-(2-aminoethylamino)pyridin
6-(3,4-Dihydroxypyrrolidin-1-yl)-3-amino-2-(2-aminoethylamino)pyridin
6-(2-Carboxypyrrolidin-1-yl)-3-amino-2-(2-aminoethylamino)pyridin
6-(2-Carboxy-3-hydroxypyrrolidin-1-yl)-3-amino-2-(2-aminoethylamino)pyridin
6-(2-Carboxy-4-hydroxypyrrolidin-2-yl)-3-amino-2-(2-aminoethylamino)pyridin
6-(2-(Hydroxymethyl)pyrrolidin-1-yl)-3-amino-2-(2-aminoethylamino)pyridin
6-(2-Hydroxymethyl-4-hydroxypyrrolidin-1-yl)-3-amino-2-(2-aminoethylamino)pyridin
6-(2-Hydroxymethyl-2-hydroxypyrrolidin-1-yl)-3-amino-2-(2-aminoethylamino)pyridin
6-(2-Carboxamidopyrrolidin-1-yl)-3-amino-2-(2-aminoethylamino)pyridin
6-(2-Dimethylcarboxamidopyrrolidin-1-yl)-3-amino-2-(2-aminoethylamino)pyridin
6-(2-Carboxamido-3-hydroxypyrrolidin-1-yl)-3-amino-2-(2-aminoethylamino)pyridin
6-(2-Carboxamido-4-hydroxypyrrolidin-1-yl)-3-amino-2-(2-aminoethylamino)pyridin
6-(pyrrolidin-1-yl)-3-amino-2-(2-acetamidoethylamino)pyridin
6-(3-Hydroxypyrrolidin-1-yl)-3-amino-2-(2-acetamidoethylamino)pyridin
6-(3-Aminopyrrolidin-1-yl)-3-amino-2-(2-acetamido ethylamino)pyridin
6-(3-Acetamidopyrrolidin-1-yl)-3-amino-2-(2-acetamidoethylamino)pyridin
6-(3,4-Dihydroxypyrrolidin-1-yl)-3-amino-2-(2-acetamidoethylamino)pyridin
6-(2-Carboxypyrrolidin-1-yl)-3-amino-2-(2-acetamidoethylamino)pyridin
6-(2-Carboxy-3-hydroxypyrrolidin-1-yl)-3-amino-2-(2-acetamidoethylamino)pyridin
6-(2-Carboxy-4-hydroxypyrrolidin-1-yl)-3-amino-2-(2-acetamidoethylamino)pyridin
6-(2-(Hydroxymethyl)pyrrolidin-1-yl)-3-amino-2-(2-acetamidoethylamino)pyridin
6-(2-Hydroxymethyl-4-hydroxypyrrolidin-1-yl)-3-amino-2-(2-acetamidoethylamino)pyridin
6-(2-Hydyoxymethyl-2-hydroxypyrrolidin-1-yl)-3-amino-2-(2-acetamidoethylamino)pyridin
6-(2-Carboxamidopyrrolidin-1-yl)-3-amino-2-(2-acetamidoethylamino)pyridin
6-(2-Dimethylcarboxamidopyrrolidin-1-yl)-3-amino-3-(2-acetamidoethylamino)pyridin
6-(2-Carboxamido-3-hydroxypyrrolidin-1-yl)-3-amino-2-(2-acetamidoethylamino)pyridin
6-(2-Carboxamido-4-hydroxypyrrolidin-1-yl)-3-amino-2-(2-acetamidoethylamino)pyridin
6-(Pyyrolidin-1-yl)-3-amino-2-(pyrrolidin-1-yl)-pyridin
6-(3-Hydroxypyrrolidin-1-yl)-3-amino-2-(pyrrolidin-1-yl)pyridin
6-(3-Aminopyrrolidin-1-yl)-3-amino-2-(pyrrolidin-1-yl)pyridin
6-(3-Acetamidopyrrolidin-1-yl)-3-amino-2-(pyrrolidin-1-yl)pyridin
6-(3,4-Dihydroxypyrrolidin-1-yl)-3-amino-2-(pyrrolidin-1-yl)pyridin
6-(2-Carboxypyrrolidin-1-yl)-3-amino-2-(pyrrolidin-1-yl)pyridin
6-(2-Carboxy-3-hydroxypyrrolidin-1-yl)-3-amino-2-(pyrrolidin-1-yl)-pyridin
6-(2-Carboxy-4-hydroxypyrrolidin-1-yl)-3-amino-2-(pyryolidin-1-yl)pyridin
6-(2-(Hydroxymethyl)pyrrolidin-1-yl)-3-amino-2-(pyrrolidin-1-yl)pyridin
6-(2-Hydroxymethyl-4-hydroxypyrrolidin-1-yl)-3-amino-2-(pyrrolidin-1-yl)pyridin
6-(2-Hydroxymethyl-2-hydroxypyrrolidin-1-yl)-3-amino-2-(pyrrolidin-1-yl)pyridin
6-(2-carboxamidopyrrolidin-1-yl)-3-amino-2-(pyrrolidin-1-yl)pyridin
6-(2-Dimethylcarboxamidopyrrolidin-1-yl)-3-amino-2-(pyrrolidin-1-yl)pyridin
6-(2-Carboxamido-3-hydroxypyrrolidin-1-yl)-3-amino-2-(pyrrolidin-1-yl)pyridin
6-(2-Carboxamido-4-hydroxypyrrolidin-1-yl)-3-amino-2-(pyrrolidin-1-yl)pyridin
6-(Pyrrolidin-1-yl)-3-amino-2-(3-hydroxypyrrolidin-1-yl)pyridin
6-(3-Hydroxypyrrolidin-1-yl)-3-amino-2-(3-hydroxypyrrolidin-1-yl)pyridin
6-(3-Aminopyrrolidin-1-yl)-3-amino-2-(3-hydroxypyrrolidin-1-yl)pyridin
6-(3-Acetamidopyrrolidin-1-yl)-3-amino-2-(3-hydroxypyrrolidin-1-yl)pyridin
6-(3,4-Dihydroxypyrrolidin-1-yl)-3-amino-2-(3-hydroxypyrrolidin-1-yl)pyridin
6-(3-Carboxypyrrolidin-1-yl)-amino-2-(3-hydroxypyrrolidin-1-yl)pyridin
6-(2-Carboxy-3-hydroxypyrrolidin-1-yl)-3-amino-2-(3-hydroxypyrrolidin-1-yl)pyridin
6-(2-carboxy-4-hydroxypyrrolidin-1-yl)-3-amino-2-(3-hydroxypyrrolidin-1-yl)pyridin
6-(2-(Hydroxymethyl)pyrrolidin-1-yl)-3-amino-2-(3-hydroxypyrrolidin-1-yl)pyridin
6-(2-Hydroxymethyl-4-hydroxypyrrolidin-1-yl)-3-amino-2-(3-hydroxypyrrolidin-1-yl)pyridin
6-(2-Hydroxymethyl-2-hydroxypyrrolidin-1-yl)-3-amino-2-(3-hydroxypyrrolidin-1-yl)pyridin
6-(2-Carboxamidopyrrolidin-1-yl)-3-amino-2-(3-hydroxypyrrolidin-1-yl)pyridin
6-(2-Dimethylcarboxamidopyrrolidin-1-yl)-3-amino-2-(3-hydroxypyrrolidin-1-yl)pyridin
6-(2-Carboxamido-3-hydroxypyrrolidin-1-yl)-3-amino-2-(3-hydroxypyrrolidin-1-yl)pyridin
6-(2-Carboxamido-4-hydroxypyrrolidin-1-yl)-3-amino-2-(3-hydroxypyrrolidin-1-yl)pyridin
6-(pyrrolidin-1-yl)-3-amino-2-(2-carboxypyrrolidin-1-yl)pyridin
6-(3-Hydroxypyrrolidin-1-yl)-3-amino-2-(2-carboxypyrrolidin-1-yl)pyridin
6-(3-Aminopyrrolidin-1-yl)-3-amino-2-(2-carboxypyrrolidin-1-yl)pyridin
6-(3-Acetamidopyrrolidin-1-yl)-3-amino-2-(2-carboxypyrrolidin-1-yl)pyridin
6-(3,4-Dihydroxypyrrolidin-1-yl)-3-amino-2-(2-carboxypyrrolidin-1-yl)pyridin
6-(2-Carboxypyrrolidin-1-yl)-3-amino-2-(2-carboxypyrrolidin-1-yl)pyridin
6-(2-Carboxy-3-hydroxypyrrolidin-1-yl)-3-amino-2-(2-carboxypyrrolidin-1-yl)pyridin
6-(2-Carboxy-4-hydroxypyrrolidin-1-yl)-3-amino-2-(2-carboxypyrrolidin-1-yl)pyridin
6-(2-(hydroxymethyl)pyrrolidin-1-yl)-3-amino-2-(2-carboxypyrrolidin-1-yl)pyridin
6-(2-Hydroxymethyl-4-hydroxypyrrolidin-1-yl)-3-amino-2-(2-carboxypyrrolidin-1-yl)pyridin
6-(2-Hydroxymethyl-2-hydroxypyrrolidin-1-yl)-3-amino-2-(2-carboxypyrrolidin-1-yl)pyridin
6-(3-Carboxamidopyrrolidin-1-yl)-3-amino-2-(2-carboxypyrrolidin-1-yl)pyridin
6-(2-Dimethylcarboxamidopyrrolidin-1-yl)-3-amino-2-(2-carboxypyrrolidin-1-yl)pyridin
6-(2-Carboxamido-3-hydroxypyrrolidin-1-yl)-3-amino-2-(2-carboxypyrrolidin-1-yl)pyridin
6-(2-Carboxanido-4-hydroxypyrrolidin-1-yl)-3-amino-2-(2-carboxypyrrolidin-1-yl)pyridin
6-(Pyrrolidin-1-yl)-3-amino-2-(2-(hydroxymethyl)-pyrrolidin-1-yl)pyridin
6-(3-Hydroxypyrrolidin-1-yl)-3-amino-2-(2-(hydroxymethyl)pyrrolidin-1-yl)pyridin
6. (3-Aminopyrrolidin-1-yl)-3-amino-2-(2-(hydroxymethyl)pyrrolidin-1-yl)pyridin
6-(3-Acetamidopyrrolidin-1-yl)-3-amino-2-(2-(hydroxymethyl)pyrrolidin-1-yl)pyridin
6-(3,4-Dihydroxypyrrolidin-1-yl)-3-amino-2-(2-(hydroxymethyl)pyrrolidin-1-yl)pyridin
6-(2-Carboxypyrrolidin-1-yl)-3-amino-2-(2-(hydroxymethyl)pyrrolidin-1-yl)pyridin
6-(2-Carboxy-3-hydroxypyrrolidin-1-yl)-3-amino-2-(2-(hydroxymethyl)pyrrolidin-1-yl)pyridin
6-(2-carboxy-4-hydroxypyrrolidin-1-yl)-3-amino-2-(2-(hydroxymethyl)pyrrolidin-1-yl)pyridin
6-(2-(Hydroxymethyl)pyrrolidin)-3-amino-2-(2-(hydroxymethyl)pyrrolidin-1-yl)pyridin
6-(2-Hydroxymethyl-4-hydroxypyrrolidin-1-yl)-3-amino-2-(2-(hydroxymethyl)pyrrolidin-1-yl)pyridin
6-(2-Hydroxymethyl-2-hydroxypyrrolidin-1-yl)-3-amino-2-(2-(hydroxymethyl)pyrrolidin-1-yl)pyridin
6-(2-Carboxamidopyrrolidin-1-yl)-3-amino-2-(2-(hydroxymethyl)pyrrolidin-1-yl)pyridin
6-(2-Dimethylcarboxamidopyrrolidin-1-yl)-3-amino-2-(2-(hydroxymethyl)pyrrolidin-1-yl)pyridin
6-(2-Carboxamido-3-hydroxypyrrolidin-1-yl)-3-amino-2-(2-(hydroxymethyl)pyrrolidin-1-yl)pyridin
6-(2-Carboxamido-4-hydroxypyrrolidin-1-yl)-3-amino-2-(2-(hydroxymethyl)pyrrolidin-1-yl)pyridin
6-(Pyrrolidin-1-yl)-3-amino-2-(2-carboxamidopyrrolidin-1-yl)pyridin
6-(3-Hydroxypyrrolidin-1-yl)-3-amino-2-(2-carboxamidopyrrolidin-1-yl)pyridin
6-(3-Aminopyrrolidin-1-yl)-3-amino-2-(2-carboxamidopyrrolidin-1-yl)pyridin
6-(3-Acetamidopyrrolidin-1-yl)-3-amino-2-(2-carboxamidopyrrolidin-1-yl)pyridin
6-(3,4-Dihydroxypyrrolidin-1-yl)-1-amino-2-(2-carboxamidopyrrolidin-1-yl)pyridin
6-(2-Carboxypyrrolidin-1-yl)-3-amino-2-(2-carboxamidopyrrolidin-1-yl)pyridin
6-(2-Carboxy-3-hydroxypyrrolidin-1-yl)-3-amino-2-(2-carboxamidopyrrolidin-1-yl)pyridin
6-(2-Carboxy-4-hydroxypyrrolidin-1-yl)-3-amino-2-(2-carboxamidopyrrolidin-1-yl)pyridin
6-(2-(Hydroxymethyl)pyrrolidin-1-yl)-3-amino-2-(2-carboxamidopyrrolidin-1-yl)pyridin
6-(2-Hydroxymethyl-4-hydroxypyrrolidin-1-yl)-3-amino-2-(2-carboxamidopyrrolidin-1-yl)pyridin
6-(2-Hydroxymethyl-2-hydroxypyrrolidin-1-yl)-3-amino-2-(2-carboxamidopyrrolidin-1-yl)pyridin
6-(2-Carboxamidopyrrolidin-1-yl)-3-amino-2-(2-carboxamidopyrrolidin-1-yl)pyridin
6-(2-Dimethylcarboxamidopyrrolidin-1-yl)-3-amino-2-(2-carboxamidopyrrolidin-1-yl)pyridin
6-(2-Carboxamido-3-hydroxypyrrolidin-1-yl)-3-amino-2-(2-carboxamidopyrrolidin-1-yl)pyridin
6-(2-Carboxamido-4-hydroxypyrrolidin-1-yl)-3-amino-2-(2-carboxamidopyrrolidin-1-yl)pyridin
6-(Pyrrolidin-1-yl)-3-amino-2-[methyl(2-hydroxyethyl)amino]pyridin
6-(3-Hydroxypyrrolidin-1-yl)-3-amino-2-[methyl(2-hydroxyethyl)amino]pyridin
6-(3-Aminopyrrolidin-1-yl)-3-amino-2-[methyl(2-hydroxyethyl)amino]pyridin
6-(3-Acetamidopyrrolidin-1-yl)-amino-2-[methyl-(2-hydroxyethyl)amino]pyridin
6-(3,4-Dihydroxypyrrolidin-1-yl)-3-amino-2-[methyl(2-hydroxyethyl)amino]pyridin
6-(2-Carboxypyrrolidin-1-yl)-3-amino-2-[methyl(2-hydroxyethyl)amino]pyridin
6-(2-Carboxy-3-hydroxypyrrolidin-1-yl)-3-amino-2-[methyl(2-hydroxyethyl)amino]pyridin
6-(2-Carboxy-4-hydroxypyrrolidin-1-yl)-3-amino-2-[methyl(2-hydroxyethyl)amino]pyridin
6-(2-(Hydroxymethyl)pyrrolidin-1-yl)-3-amino-2-[methyl(2-hydroxyethyl)amino]pyridin
6-(2-Hydroxymethyl-4-hydroxypyrrolidin-1-yl)-3-amino-2-[methyl(2-hydroxyethyl)amino]pyridin
6-(2-Hydroxymethyl-2-hydroxypyrrolidin-1-yl)-3-amino-2-[methyl(2-hydroxyethyl)amino]pyridin
6-(2-Carboxamidopyrrolidin-1-yl)-3-amino-2-[methyl(2-hydroxyethyl)amino]pyridin
6-(2-Dimethylcarboxamidopyrrolidin-1-yl)-3-amino-2-[methyl(2-hydroxyethyl)amino]pyridin
6-(2-Carboxamido-3-hydroxypyrrolidin-1-yl)-3-amino-2-[methyl(2-hydroxyethyl)amino]pyridin
6-(2-Carboxamido-4-hydroxypyrrolidin-1-yl)-3-amino-2-[methyl(2-hydroxyethyl)amino]pyridin
6-(Pyrrolidin-1-yl)-3-amino-2-[methyl(2-aminoethyl)amino]pyridin
6-(3-Hydroxypyrrolidin-1-yl)-3-amino-2-[methyl(2-aminoethyl)amino]pyridin
6-(3-Aminopyrrolidin-1-yl)-3-amino-2-[methyl(3-aminoethyl)amino]pyridin
6-(3-Acetamidopyrrolidin-1-yl)-3-amino-2-[methyl-(2-aminoethyl)amino]pyridin
6-(3,4-Dihydroxypyrrolidin-1-yl)-3-amino-2-[methyl(2-aminoethyl)amino]pyridin
6-(2-Carboxypyrrolidin-1-yl)-3-amino-2-[methyl(2-aminoethyl)amino]pyridin
6-(2-Carboxy-3-hydroxypyrrolidin-1-yl)-1-amino-2-[methyl(2-aminoethyl)amino]pyridin
6-(2-Carboxy-4-hydroxypyrrolidin-1-yl)-3-amino-2-[methyl(2-aminoethyl)amino]pyridin
6-(2-Hydroxymethyl)pyrrolidin-1-yl)-3-amino-2-[methyl(2-aminoethyl)amino]pyridin
6-(2-Hydroxymethyl-4-hydroxypyrrolidin-1-yl)-3-amino-2-[methyl(2-aminoethyl)amino]pyridin
6-(2-Hydroxymethyl-2-hydroxypyrrolidin-1-yl)-3-amino-2-[methyl(2-aminoethyl)amino]pyridin
6-(2-Carboxamidopyrrolidin-1-yl)-3-amino-2-[methyl(2-aminoethyl)amino]pyridin
6-(2-Dimethylcarboxamidopyrrolidin-1-yl)-3-amino-2-[methyl(2-aminoethyl)amino]pyridin
6-(2-Carboxamido-3-hydroxypyrrolidin-1-yl-)-3-amino-2-[methyl(2-aminoethyl)amino]pyridin
6-(2-Carboxamido-4-hydroxypyrrolidin-1-yl)-3-amino-2-[methyl(2-aminoethyl)amino]pyridin
6-(Pyrrolidin-1-yl)-3-amino-2-[methyl(2-acetamidoethyl)amino]pyridin
6-(3-Hydroxypyrrolidin-1-yl)-3-amino-2-[methyl(2-acetamidoethyl)amino]pyridin
6-(3-Aminopyrrolidin-1-yl)-3-amino-2-amino-2-[methyl(2-acetamidoethyl)amino]pyridin
6-(3-Acetamidopyrrolidin-1-yl)-3-amino-2-[methyl-(2-acetamidoethyl)amino]pyridin
6-(3,4-Dihydroxypyrrolidin-1-yl)-3-amino-2-[methyl(2-acetamidoethyl)amino]pyridin
6-(2-Carboxypyrrolidin-1-yl)-3-amino-2-[methyl(2-acetamidoetnyl)amino]pyridin
6-(2-Carboxy-3-hydroxypyrrolidin-1-yl)-3-amino-2-[methyl(3-acetamidoethyl)amino]pyridin
6-(2-Carboxy-4-hydroxypyrrolidin-1-yl)-3-amino-2-[methyl(2-acetamidoethyl)amino]pyridin
6-(2-(Hydroxymethyl)pyrrolidin-1-yl)-3-amino-2-[methyl(2-acetamidoethyl)amino]pyyidin
6-(2-Hydroxymethyl-4-hydroxypyrrolidin-1-yl)-3-amino-2-[methyl(2-acetamidoethyl)aminolpyridin
6-(2-Hydroxymethyl-2-hydroxypyrrolidin-1-yl)-3-amino-2-[methyl(2-acetamidoethyl)amino]pyridin
6-(2-Carboxamidopyrrolidin-1-yl)-3-amino-2-[methyl(2-acetamidoethyl)amino-pyridin
6-(2-Dimethylcarboxamidopyrrolidin-1-yl)-3-amino-2-[methyl(2-acetamidoethyl)amino]pyridin
6-(2-Carboxamido-3-hydroxypyrrolidin-1-yl)-3-amino-2-[methyl(2-acetamidoethyl)amino]pyridin
6-(2-Carboxamido-4-hydroxypyrrolidin-1-yl)-3-amino-2-[methyl(2-acetamidoethyl)amino]pyridin
6-(Pyrrolidin-1-yl)-3-amino-2-methoxypyridin
6-(3-Hydroxypyrrolidin-1-yl)-3-amino-2-methoxypyridin
6-(3-Aminopyrrolidin-1-yl)-3-amino-2-methoxypyridin
6-(3-Acetamidopyrrolidin-1-yl)-3-amino-2-methoxypyridin
6-(3,4-Dihydroxypyrrolidin-1-yl)-3-amino-2-methoxypyridin
6-(2-Carboxypyrrolidin-1-yl)-3-amino-2-methoxypyridin
6-(2-Carboxy-3-hydroxypyrrolidin-1-yl)-3-amino-2-methoxypyridin
6-(2-Carboxy-4-hydroxypyrrolidin-1-yl)-3-amino-2-methoxypyridin
6-(2-(Hydroxymethyl)pyrrolidin-1-yl)-3-amino-2-methoxypyridin
6-(2-Hydroxymethyl-4-hydroxypyrrolidin-1-yl)-3-amino-2-methoxypyridin
6-(2-Hydroxymethyl-2-hydroxypyrrolidin-1-yl)-3-amino-2-methoxypyridin
6-(2-Carboxamidopyrrolidin-1-yl)-3-amino-2-methoxypyridin
6-(2-Dimethylcarboxamidopyrrolidin-1-yl)-3-amino-2-methoxypyridin
6-(2-carboxamido-3-hydroxypyrrolidin-1-yl)-3-amino-2-methoxypyridin
6-(2-Carboxamido-4-hydroxypyrrolidin-1-yl)-3-amino-2-methoxypyridin
6-(Pyrrolidin-1-yl)-3-amino-2-(2-hydroxyethyloxy)-pyridin
6-(3-Hydroxypyrrolidin-1-yl)-3-amino-2-(2-hydroxyethyloxy)pyridin
6-(3-Aminopyrrolidin-1-yl)-3-amino-2-(2-hydroxyethyloxy)pyridin
6-(3-Acetamidopyrrolidin-1-yl)-3-amino-2-(2-hydroxyethyloxy)pyridin
6-(3,4-Dihydroxypyrrolidin-1-yl)-3-amino-2-(2-hydroxyethyloxy)pyridin
6-(2-Carboxypyrrolidin-1-yl)-3-amino-2-(2-hydroxyethyloxy)pyridin
6-(2-Carboxy-3-hydroxypyrrolidin-1-yl)-3-amino-2-(2-hydroxyethyloxy)pyridin
6-(2-Carboxy-4-hydroxypyrrolidin-1-yl)-3-amino-2-(2-hydroxyethyloxy)pyridin
6-(3-(Hydroxymethyl)pyrrolidin-1-yl)-3-amino-2-(2-hydroxyethyloxy)pyridin
6-(2-Hydroxymethyl-4-hydroxypyrrolidin-1-yl)-3-amino-2- (2-hydroxyethyloxy)pyridin
6-(2-Hydroxymethyl-2-hydroxypyrrolidin-1-yl)-3-amino-2-(2-hydroxyethyloxy)pyridin
6-(2-Carboxamidopyrrolidin-1-yl)-3-amino-2-(2-hydroxyethyloxy)pyridin
6-{2-Dimethylcarboxamidopyrrolidin-1-yl)-3-amino-2-(2-hydroxyethyloxy)pyridin
6-(2-Carboxamido-3-hydroxypyrrolidin-1-yl)-3-amino-2-(2-hydroxyethyloxy)pyridin
6-(2-Carboxamido-4-hydroxypyrrolidin-1-yl)-3-amino-2-(2-hydroxyethyloxy)pyridin
6-(Pyrrolidin-1-yl)-3-amino-2-ethoxypyrridin
6-(3-Hydroxypyrrolidin-1-yl)-3-amino-2-ethoxypyridin
6-(3-Aminopyrrolidin-1-yl)-3-amino-2-ethoxypyridin
6-(3-Acetamidopyrrolidin-1-yl)-1-amino-2-ethoxypyridin
6-(3,4-Dihydroxypyrrolidin-1-yl)-3-amino-2-ethoxypyridin
6-(2-Carboxypyrrolidin-1-yl)-3-amino-2-ethoxypyridin
6-(2-Carboxy-3-hydroxypyrrolidin-1-yl)-3-amino-2-ethoxypyridin
6-(2-Carboxy-4-hydroxypyrrolidin-1-yl)-3-amino-2-ethoxypyridin
6-(2-(Hydroxymethyl)pyrrolidin-1-yl)-3-amino-2-ethoxypyridin
6-(3-Hydroxymethyl-4-hydroxypyrrolidin-1-yl)-3-amino-2-ethoxypyridin
6-(2-Hydroxymethyl-2-hydroxypyrrolidin-1-yl)-3-amino-2-ethoxypyridin
6-(2-Carboxamidopyrrolidin-1-yl)-3-amino-2-ethoxypyridin
6-(2-Dimethylcarboxamidopyrrolidin-1-yl)-3-amino-2-ethoxypyridin
6-(2-Carboxamido-3-hydroxypyrrolidin-1-yl)-3-amino-2-ethoxypyridin
6-(2-Carboxamido-4-hydroxypyrrolidin-1-yl)-3-amino-2-ethoxypyridin
und Additionssalzen davor.
